# EUROPEAN PATENT SPECIFICATION

(11) **EP 4 552 699 B1**
(45) Date of publication and mention of the grant of the patent: **09.12.2026**
(21) Application number: 25153140.6
(22) Date of filing: 20.02.2018
(51) Int. Cl.: C07K 16/18, C07K 14/715, A61K 38/20, A61K 39/395, C07K 16/22, C07K 16/28, A61P 35/00

(54) **ALBUMIN BINDING DOMAIN FUSION PROTEINS**
FUSIONSPROTEINE MIT ALBUMINBINDUNGSDOMÄNE
PROTÉINES DE FUSION À DOMAINE DE LIAISON À L'ALBUMINE

(30) Priority: 16.02.2017 US 201762459975 P; 16.02.2017 US 201762459981 P
(43) Date of publication of application: 14.05.2025
(62) Document number(s) of the earlier application(s) in accordance with Art. 76 EPC: 18710585.3 / 3 583 125
(73) Proprietor: Guidant Biotherapeutics, Inc., Princeton, NJ 08540 (US)
(72) Inventor: CINI, John, K, Blairstown, 07825 (US); HUANG, Haomin, Yardley, 19067 (US)
(74) Representative: HGF

(56) References cited:
- WO-A1-2013/068571
- WO-A2-2008/096158
- EMMA DAVE ET AL: "Fab-dsFv: A bispecific antibody format with extended serum half-life through albumin binding", MABS, vol. 8, no. 7, 17 August 2016 (2016-08-17), US, pages 1319 - 1335, XP055428628, ISSN: 1942-0862, DOI: 10.1080/19420862.2016.1210747
- CUNDALL MEGHAN: "PRODUCTION OF SINGLE-CHAIN ANTI-ALBUMIN ANTIBODIES FOR USE IN PROTEIN STABILIZATION", 1 January 1998 (1998-01-01), National Library of Canada, XP093283133, Retrieved from the Internet <URL:https://www.collectionscanada.gc.ca/obj/s4/f2/dsk2/ftp01/MQ33217.pdf>
- JUNKO MURAOKA ET AL: "Selection and Characterization of Human Serum Albumin-specific Porcine scFv Antibodies Using a Phage Display Library", MONOCLONAL ANTIBODIES IN IMMUNODIAGNOSIS AND IMMUNOTHERAPY, vol. 33, no. 1, 1 February 2014 (2014-02-01), pages 42 - 48, XP055476191, ISSN: 2167-9436, DOI: 10.1089/mab.2013.0068
- GORAWIT YUSAKUL ET AL: "Effect of linker length between variable domains of single chain variable fragment antibody against daidzin on its reactivity", BIOSCIENCE, BIOTECHNOLOGY, AND BIOCHEMISTRY, vol. 80, no. 7, 2 July 2016 (2016-07-02), JP, pages 1306 - 1312, XP055493793, ISSN: 0916-8451, DOI: 10.1080/09168451.2016.1156482
- HAOMIN HUANG ET AL: "SITC Poster # P313 11/10/17 In vivo Effect of Albumin Binding Domains (ABD) Attached to Immune Modulators", 10 November 2017 (2017-11-10), XP055758942, Retrieved from the Internet <URL:https://d1io3yog0oux5.cloudfront.net/_110148447f0bdb173fbd1519fe8ee3fe/sonnetbio/db/839/6669/pdf/Sonnet_SITC_Poster_FINAL_2017.pdf> [retrieved on 20201210]
- HUANG HAOMIN ET AL: "In vivo Effect of Albumin Binding Domains (ABD) Attached to Immune Modulators", 10 November 2017 (2017-11-10), XP093283201, Retrieved from the Internet <URL:https://d1io3yog0oux5.cloudfront.net/_110148447f0bdb173fbd1519fe8ee3fe/sonnetbio/db/839/6669/pdf/Sonnet_SITC_Poster_FINAL_2017.pdf>
- JOHN CINI: "Abstract B004: Enhanced efficacy of immune modulators with albumin binding domains | Molecular Cancer Therapeutics", 1 January 2018 (2018-01-01), XP055476368, Retrieved from the Internet <URL:http://mct.aacrjournals.org/content/17/1_Supplement/B004> [retrieved on 20180517]
- HUANG HAOMIN ET AL: "A novel strategy to produce high level and high purity of bioactive IL15 fusion proteins from mammalian cells", PROTEIN EXPRESSION AND PURIFICATION, ACADEMIC PRESS, SAN DIEGO, CA, vol. 148, 26 March 2018 (2018-03-26), pages 30 - 39, XP085392323, ISSN: 1046-5928, DOI: 10.1016/J.PEP.2018.03.010

## Description

### BACKGROUND

While biologics have been useful in the treatment of many diseases, including cancers, the short circulatory half-life of such molecules represent a major obstacle.

Biologics are useful for the treatment of cancers in a variety of ways. Cytokine-based therapies can work directly on cancer cells by interfering with how such cells grow and multiply. Cytokines may also stimulate the immune system, by encouraging the growth of killer T cells and other cells that attack cancer cells. Further, cytokines can promote cancer cells to send out chemicals that attract immune system cells. *See, e.g.,* Dranoff, Nature Reviews Cancer 4: 11-22 (2004); and Zhang et al., Proc Natl Acad Sci U.S.A. 106(18): 7513-7518 (2009). Antibodies are desirable as therapeutics due to their ability to recognize targets with both specificity and high affinity. Monoclonal antibody based therapies, including those that target tumor surface antigens and inhibitory signals that limit T-cell activation, have been a standard component of cancer therapeutics for over 20 years. *See, e.g.,* Weiner, Nat Rev Cancer 15(6): 361-370 (2015).

Short circulatory half-life represents a major obstacle for many biologics. *See, e.g.,* Perdreau et al., European Cytokine Network 21: 297-307 (2010). Such short-acting therapeutics require frequent dosing profiles that can reduce applicability to the clinic, particular for chronic conditions. Long serum half-life is desirable as it would decrease the need for repetitive injections of the molecule to achieve a therapeutically relevant serum concentration. Methods of extending the half-life of therapeutic proteins include PEGylation, fusion to human serum albumin (HSA), fusion to the constant fragment (Fc) of a human immunoglobulin IgG, and fusion to non-structured polypeptides such as XTEN. *See, e.g*., Stohl, BioDrugs 29(4): 215-239 (2015). Half-life extension technologies enable new and improved biologic therapies that reduce the cost and burden of frequent dosing. Thus, there remains a continued need for novel reagents and methods useful for extending the half-lives of protein and peptide based therapeutics.

WO2013/068571 discloses anti-serum albumin binding scFvs.

Emma Dave et al., "Fab-dsFv: A bispecific antibody format with extended serum half-life through albumin binding, MABS, vol. 8, no. 7, 17 August 2016, pages1319-1335, discloses the use of Fab-Fv and Fab-dsFv format antibodies that include an anti-human serum albumin (HSA) Fv region and a Fab region.

Meghan Cundall, "Production of single-chain anti-albumin antibodies for use in protein stabilization", National Library of Canada, 1 January 1998, discloses two fusion proteins in which IL-2 is conjugated to an albumin binding scFv.

Junko Muraoka et al., "Selection and Characterization of Human Serum Albumin-specific Porcine scFv Antibodies Using a Phage Display Library", Monoclonal Antibodies in Immunodiagnosis and Immunotherapy, vol. 33, no. 1, 1 February 2014, pages 42-48, discloses four different albumin binding scFvs based on four parental monoclonal antibodies known to bind serum albumin.

### SUMMARY

The presently claimed invention is as defined in the claims.

In one aspect, the present invention provides an IL-12 albumin binding domain fusion protein comprising the amino acid sequence of

IWELKKDVYVVELDWYPDAPGEMVVLTCDTPEEDGITWTLDQSSEVLGSGKTLTIQVK
EFGDAGQYTCHKGGEVLSHSLLLLHKKEDGIWSTDILKDQKEPKNKTFLRCEAKNYSG
RFTCWWLTTISTDLTFSVKSSRGSSDPQGVTCGAATLSAERVRGDNKEYEYSVECQE
DSACPAAEESLPIEVMVDAVHKLKYENYTSSFFIRDIIKPDPPKNLQLKPLKNSRQVEVS
WEYPDTWSTPHSYFSLTFCVQVQGKSKREKKDRVFTDKTSATVICRKNASISVRAQDR
YYSSSWSEWASVPCSGGGGGGSRNLPVATPDPGMFPCLHHSQNLLRAVSNMLQKA
RQTLEFYPCTSEEIDHEDITKDKTSTVEACLPLELTKNESCLNSRETSFITNGSCLASRK
TSFMMALCLSSIYEDLKMYQVEFKTMNAKLLMDPKRQIFLDQNMLAVIDELMQALNFNS
ETVPQKSSLEEPDFYKTKIKLCILLHAFRIRAVTIDRVTSYLNASGGGGSGGGGSGGGG
SGGGGSGGGGSEVQLVESGGGLIQPGRSLRLSCAASGITFDDAVMHWVRQAPGKGL
EWVAGISSNSGYIGYADSVKGRFTISRDNAKNSLYLQMNRLRAEDTAVYYCVKGLYSN
PRGGAFDIWGQGTMVTVSSASTGGGGSGGGGSGGGGSVHSSYVLTQPPSVSVAPG
QTATITCGGNNIGTKSVHWYQQKPGQAPVLVVYADSDRPSGIPERVSGSNSGNTATLT
ISRVEAGDEADYYCQVWDSRSDHLWVFGGGTKLTVLG.

In one aspect, the present invention provides a nucleic acid encoding the IL-12 albumin binding domain fusion protein as provided herein.

In one aspect, the present invention provides an expression vector comprising the nucleic acid as provided herein.

In one aspect, the present invention provides a host cell comprising the nucleic acid as provided herein or the expression vector as provided herein.

In one aspect, the present invention provides a method of making the IL-12 albumin binding domain fusion protein as provided herein, comprising culturing the host cell as provided herein under conditions wherein the IL-12 albumin binding domain fusion protein is expressed, and recovering the IL-12 albumin binding domain fusion protein.

In one aspect, the present invention provides an IL-12/IL-15 albumin binding domain fusion protein comprising the amino acid sequence of

IWELKKDVYVVELDWYPDAPGEMVVLTCDTPEEDGITWTLDQSSEVLGSGKTLTIQVKE
FGDAGQYTCHKGGEVLSHSLLLLHKKEDGIWSTDILKDQKEPKNKTFLRCEAKNYSGRF
TCWWLTTISTDLTFSVKSSRGSSDPQGVTCGAATLSAERVRGDNKEYEYSVECQEDSA
CPAAEESLPIEVMVDAVHKLKYENYTSSFFIRDIIKPDPPKNLQLKPLKNSRQVEVSWEY
PDTWSTPHSYFSLTFCVQVQGKSKREKKDRVFTDKTSATVICRKNASISVRAQDRYYSS
SWSEWASVPCSGGGGGGSRNLPVATPDPGMFPCLHHSQNLLRAVSNMLQKARQTLE
FYPCTSEEIDHEDITKDKTSTVEACLPLELTKNESCLNSRETSFITNGSCLASRKTSFMM
ALCLSSIYEDLKMYQVEFKTMNAKLLMDPKRQIFLDQNMLAVIDELMQALNFNSETVPQ
KSSLEEPDFYKTKIKLCILLHAFRIRAVTIDRVTSYLNASGGGGSGGGGSGGGGSGGGG
SGGGGSEVQLVESGGGLIQPGRSLRLSCAASGITFDDAVMHWVRQAPGKGLEWVAGI
SSNSGYIGYADSVKGRFTISRDNAKNSLYLQMNRLRAEDTAVYYCVKGLYSNPRGGAF
DIWGQGTMVTVSSASTGGGGSGGGGSGGGGSVHSSYVLTQPPSVSVAPGQTATITCG
GNNIGTKSVHWYQQKPGQAPVLVVYADSDRPSGIPERVSGSNSGNTATLTISRVEAGD
EADYYCQVWDSRSDHLWVFGGGTKLTVLGGGGGSGGGGSGGGGSGGGGSGGGGS
NWVNVISDLKKIEDLIQSMHIDATLYTESDVHPSCKVTAMKCFLLELQVISLESGDASIHD
TVENLIILANNSLSSNGNVTESGCRECEELEEKNIKEFLQSFVHIVQMFIATS.

In one aspect, the present invention provides a nucleic acid encoding the IL-12/IL-15 albumin binding domain fusion protein as provided herein.

In one aspect, the present invention provides an expression vector comprising the nucleic acid as provided herein.

In one aspect, the present invention provides a host cell comprising the nucleic acid as provided herein or the expression vector as provided herein.

In one aspect, the present invention provides a method of making an IL-12/IL-15 albumin binding domain fusion protein comprising culturing the host cell as provided herein under conditions wherein the IL-12/IL-15 albumin binding domain fusion protein is expressed, and recovering the IL-12/IL-15 albumin binding domain fusion protein.

### BRIEF DESCRIPTION OF THE DRAWINGS

The invention is defined in the claims. Any of the following Figures that do not fall within the scope of the claims do not form part of the invention and are provided for comparative purposes only.
Figures 1A-C illustrates several exemplary albumin binding domain fusion proteins disclosed herein, including fusion proteins where an albumin binding domain is attached to a cytokine (*e.g*., IL-12 or IL-15) and fusion proteins where an albumin bind domain is attached to 1) two cytokines; 2) two binding moieties (*e.g*., scFvs); 3) a binding moiety and a cytokine.
Figures 2A -2Z shows the sequences of exemplary albumin binding domains included in certain embodiments of the subject albumin binding domain fusion proteins described herein. Included in these figures are the variable heavy domain and variable light domain sequences, as well as particular vhCDR1-3 and vlCDR1-3 sequences.
Figure 3 shows the sequences of exemplary IL-15 variants described herein.
Figure 4 shows exemplary embodiments of the IL-15-ABD fusion proteins described herein. The IL-15-ABD fusion proteins depicted in Figure 4 include A10m3 ABD.

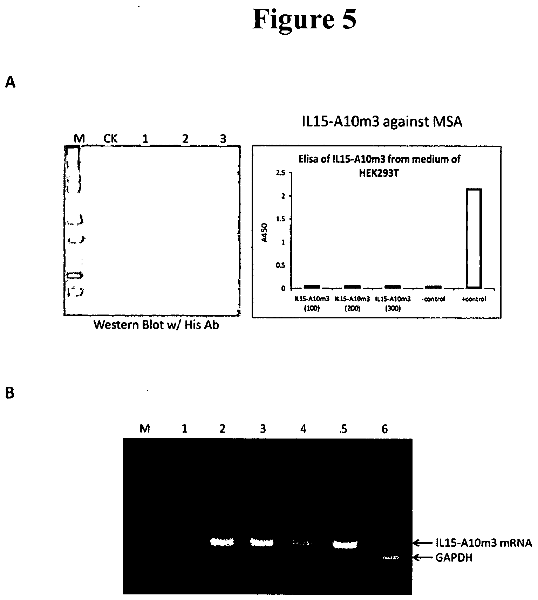

Figure 5 depicts the results of studies showing that transcription does not account for the low expression of IL-15-A10m3 in HEK293T cells. Verification of IL15-A10m3 mRNA in transfected cells. A) Expression of IL15-A10m3 in HEK293 cannot be detected by Western blotting using either anti-His tag antibody (left), or by functional ELISA binding to MSA (right). M: Marker, CK: non-transfected culture medium as control, 1-3: media from 3 independently transfected cell cultures (100, 200 and 250 = 100, 200, 250ug/ml Zeocin respectfully). 10ug/ml E. coli produced IL15-A10m3 was served as the positive control. B) mRNA was prepared from 4 independently IL15-A10m3 transfected cells and RT-PCR was performed to quantify the mRNA level of IL15-A10m3 mRNA in comparison with that of a house-keeping gene, GAPDH. Lane1) untransfected cell control; 2-5) Transcription of IL15-A10m3 mRNA appears to be normal in all transfected cells; 6) GAPDH mRNA positive control.
Figure 6 depicts the identification of the potential IL-15 ubiquitination sites that are putatively protected by IL-15 receptor alpha (IL-15Rα) binding to IL-15. (A) K86 in red is a putative ubiquitination site which is next to the IL-15Rα binding sites (marked by stars); (B) K86 is a hit for ubiquitination from UbPred, an online ubiquitination site database (www.ubpred.org).
Figure 7 provides schematic illustrations of various IL-15-ABD fusion proteins with improved stability described herein, including an IL-15-ABD with a IL-15Ra/IL15 "sushi domain" fusion partner (A) and IL-15-ABD fusion proteins that include IL-15 variant fusion partners having amino acid substitutions at putative ubiquitination site K86 (B).

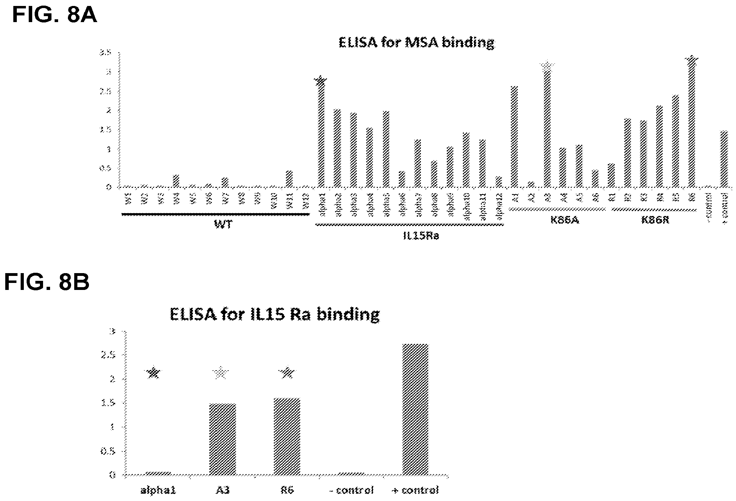

Figure 8 depicts studies assessing the expression level (Figure 8A) and ability of HEK293 cell-produced IL-15-ABD K86R and K86A variants and IL-15Rα/IL-15-ABD to bind mouse serum albumin (Figure 8A) and IL-15Rα (Figure 8B). A) ELISA readout for IL15-ABD binding to mouse serum albumin (MSA) of 12 WT IL15-A10m3 clones (WT), 12 IL-15Rα/IL-15-A10m3 clones (IL15Ra), 6 IL-15 K86A-A10m3 mutant clones (K86A) and 6 IL-15 K86R-A10m3 mutant clones (K86R). Culture medium of each sample well from 24-well plates was added to ELISA plates coated with MSA. B) ELISA for binding of IL-15 to IL-15 receptor alpha (1L-15Rα) which was coated on the plate was used to confirm that K86A (clone A3, yellow star) and K86R (clone R6, green star) mutations had no impact on the binding activity of IL-15 to IL-15Rα. The internal IL-15Rα in IL-15Rα -IL-15-A10m3 (clone alpha1, red star) could bind to internal IL15 and thus block its binding to IL-15Rα coated on the plate. 10ug/ml of E.coli produced WT IL-15-A10m3 was used as positive control.
Figure 9 depicts the scaled-up production of IL15K86R-A10m3 clone #6. A) Chromatograph of IL15K86R-A10m3 by a size exclusion column; B) SDS-PAGE analysis of the SEC fractions from 14 to 42; C) Final products (1: IL15K86R-A10m3, 2: IL15Ra-IL15-A10m3) were final confirmed by SDS-PAGE (left) and Western blotting with anti-His tag antibody (right).
Figure 10 depicts the results of *in vitro* binding assays, confirming the ability of IL-15 K86R-A10m3 to bind mouse serum albumin (MSA).
Figure 11 depicts the results of CTLL2 proliferation assays, showing that IL-15 K86R -A10m3 polypetides produced form HEK293T cells have reduced bioactivity compared to wildtype IL-15 and IL-15-A10m3 produced from E. coli.
Figure 12 depicts results of studies showing that the reduced bioactivity of HEK cell produced IL-15 K86R - A10m3 is at least partially due to its glycosylation.
Figure 13 provides the results of studies, showing that N112A mutation introduced into IL15 K86R-A10m3 can restore IL-15 bioactivity comparable to that of deglycosylated IL-15R-A10m3 in CTLL2 proliferation assays (A). (B) further shows that IL-15R-A10m3 fusion proteins with IL-15 amino acid substitutions N112A, N112Q and N112S displayed increased bioactivity inversely proportional to the size of the substituted side chain.
Figure 14 depicts the experimental set up of a study to assess the *in vivo* effects of IL-15 and IL-15-ABD on tumor growth in a B16-F10 mouse melanoma model.

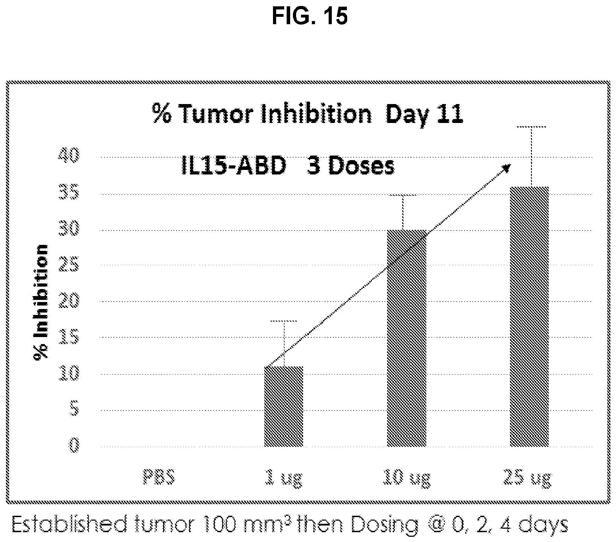

Figure 15 is a graph, showing the amount of B16-F10 tumor growth inhibition in C57BL/6 mice treated with IL-15-ABD fusion protein, 11 days after treatment.
Figure 16 provides FACS analyses, depicting the effect of IL-15-ABD treatment on tumor infiltration lymphocyte populations.
Figures 17 and 18 provide a summary of the effects of IL-15-ABD treatment on lymphocyte populations in spleens and tumors.
Figures 19A and B provide results of studies showing the stability of subject IL-15-ABD in a mouse model (A) and in human serum (B), as compared to control IL-15 WT.
Figure 20 depicts the sequences of exemplary IL-12-ABDs described herein: mIL-12sc-A10m3 and human IL-12sc-A10m3.
Figures 21 and 22 are studies showing that subject IL-12-ABD produced from HEK293T cells are biologically active in *in vitro* assays and cell based assays.
Figure 23 depicts the experimental set up of a study to assess the *in vivo* effects of IL-12 and IL-12-ABD on tumor growth in a B16-F10 mouse melanoma model. Similar molar concentrations of IL-12 and IL-12 were used at three different concentrations. For example, 3 µg of IL-12 is the same molar concentration as 4.5 µg of IL-12-ABD, 10 µg of IL-12 is the same molar concentration as 15 µg of IL-12-ABD, and 20 µg of IL-12 is the same molar concentration as 30 µg of IL-12-ABD. The molecular weight of IL-12 is 70 kD and the molecular weight of IL-12-ABD is 107 kD.
Figure 24 are graphs depicting the tumor growth kinetics of various groups in the *in vivo* IL-12-ABD/IL-12 studies. Figure 24A shows the results of tumor size assessments in IL-12 and IL-12-ABD groups, Figure 24B shows IL-12 treatment groups separately and Figure 24C shows IL-12-ABD treatment groups separately.
Figure 25 are graphs depicting the tumor growth kinetics of individual animals in each of the various group of the *in vivo* IL-12-ABD/IL-12 studies.
Figure 26 are graphs showing the tumor volumes of various groups in the *in vivo* IL-12-ABD/IL-12 studies, 10 days post-treatment.
Figure 27 depicts longitudinal body weight (left) and % body weight (right) measurements in B16-F10 tumor-bearing mice at various time points after treatment with IL-12 or IL-12-ABD.
Figure 28 provides Kalpan-Meier Pseuo-Survival Curves for various groups in the *in vivo* IL-12-ABD/IL-12 studies.
Figure 29 depicts a summary of studies comparing the pharmacodynamic effects of a single dose of IL-12-ABD versus IL-12 on B16-F10 tumor-bearing mice at five days. Comparisons of tumor weight, spleen weight, serum IFN-γ and body weight are shown.
Figure 30 shows the result of a study comparing tumor volume of B16-F10 tumor-bearing mice at 10 days, injected either with IL-12-ABD (1.3 µg), IL-12 (30 µg), or placebo. IL-12-ABD is administered at a lower molar dose ~30 fold than IL-12 in this study.
Figure 31 further depicts the hematopoietic effects of IL-12-ABD and IL-12 in mice from the study depicted in Figure 32 at 3 and 7 days.
Figure 32 depicts a study, showing the effect of single dose combination therapies using IL-12-ABD or IL-12 with anti-PD-1 antibodies on tumor growth in B16-F10 tumor-bearing mice.
Figure 33 depicts the results of a serum analysis, showing an increase in PK for IL12-ABD over IL-12 WT.
Figure 34 depicts the sequences of exemplary subject IL-15-ABD-IL-12 described herein: 1) hIL-15 (K86R/N112A)-A10m3-mIL-12sc; 2) mIL-12sc-A10m3- hIL-15 (K86R); and 3) mIL-12sc-A10m3- hIL-15 (K86R/N112A).
Figure 35 shows binding activities of both hIL-15 (K86R)-A10m3-mIL-12sc and mIL-12sc -A10m3- hIL-15 (K86R) to MSA (A), IL-15 receptor alpha and IL-12 receptor beta2 (B), as confirmed by ELISA.
Figure 36 depicts the sequences of additional exemplary subject IL-15-ABD-IL-12 described herein: 1) hIL-15 (K86R)-A10m3-hIL-12sc; 2) hIL-12sc-A10m3- hIL-15 (K86R); and 3) hIL-12sc-A10m3- hIL-15 (K86R/N112A).
Figures 37 and 38 depict the results of experiments showing that IL-15-ABD-IL-12 described herein exhibit both IL-12 (Figure 39) and IL-15 (Figure 40) activity.
Figure 39 depicts the results of a study, showing that IL-15-ABD-IL-12 described herein exhibited superior activity as compared to IL-12-ABD alone.
Figures 40A and B depict the sequences of two exemplary TGFβ binding domains described herein: 4H7 and 4D9, including variable heavy chain, variable light chain and scFv format sequences.
Figure 41 depicts the results of a study, showing that TGFβ scFvs described herein are capable of blocking TGFβ1 induced Treg (CD4+Foxp3+) expansion.

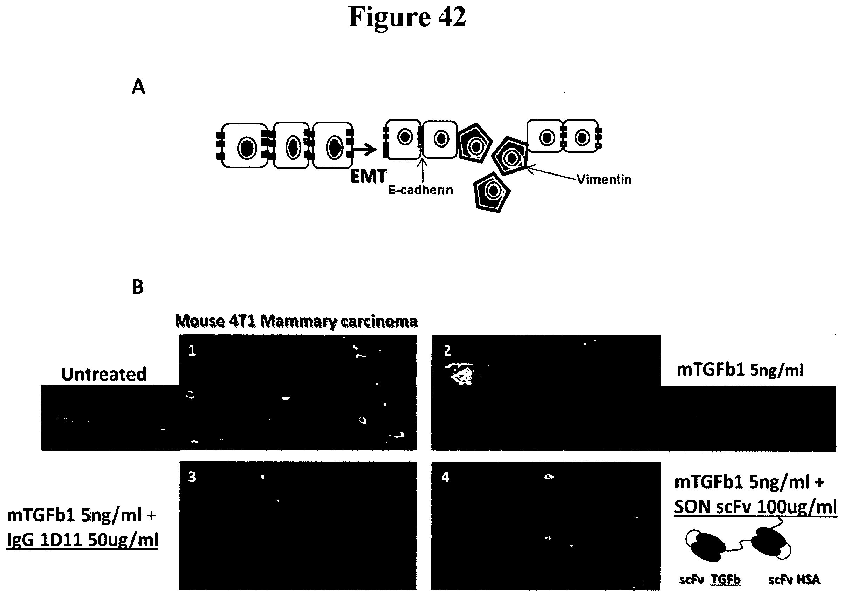

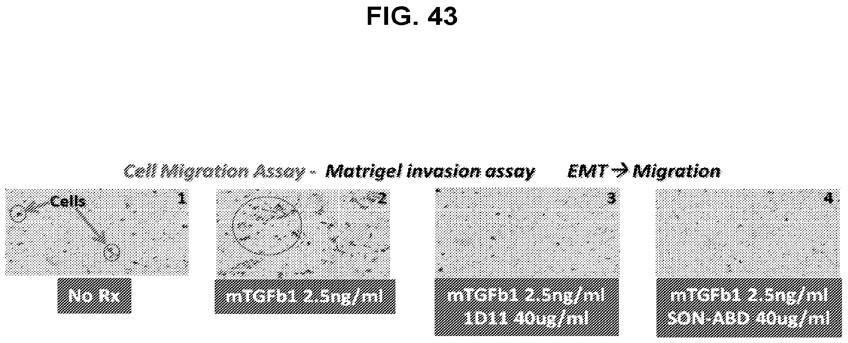

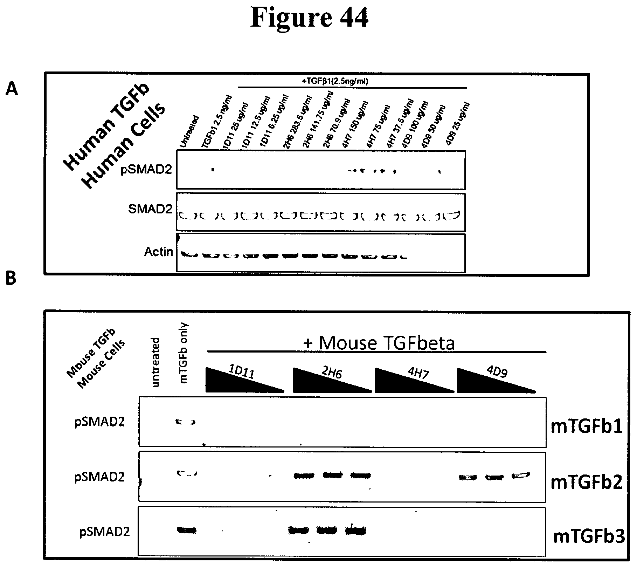

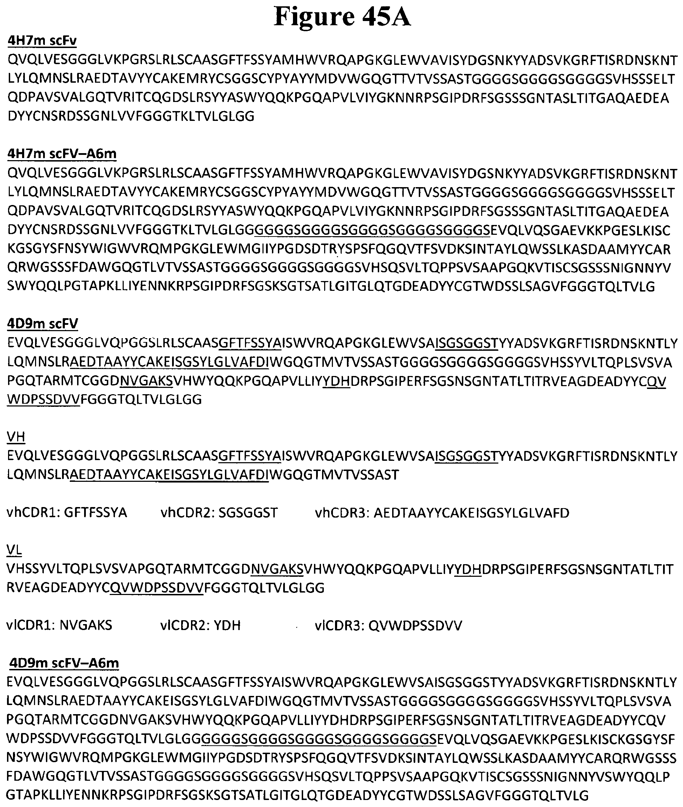

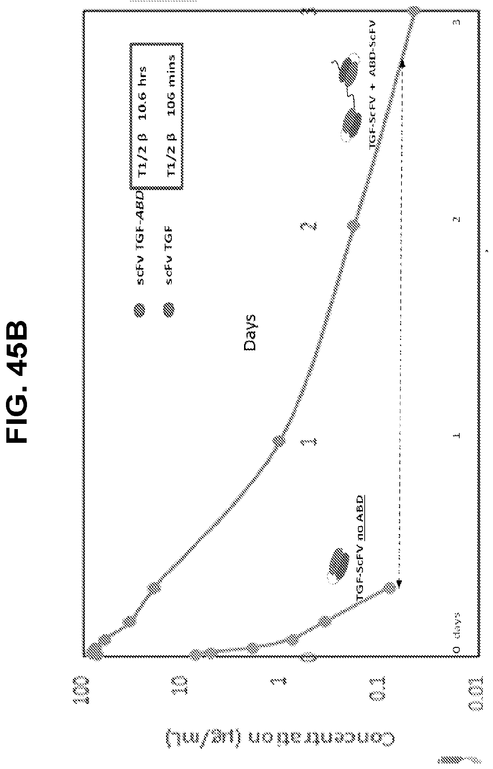

Figures 42 and 43 show that TGF-β blockade reverses TGF-β1-induced epithelial-to-mesenchymal transition (Figure 44) and migration (Figure 45). (Figure 42A) Schematic representation of the loss of E-cadherin and induced expression of vimentin during EMT. (Figure 44B) Mouse 4T1 cells were cultured in growth media supplemented with TGF-β1 alone or with 1D11 (panel 3) or anti-TGF-β1 scFv (panel 4), then fixed and stained with E-cadherin antibody (green) and vimentin antibody (purple). Nuclei were counterstained with DAPI (blue). Treatment with TGF-β1 induced loss of E-cadherin from cell-cell junctions and increased expression of vimentin. This effect is reversed by the addition of 1D11 or subject anti-TGF-β1 scFv described herein. Figure 43 further shows that subject anti-TGF-β1 scFvs described herein block TGF-β1-mediated carcinoma cell migration.
Figure 44 provides a summary of studies showing that subject anti-TGF-β1 scFvs described herein are capable of inhibiting TGF-β-mediated Smad2 phosphorylation in human (A) and mouse cells (B). In A, subject anti-TGF-β1 scFvs were capable of inhibiting human TGF-β1 mediated Smad2 phosphorylation in human cells in a dose dependent manner. In B, subject anti-TGF-β1 scFvs were capable of inhibiting mouse TGF-β1, -β2 and -β3 mediated Smad2 phosphorylation in mouse cells.
Figure 45A depicts the sequences of exemplary TGF-β1 scFv-ABD constructs (4D9M-A6m and 4H7-A6m).
Figure 45B provides a summary of a study, showing that anti-TGFβ-1-ABD extends the anti-TGFβ-1 scFv.
Figure 46 provides a summary of a study, showing that various anti-TGF-β1 scFv-ABDs (bivalent for TGF-β1) produced in E. Coli or mammalian (HEK) cells are capable of binding to mouse serum albumin.
Figure 47 provides the summary of a studying, showing that TGFβ-1 mediated inhibition of T cell proliferation was reversed (i.e., increase T cell proliferation) by exemplary TGF-β1 scFv-ABD constructs (4D9M-A6m and 4H7-A6m).
Figure 48 depicts exemplary linkers that can be used with the subject fusion proteins. Such linkers can be used as scFv linkers for ABD variable heavy chain and variable light chains. Such linkers can also be used to attach the albumin binding domains described herein to IL-12 and IL-15 fusion partners or to connect components of IL-12 fusion partners (p35 and p40) or IL-15 fusion partners (IL-15 and IL-15Rα) to each other.
Figure 49A-G provides the amino acid sequence of exemplary cytokines that can be included in the ABD fusion proteins described herein. Also depicted in Figures 49A-G are exemplary cytokine-ABD fusion proteins where the ABD is A10m3. Although such cytokines-ABD fusion proteins are depicted with A10m3 ABD, any ABD, including ABD described herein can be included in the cytokine-ABD fusion proteins.
Figure 50 provides the amino sequence of an exemplary anti-PD-L1 binding domain, 10D12 that can be used with the ABD fusion proteins described herein. 10D12 binds to hPD-L1 at low pH and is crossreactive with mPD-L1. 10D12 does not bind to hPD-L2 or mPD-L2. Further, 10D12 blocks PD-1/PD-L1 interaction, as well as B71/PD-L1 interaction.
Figures 51A and B depict several useful cytokine-ABD-cytokine (A) and binding moiety-ABD-cytokine/binding moiety (B) combinations. "A" and "B" in each combination can be switched to the opposite orientation. Also depicted in these figures is an linker-A10m3-linker backbone that can be included in the cytokine-ABD-cytokine and binding moiety-ABD-cytokine/binding moiety combinations. Although A10m3 ABD is depicted, any ABD, include any of those depicted herein (*e.g*., Figure 2) can be used in such constructs.

### DETAILED DESCRIPTION

### A. Overview

Biologics, including cytokine and antibody based therapeutics are useful for the treatment of cancers.

Current and potential cytokine based therapeutics include those utilizing IL-2, IL-7, IL-12, IL-15, IL-18, IL-21 GM-CSF and IFN-α.

IL-12 is capable of mediating immune effector functions in a manner compatible to enhancing pro-inflammatory, endogenous anti-tumor immune response. (*See, e.g*., Boggio et al., J Exp Med 188:589-96 (1998); Cavallo et al., Cancer Res 59:414-21 (1999); Yu et al. Int Immunol 8:855-65 (1996); Nastala et al., J Immunol 153:1697:706 (1994); Brunda et al., J Exp Med 178. 1223-30 (1993). IL-12 is known to induce an inflammatory Th1 CD4+ T cell response as well as enhance CD8+ T cell cytotoxicity. Studies have also shown that T cell secretion of IFNγ mediated by IL-12 may reverse T cell anergy and confer effector T cell resistance to immune suppressive regulatory T cells. The ability of IL-12 to not only activate the adaptive and the innate immune systems, but also further modulate the otherwise immune-hostile tumor microenvironment makes IL-12 an ideal candidate for tumor immunotherapy.

IL-15 is capable of stimulating T cell proliferation inside tumors (*see, e.g*., Miecnik et al., Sci Transl Med 6(228):228ra37 (2014) as well as extend the survivability of effector memory CD8+ T cells and is critical for the development of NK cells. Thus, it is believed that IL-15 can boost the potency of checkpoint inhibitors and other immunotherapies that harness T cells to attack cancer cells. IL-15 monomer, however, has a short half-life of less than 40 minutes *in vivo.* Modifications to IL-15 monomer can improve its *in vivo* pharmacokinetics in the treatment of cancers. These modifications have generally centered on improving the *trans*-presentation of IL-15 with the alpha subunit of IL-15 receptor, IL-15Rα. Such modifications include: 1) pre-association of IL-15 and its soluble receptor a-subunit-Fc fusion to form IL-15: IL-15Rα-Fe complex (*see, e.g.,* Rubinstein et al., Proc Natl Acad Sci U.S.A. 103:9166-71 (2006)); 2) expression of the hyperagonists IL-15-sIL-15Rα-sushi protein (*see, e.g.,* Bessard et al., Molecular cancer therapeutics 8: 2736-45 (2009)); and 3) pre-association of human IL-15 mutant IL-15N72D with IL-15Rα-Fc sushi-Fc fusion complex (*see, e.g.,* Zhu et al., Journal of Immunology 183: 3598-6007 (2009)).

Monoclonal antibody based therapies, including those that target tumor surface antigens and inhibitory signals that limit T-cell activation, have been a standard component of cancer therapeutics for over 20 years. *See, e.g.,* Weiner, Nat Rev Cancer 15(6): 361-370 (2015).

Short circulatory half-life represents a major obstacle for many biologics, including cytokine and antibody based therapeutics. *See, e.g.,* Herrington-Symes et al., Advances in Bioscience and Biotechnology 4: 689-698 (2013) and Perdreau et al., European Cytokine Network 21: 297-307 (2010). Such short-acting therapeutics require frequent dosing profiles that can reduce applicability to the clinic, particular for chronic conditions. Long serum half-life is desirable as it would decrease the need for repetitive injections of the molecule to achieve a therapeutically relevant serum concentration. Methods of extending the half-life of therapeutic proteins include PEGylation, fusion to human serum albumin (HSA), fusion to the constant fragment (Fc) of a human immunoglobulin IgG, and fusion to non-structured fusion proteins such as XTEN. *See, e.g.,* Stohl, BioDrugs 29(4): 215-239 (2015). Half-life extension technologies enable improved or new biologic therapies that reduce the cost and burden of frequent dosing. Thus, there remains a continued need for novel reagents and methods that can extend the half-lives of protein and peptide based therapeutics.

Provided herein are novel albumin binding domain (ABD) fusion proteins, useful for extending the half-lives of biologics (*e.g*., interleukins and antibodies). Serum albumin possess a long half-life in the range of 2-4 weeks due to recycling through the neonatal Fc receptor (FcRn). Albumin is taken up by endothelial cells through macropinocytosis and binds to the FcRn in a pH-dependent manner in the acidic environment of the early endosome. Albumin-FcRn binding diverts albumin molecules from degradation in the lysosomal compartment and redirects the albumin molecules to the plasma membrane, where they are released back into the blood plasma due to the neutral pH.

Albumin binding domains (ABDs) described herein do not compete with FcRN for albumin binding and bind albumin at a pH range that allows for the ABD to also undergo FcRn-driven endosomal albumin recycling when bound to albumin. As such, biologics that include the subject albumin binding domain (ABD) are capable of evading lysosomal degradation using the albumin-FcRn pathway and, consequently, exhibit longer serum half-lives than counterparts lacking ABDs.

Moreover, such ABD containing therapeutics advantageously localize to tumors, which are known to contain high levels of serum albumin. Thus, such ABD containing therapeutics are particularly useful for the treatment of cancers.

### B. Albumin Binding Domains

In one aspect, provided herein are compositions that include an albumin binding domain. As used herein, "serum albumin" refers to a member of a family of globular proteins produced by the liver that functions primarily as a carrier protein of steroids, fatty acids, and thyroid hormones in the blood. Serum albumin also plays a major role in stabilizing extracellular fluid volume by contributing to oncotic pressure of plasma, and includes, but is not limited to, human serum albumin (HSA, Genbank Accession numbers: NM_000477 and NP_000468) and mouse serum albumin (MSA, Genbank accession numbers: NM_009654 and NP_0033784). The structure of albumin is characterized by several long α helices and contains eleven distinct binding domains for hydrophobic compounds. In humans, serum albumin is encoded by the ALB gene.

Fusion proteins that include the subject albumin binding domain (ABD) can bind serum albumin (SA), which allows the fusion protein to be taken in by cells by macropinocytosis. In certain embodiments, the ABDs described herein bind at a pH range of about pH 5.5 to about pH 7.2. In the early endosome, such SA bound ABD fusion proteins bind to FcRn via SA at an acidic pH (*e.g.,* pH 5.5), which in turn diverts the SA bound ABD fusion protein from the lysosome compartment of the cell and back to the plasma membrane. At the plasma membrane, the SA dissociates from FcRn due to the neutral pH (*e.g.,* pH 7.1-7.5) and the SA and ABD fusion protein are released back into the bloodstream. As therapeutics that include the subject ABD are capable of binding to albumin at a pH range of about pH 5.5 to about pH 7.2, such therapeutics advantageously also undergo FcRn-driven endosomal albumin recycling and, thus, evade lysosomal degradation. Accordingly, therapeutics that include such ABDs advantageously exhibit longer serum half-lives than counterparts lacking ABDs. Such therapeutics are particular useful for the treatment of cancers, which are known to contain high levels of serum albumin.

In some embodiments, the albumin binding domain binds albumin at a site that does not interfere with SA binding to a neonatal Fc receptor (FcRn). By "FcRn" or "neonatal Fc Receptor" as used herein is meant a protein that binds the IgG antibody Fc region and is encoded at least in part by an FcRn gene. The FcRn may be from any organism, including but not limited to humans, mice, rats, rabbits, and monkeys. As is known in the art, the functional FcRn protein comprises two fusion proteins, often referred to as the heavy chain and light chain. The light chain is beta-2-microglobulin and the heavy chain is encoded by the FcRn gene. Unless otherwise noted herein, FcRn or an FcRn protein refers to the complex of FcRn heavy chain with beta-2-microglobulin.

In some embodiments, the albumin binding domain described or exemplified herein preferably specifically binds to serum albumin (*e.g*., HSA) at an epitope on the serum albumin molecule that does not participate in the interaction of the serum albumin molecule with the FcRn. Binding of the SA binding moiety to the serum albumin molecule thus preferably does not substantially interfere with, inhibit, prevent, or otherwise reduce binding of the serum albumin molecule (*e.g*., IISA) with the FcRn. Preferably, the albumin binding domain does not compete with the FcRn for binding to the serum albumin molecule. Preferably, the albumin binding domain does not sterically inhibit binding of serum albumin to the FcRn. Preferably, the SA binding moiety does not change the conformation of the serum albumin molecule such that the albumin cannot interact with the FcRn.

In some embodiments, the albumin binding domain binds SA (*e.g*., HSA) at a pH of 5.0 ± 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0. 2.1, 2.2, .2.3, .2.4, .2.5, .2.6, 2.7, 2.8, 2.9, or 3.0. In some embodiments, the albumin binding domain binds SA at a range of pH of about pH 5.5- about pH 7.2. In some embodiments, the SA binding moiety binds SA at a pH of 5.5.

In certain embodiments, the albumin binding domain is a human serum albumin (HSA) binding domain. HSA binding domains include, but are not limited to, albumin binding domains that can bind to an HSA molecule such as a whole HSA molecule or a fragment of an HSA. In some embodiments, the HSA binding domain also binds mouse serum albumin. In some embodiments, the HSA binding domain also binds cyno monkey albumin. In certain embodiments, the HSA binding domain does not bind to bovine serum albumin (BSA).

Albumin binding domains provided herein can include a variable heavy chain alone or a variable heavy chain in association with a variable light chain. In some embodiments, the albumin binding domain includes a variable heavy chain. In certain embodiments, the variable heavy chain includes a vhCDR1, vhCDR2, and vhCDR3 (variable heavy chain Complementary Determining Regions 1-3). In certain embodiments, the antigen binding domain also includes a variable light chain. In certain embodiments, the variable light chain includes a vlCDR1, vlCDR2 and vlCDR3 (variable light chain Complementary Determining Regions 1-3).

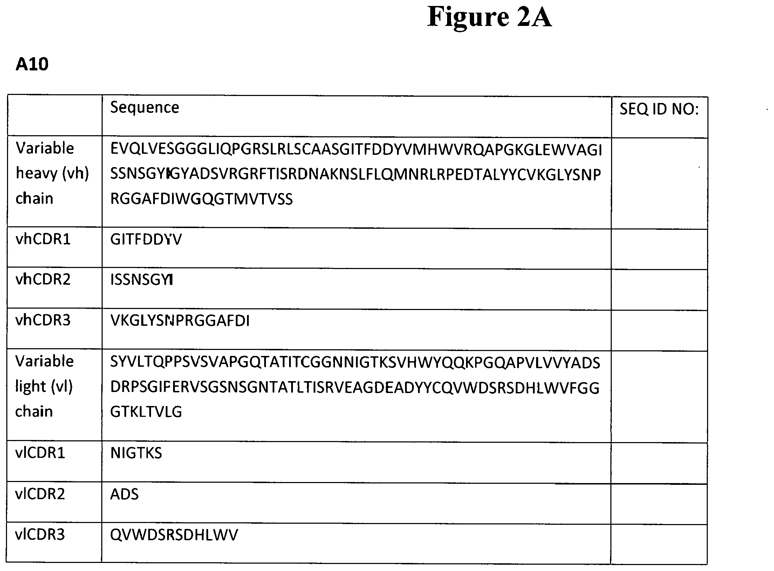

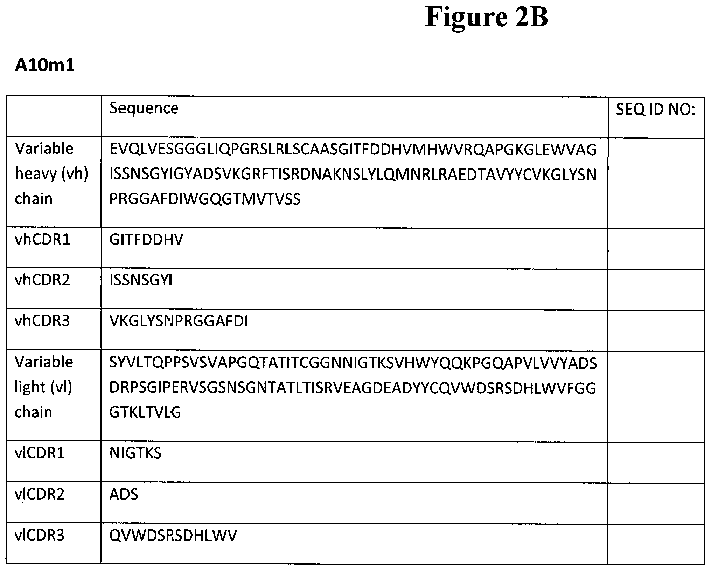

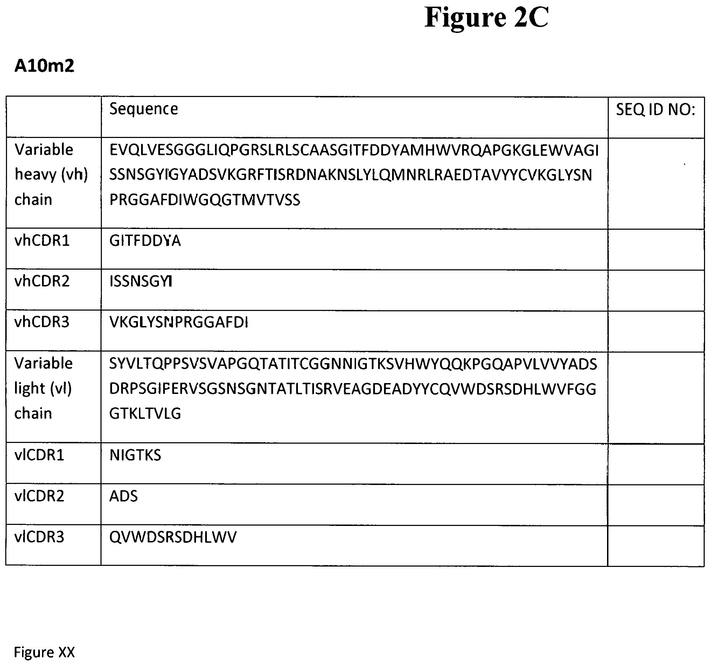

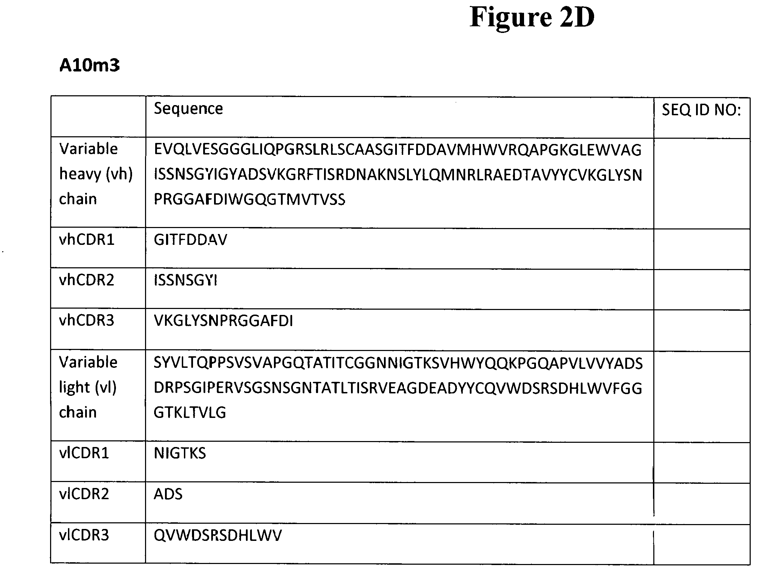

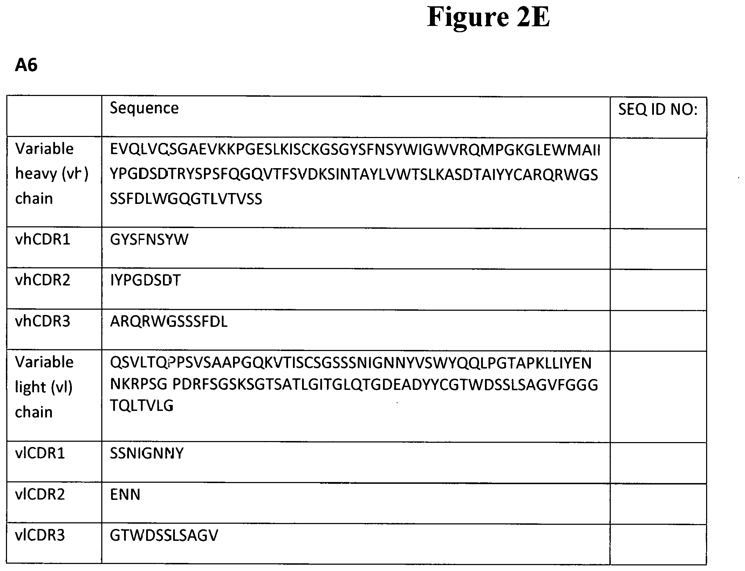

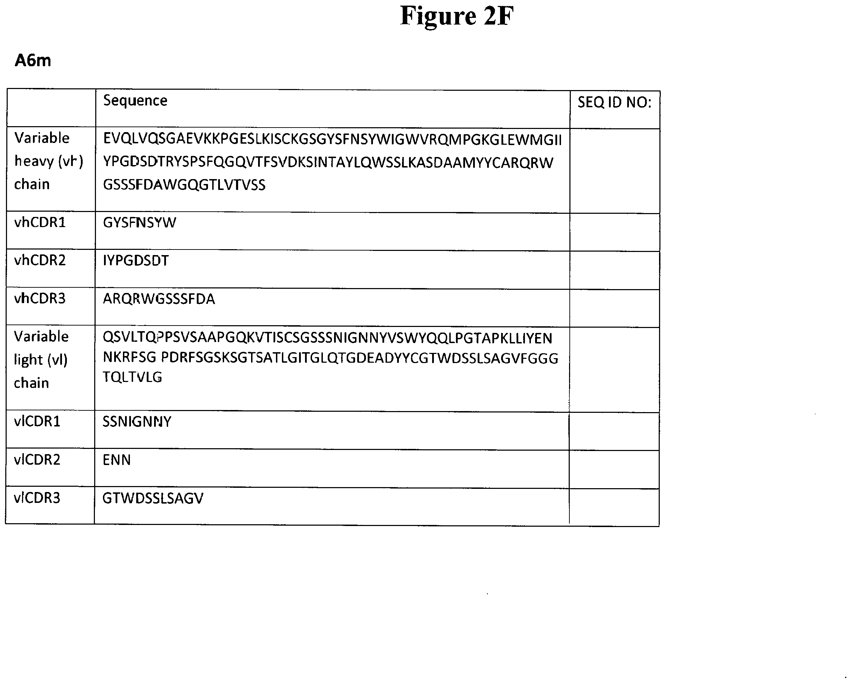

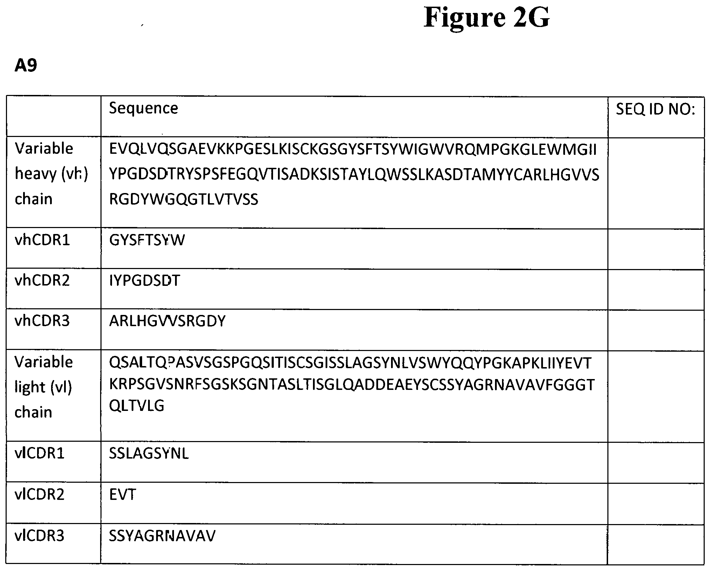

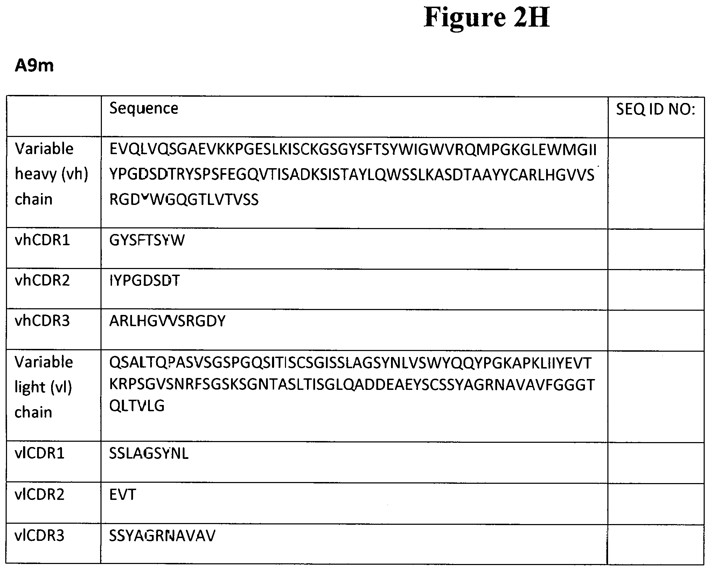

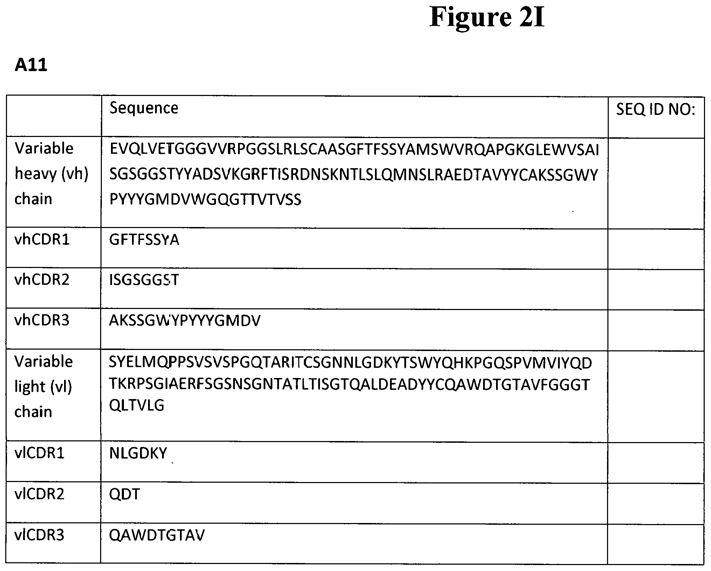

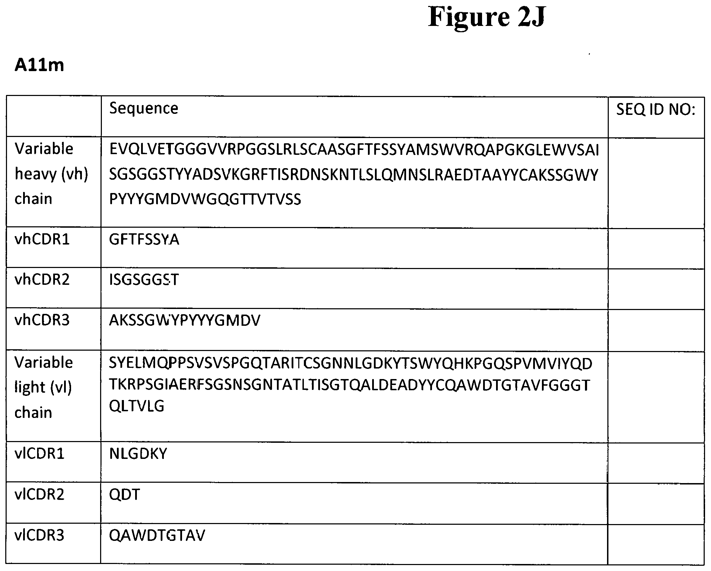

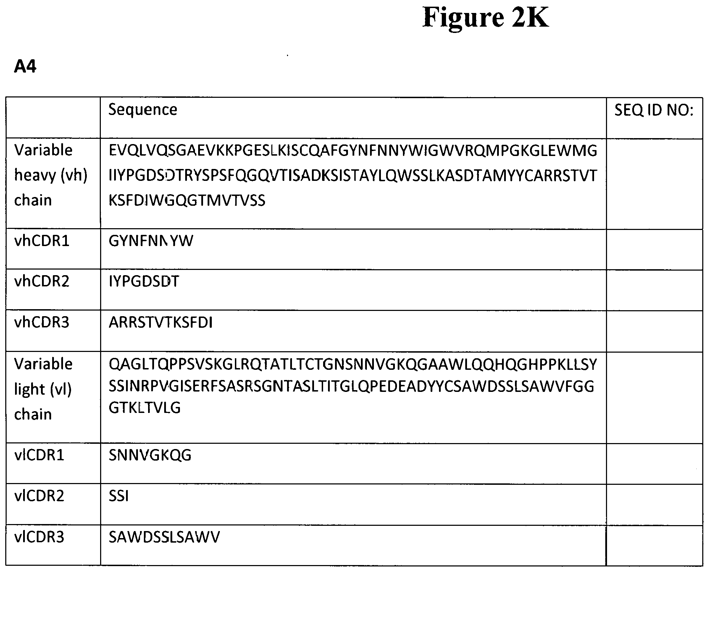

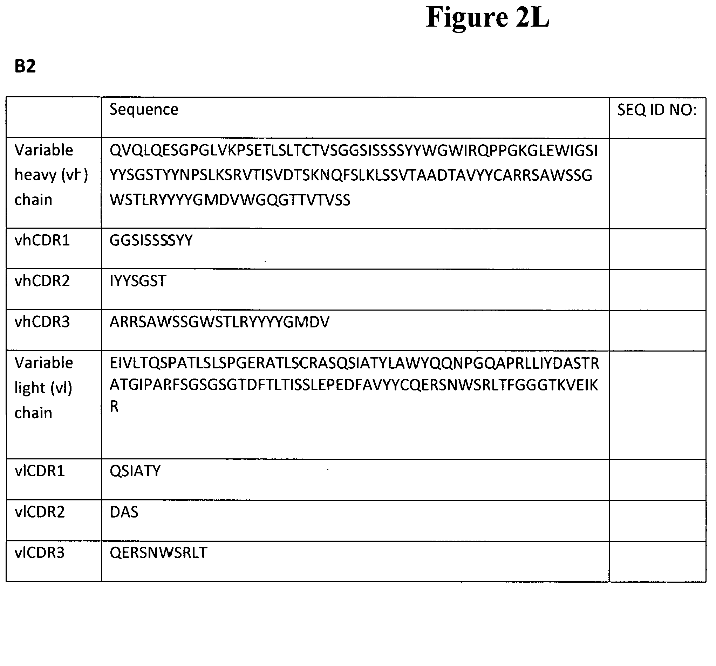

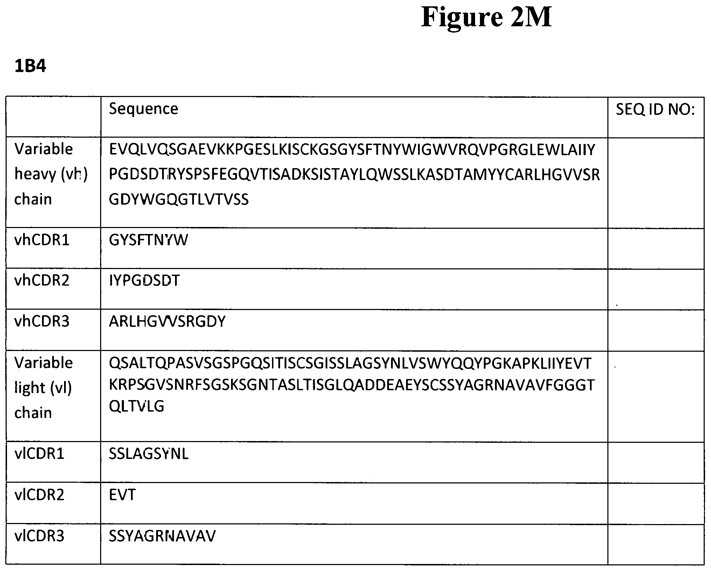

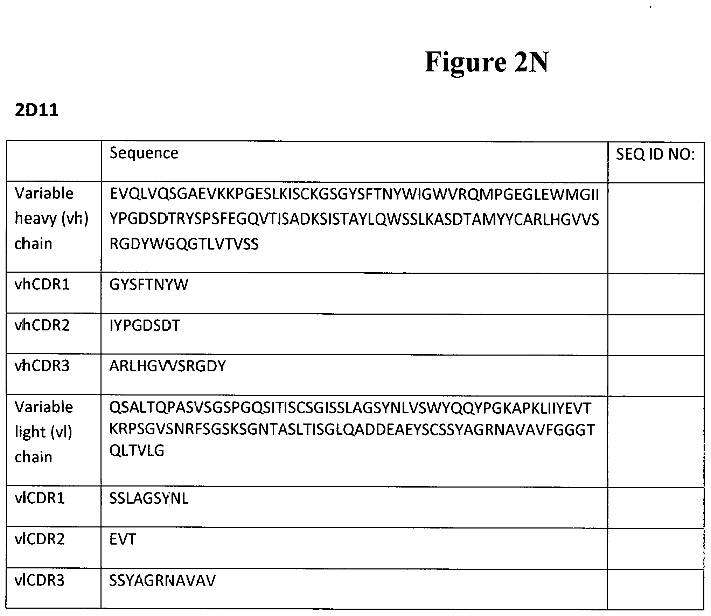

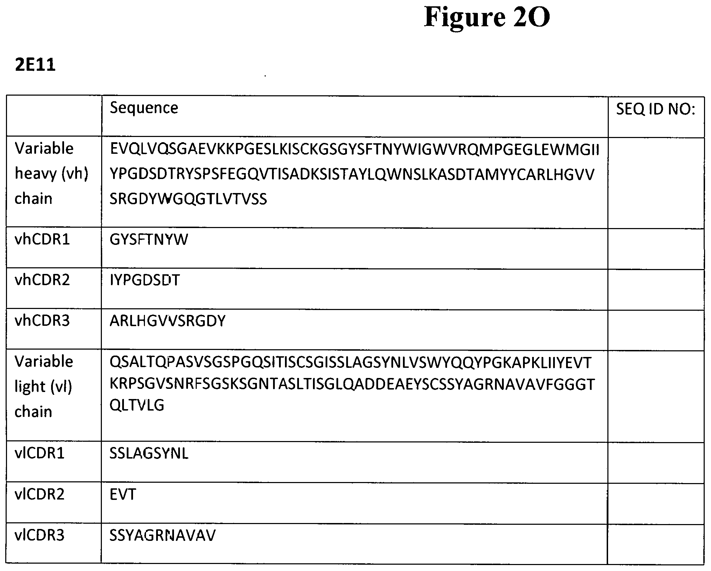

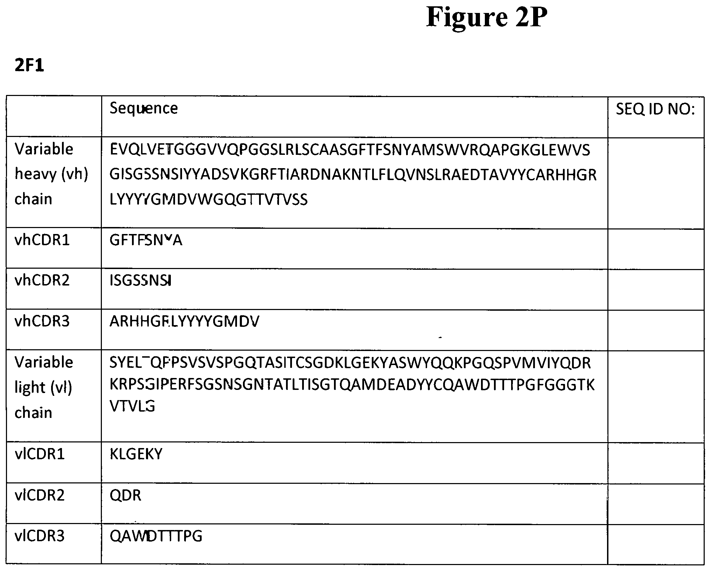

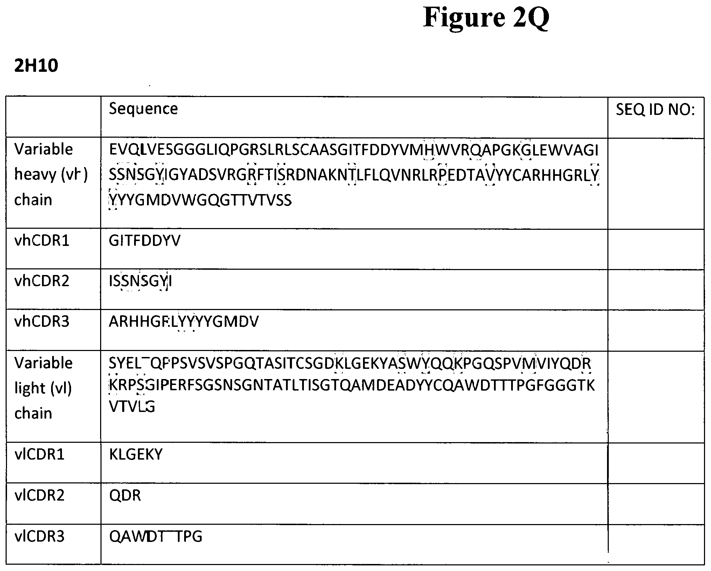

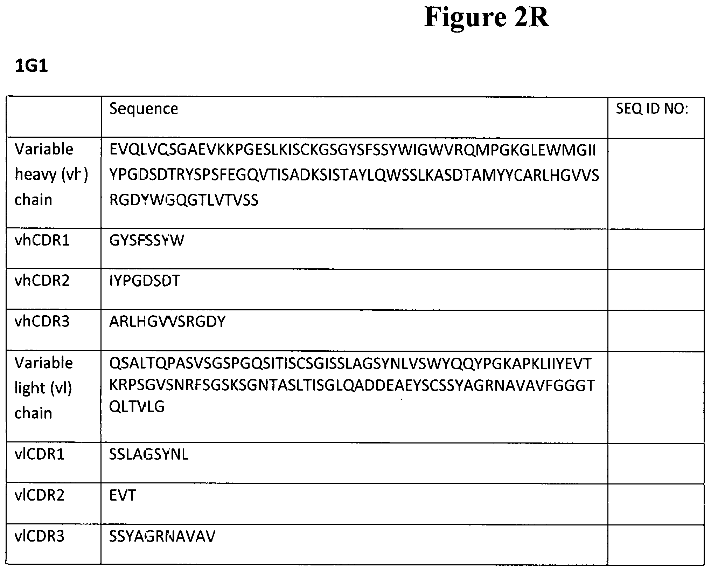

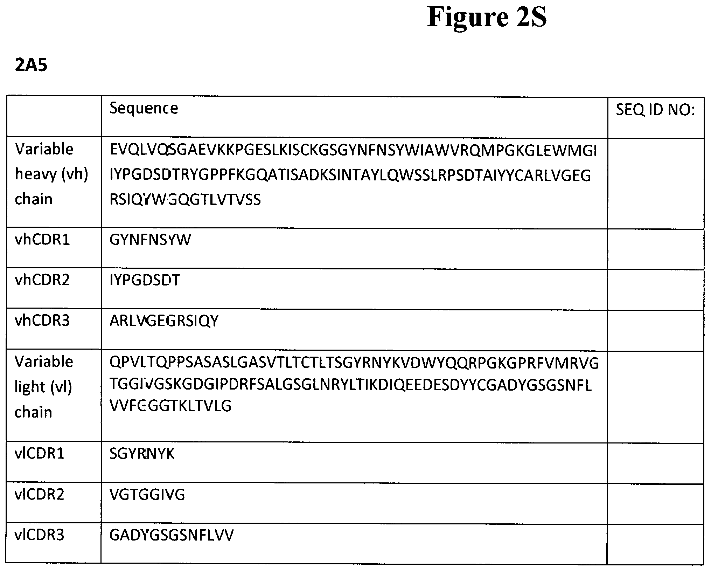

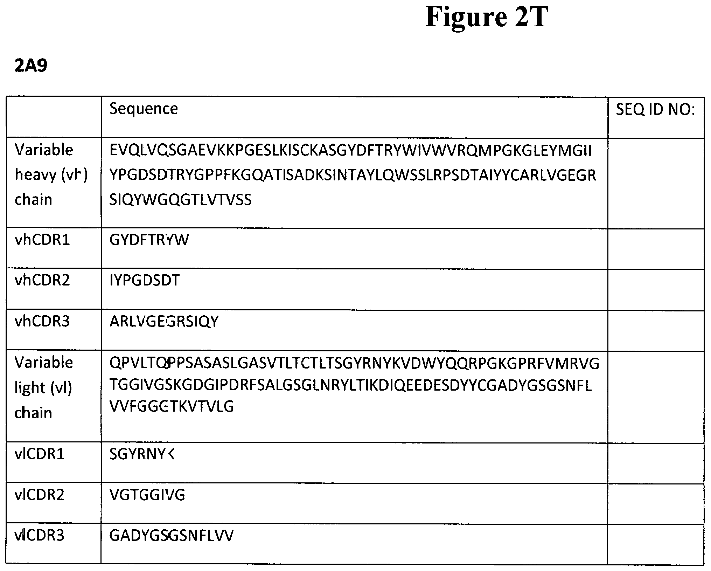

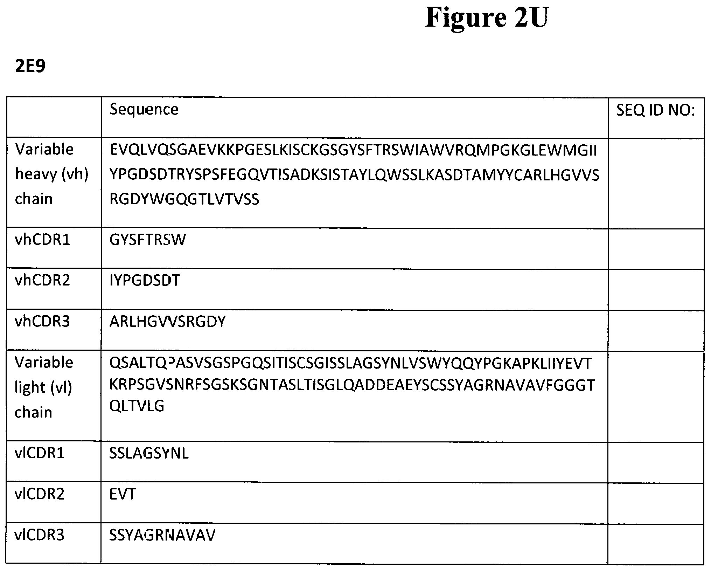

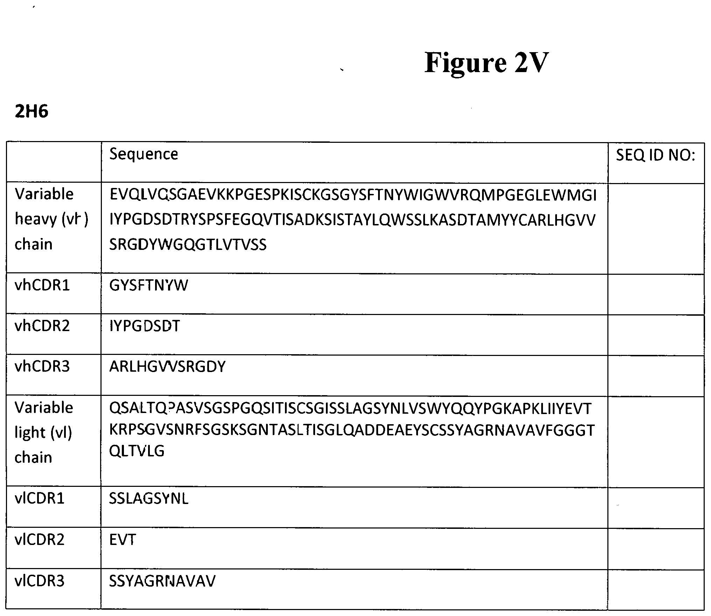

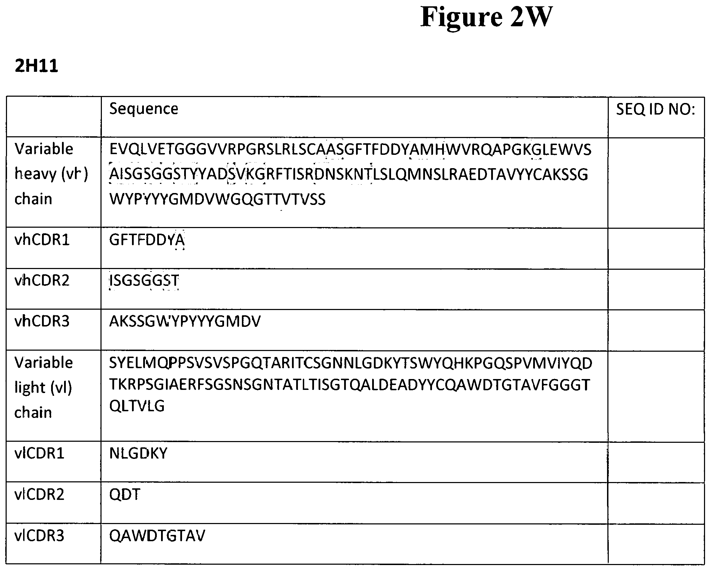

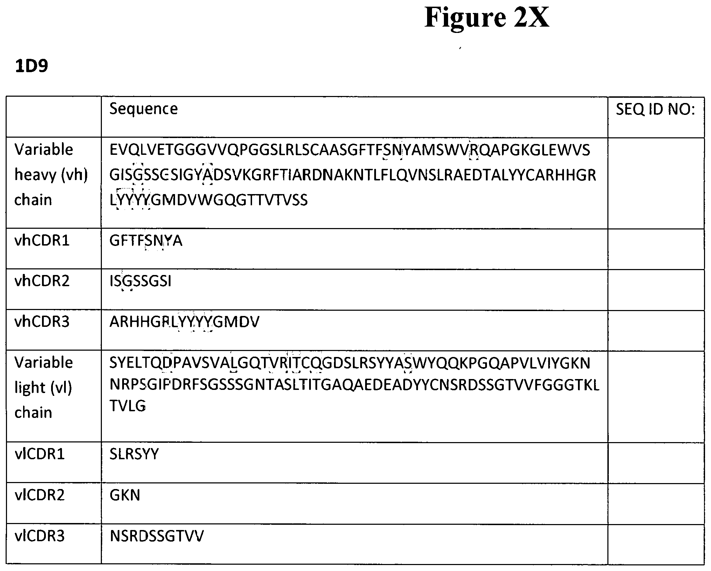

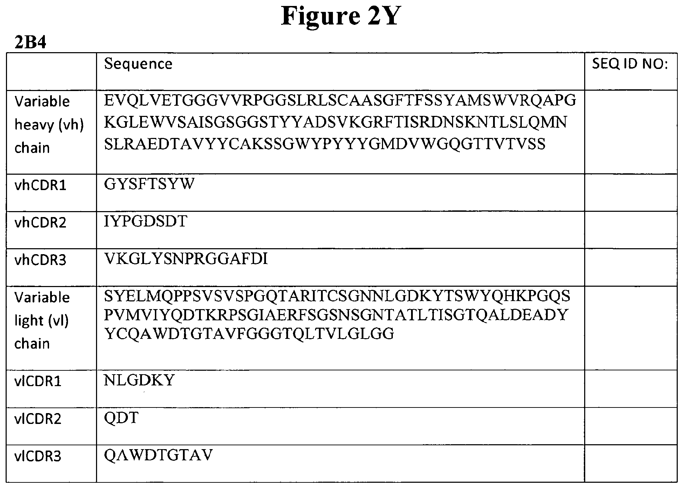

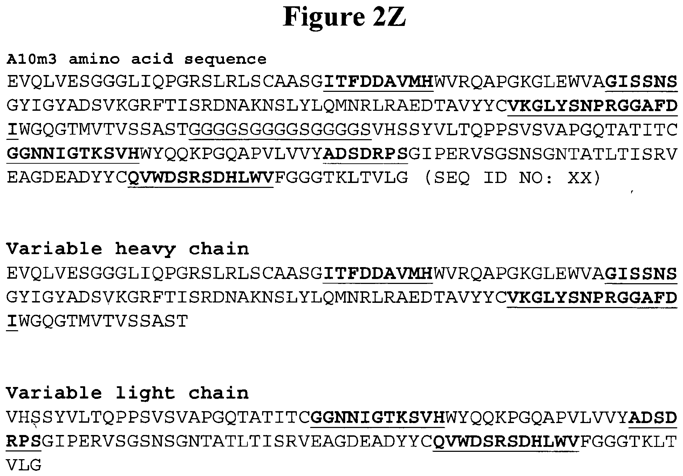

In some embodiments the albumin binding domain includes a variable heavy chain that includes the vhCDR1, vhCDR2, and vhCDR3 of any of the variable heavy chains depicted in Figure 2 In some embodiments, the albumin binding domain includes the vhCDR1, vhCDR2, and vhCDR3 of an A10m3 variable heavy chain, as shown in Figure 2D. In certain embodiments, the albumin binding domain includes the vhCDR1, vhCDR2, and vhCDR3 of A10m3 as shown in Figure 2D.

In certain embodiments the albumin binding domain also includes a variable light chain. In an exemplary embodiment, the albumin binding domain includes a variable light chain that includes the vlCDR1, vlCDR2, and vlCDR3 of any of the variable light chains depicted in Figure 2. In some embodiments, the albumin binding domain includes the vlCDR1, vlCDR2, and vlCDR3 of an A10m3 variable light chain, as shown in Figure 2D. In certain embodiments, the albumin binding domain includes the vlCDR1, vlCDR2, and vlCDR3 of A10m3 as shown in Figure 2D.

In certain embodiments, the albumin binding domain (*e.g*., HSA binding domain) is an antibody or an antibody fragment. In some embodiments, the albumin binding domain (*e.g*., HSA binding domain) is an scFv.

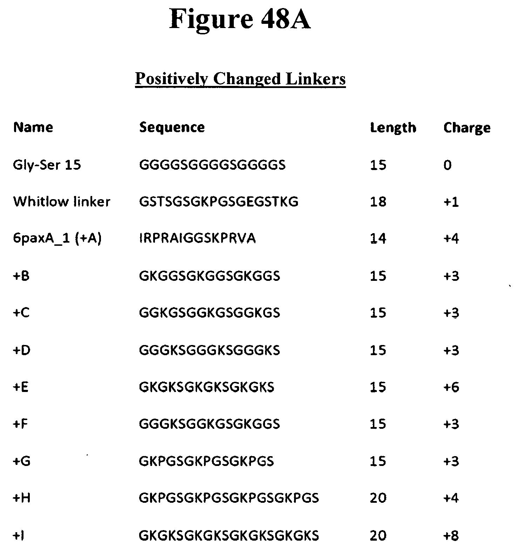

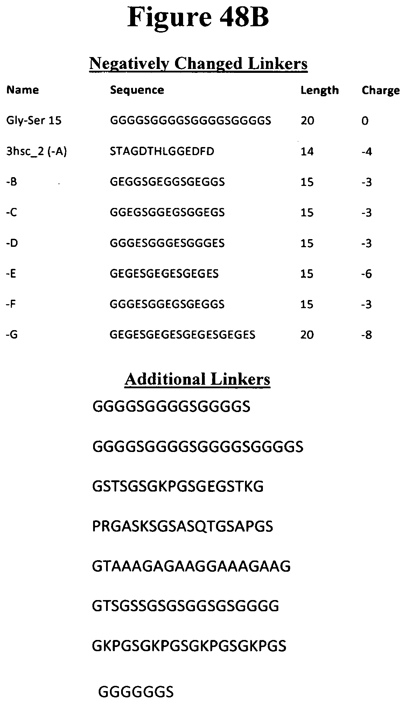

In some embodiments where the ABD includes both a variable heavy chain and a variable light chain, the variable heavy chain and the variable light chain are attached to each other by a linker (*e.g*., an scFv linker). In certain embodiments, the linker is attached to the variable heavy chain at its C-terminus and the variable light chain at its N-terminus. Suitable linkers are described herein and in Figure 48. In some embodiments, the linker is a (Gly4Ser)ₓ linker, where x is 1, 2, 3, 4, 5, 6, 7, or 8. In certain embodiments, the linker is a (Gly4Ser)₅ linker.

In certain embodiments, the albumin binding domain also includes a variable heavy chain that includes the vhCDR1, vhCDR2 and vhCDR3 of A10m3 and a variable light chain that includes the vlCDR1, vlCDR2 and vlCDR3 of A10m3 (Figure 2D). In one embodiment, the albumin binding domain includes the variable heavy sequence and variable light sequence of the A10m3 ABD depicted in Figure 2D.

### C. Interleukin-15 Variants

In another aspect, provided herein are compositions that include variant IL-15s with improved *in vivo* stability and/or biological activity as compared to wildtype IL-15.

As used herein, "interleukin 15", "IL-15" and "IL15" all refer to an interleukin that binds to and signals through a complex composed of an IL-15 specific receptor alpha chain, an IL-2/IL-15 receptor beta chain (CD122) and the common gamma chain (gamma-C, CD132) (Genbank Accession numbers: NM_00000585 and NP_000576 (human); and NM_001254747 and NP_001241676 (mouse)).

IL-15 has been shown to stimulate T cell proliferation inside tumors (*see, e.g*., Miecnik et al., *Sci Transl Med* 6(228):228ra37 (2014). IL-15 also is able to extend the survivability of effector memory CD8+ T cells and is critical for the development of NK cells. It is believed that IL-15 can boost the potency of checkpoint inhibitors and other immunotherapies that harness T cells to attack cancer cells. Therefore, without being bound by any particular theory of operation, it is believed that the IL-15s described herein are useful for the treatment of cancers.

IL-15 monomer, however, has a short half-life of less than 40 minutes *in vivo.* Modifications to IL-15 monomer can improve its *in vivo* pharmacokinetics in the treatment of cancers. These modifications have generally centered on improving the trans-presentation of IL-15 with the alpha subunit of IL-15 receptor, IL-15Rα. Such modifications include: 1) pre-association of IL-15 and its soluble receptor a-subunit-Fc fusion to form IL-15: IL-15Rα-Fe complex (*see, e.g.,* Rubinstein et al., Proc Natl Acad Sci U.S.A. 103:9166-71 (2006)); 2) expression of the hyperagonists IL-15-sIL-15Rα-sushi protein (*see, e.g.,* Bessard et al., Molecular cancer therapeutics 8: 2736-45 (2009)); and 3) pre-association of human IL-15 mutant IL-15N72D with IL-15Rα-Fc sushi-Fc fusion complex (*see, e.g.,* Zhu et al., Journal of Immunology 183: 3598-6007 (2009)).

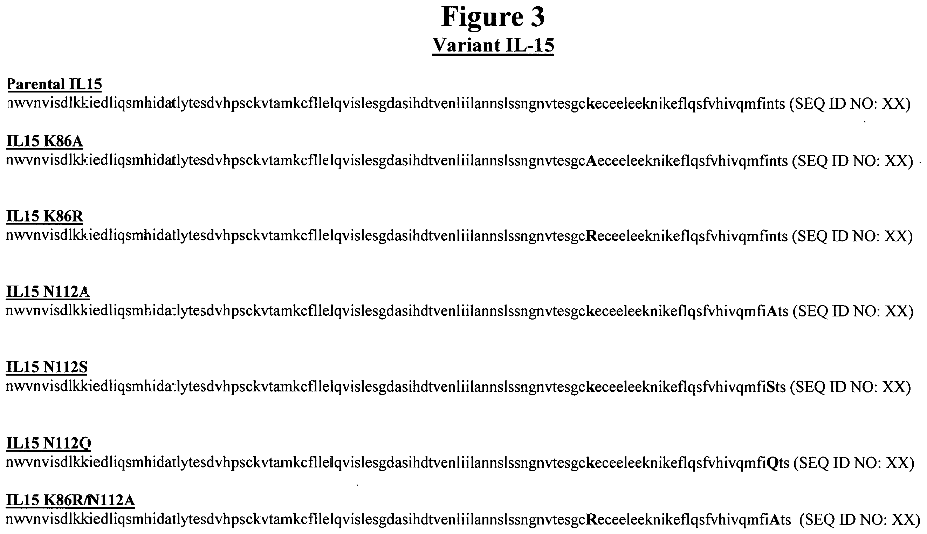

In some embodiments, the IL-15 is a variant of a parental IL-15 with increased stability as compared to wildtype IL-15. In particular embodiments, the variant IL-15 is a variant of a wildtype human IL-15. In an exemplary embodiment, the variant IL-15 includes an amino acid substitution at position K86 of the parental IL-15 shown in Figure 3. As described herein, K86 is a putative site for ubiquitin-dependent degradation (*See* Example 2) when made using particular cell types (*e.g*., HEK293 T cells). Therefore, without being bound by any particular theory of operation, it is believed that removal of the K86 ubiquitination site by amino acid substitution improves the stability of IL-15 (*See* Examples 2 and 3).

In certain embodiments, the IL-15 is a variant IL-15 having an amino acid substitution at position N112. Amino acid position N112 is a key site for IL-15 bioactivity, as it is critical for a proper IL-15/IL-15 receptor gamma interaction, particularly when IL-15 is attached to an ABD. Therefore, without being bound by any particular theory of operation, it is believed that mutations at position N112 can enhance one or more functions of IL-15 including, but not limited to, promoting T cell proliferation in tumor environments, enhancing survivability of CD8+ T cells and promoting NK cell development.

Particular amino acid substitutions that can improve IL-15 *in vivo* stability and/or biological activity include, but are not limited to: K86A, K86R, N112A, N112S, N112Q, K86A/N112A, K86R/N112A, K86A/N112S, K86R/N112S, K86A/N112Q, K86R/N112Q, K86A/N112A/N79A, K86R/N112A/N79A, K86A/N112A/N79D, K86R/N112A/N79D, K86A/N112A/N79Q, K86R/N112A/N79Q, K86A/N112A/N71D, K86R/N112A/N71D, K86A/N112A/N71Q, K86R/N112A/N71Q, K86A/N112A/N71D/N79A, K86A/N112A/N71D/N79D, K86A/N112A/N71Q/N79A, K86A/N112A/N71Q/N79D, K86R/N112A/N71D/N79A, K86R/N112A/N71D/N79D, K86R/N112A/N71D/N79Q, K86R/N112A/N71Q/N79A, K86R/N112A/N71Q/N79D, and K86R/N112A/N71Q/N79Q. Exemplary variant IL-15s that include one or more of such amino acid substitutions are depicted in Figure 3. The description describes that the variant IL-15 includes the amino acid substitutions K86A and N112A.

In one embodiment, the IL-15 described herein (wild-type and variant IL-15s) is attached to IL-15R alpha. Such IL-15 presented in trans with its receptor has been shown to have a prolonged half-life and higher potency as compared to native Il-15 alone. *See, e.g*., Wu, J Mol Genet Med 7, 85 (2013).

### D. IL-12

In another aspect, provided herein are compositions that include IL-12. As used herein, "interleukin 12", "IL-12" and "IL12" all refer to an interleukin that is a heterodimeric cytokine encoded by the IL-12A and IL-12B genes (Genbank Accession numbers: NM_000882 (IL-12A) and NM_002187 (IL-12B)). IL-12 is composed of a bundle of four alpha helices and is involved in the differentiation of native T cells into TH1 cells. IL-12 binds to the IL-12 receptor, which is a heterodimeric receptor formed by IL-12R-β1 and IL-12R-β2. IL-12 is known as a T cell-stimulating factor that can stimulate the growth and function of T cells. In particular, IL-12 can stimulate the production of interferon gamma (IFN-γ), and tumor necrosis factor-alpha (TNF-α) from T cells and natural killer (NK) cells and reduce IL-4 mediated suppression of IFN-γ. IL-12 can further mediate enhancement of the cytotoxic activity of NK cells and CD8+ cytotoxic T lymphocytes. Moreover, IL-12 can also have anti-angiogenic activity by increasing production of interferon gamma, which in turn increases the production of the chemokine inducible protein-10 (IP-10 or CXCL10). IP-10 then mediates this anti-angiogenic effect. Without being bound by any particular theory of operation, it is believed that IL-12 through its ability to induce immune responses and its anti-angiogenic activity can be used to treat cancers.

The description describes that the IL-12 is a mouse IL-12. In other embodiments, the IL-12 is a human 1L-12.

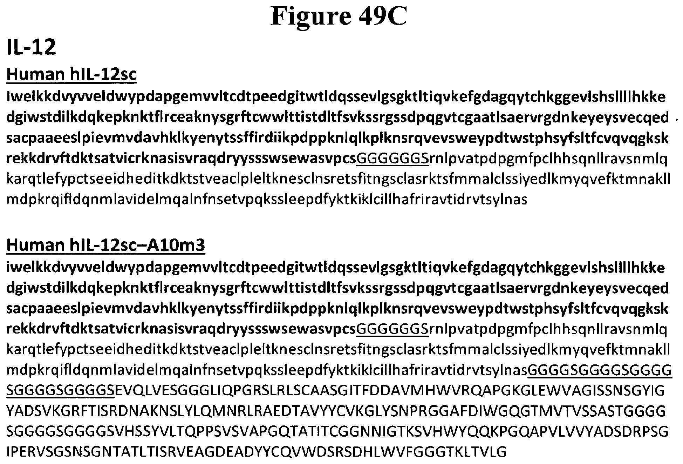

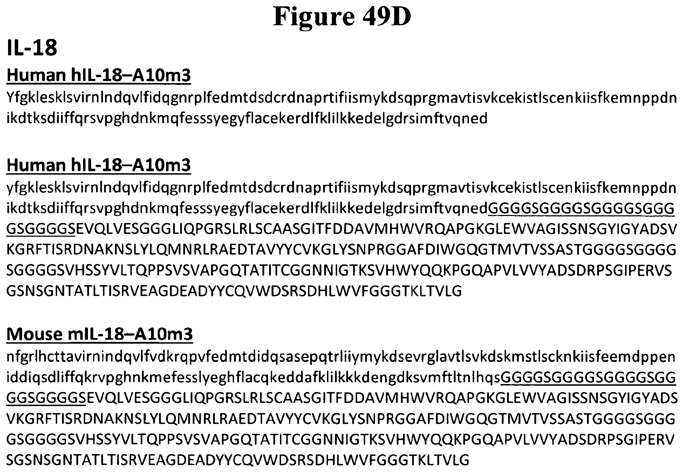

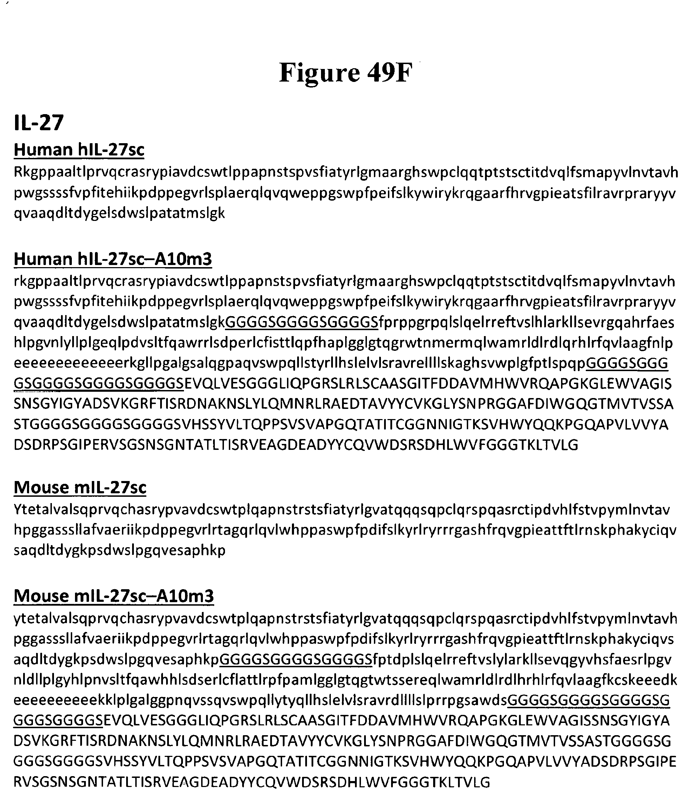

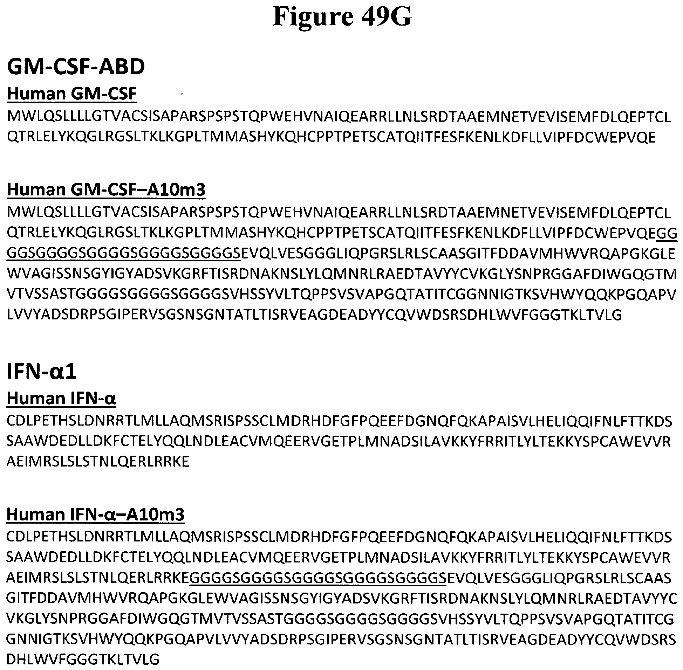

In certain embodiments, the IL-12 is a single chain IL-12 polypeptide comprising an IL-12 p35 subunit attached to an IL-12 p40 subunit. Such IL-12 single chain polypeptides advantageously retain one or more of the biological activities of wildtype IL-12. In some embodiments, the single chain IL-12 polypeptide described herein is according to the formula, from N-terminus to C-terminus, (p40)-(L)-(p35), wherein "p40" is an IL-12 p40 subunit, "p35" is IL-12 p35 subunit and L is a linker. In other embodiments, the single chain IL-12 is according to the formula from N-terminus to C-terminus, (p35)-(L)-(p40). Any suitable linker can be used in the single chain IL-12 polypeptide including those described herein and disclosed in Figure 49C. Suitable linkers can include, for example, linkers having the amino acid sequence (GGGGS)ₓ wherein x is an integer from 1-10. Other suitable linkers include, for example, the amino acid sequence GGGGGGS. Exemplary single chain IL-12 linkers than can be used with the subject single chain IL-12 polypeptides are also described in Lieschke et al., Nature Biotechnology 15: 35-40 (1997)
and particularly for its teaching of IL-12 polypeptide linkers.

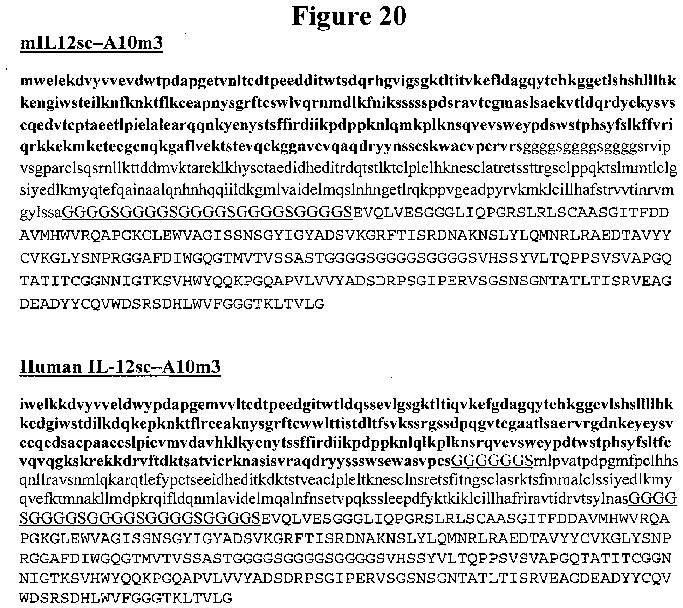

In an exemplary embodiment, the single chain IL-12 polypeptide is a single chain human IL-12 polypeptide (i.e., it includes a human p35 and p40 IL-12 subunit). The description describes that the single chain IL-12 polypeptide is a single chain mouse IL-12 polypeptide. Exemplary single chain human and mouse IL-12s are depicted in Figures 20 (shown as fusion peptide with ABD) and 49C.

### E. ABD fusion proteins

In one aspect, provided herein are ABD compositions that include an albumin binding domain attached to one or more fusion partners (*e.g*., a first fusion partner, a second fusion partner, etc.) via a linker. As discussed herein, subject ABD fusion proteins are able to under FcRn mediated endosomal recycling and, thus, advantageously exhibit extended half life compared to counterparts that do not include such ABDs.

ABDs useful for such ABD fusion proteins include, but are not limited to, those described herein. The amino acids sequences of such ABDs, including vhCDR1-3, vlCDR1-3, variable heavy chain and variable light chain sequences, are disclosed, for example, in Figure 2. In some embodiments, the ABD fusion protein includes a variable heavy chain that includes the vhCDR1, vhCDR2 and vhCDR3 of any of the ABD variable heavy chains in Figure 2, and a variable light chain that includes the vlCDR1, vlCDR2 and vCDR3 of any of the ABD variable light chains in Figure 2. In certain embodiments, the ABD includes a variable heavy chain having a vhCDR1, a vhCDR2 and a vhCDR3 of an ABD disclosed in Figure 2 and a variable light chain having a vhCDR1, a vhCDR2 and a vhCDR3 of an ABD disclosed in Figure 2. In some embodiments, the ABD fusion protein includes the variable heavy chain and variable light chain of an ABD disclosed in Figure 2.

In an exemplary embodiment, the ABD fusion protein includes a variable heavy chain that includes the vhCDR1, vhCDR2 and vhCDR3 of the A10m3 variable heavy chain, and a variable light chain that includes the vlCDR1, vlCDR2 and vCDR3 of the A10m3 variable light chain (Figure 2D). In certain embodiments, the ABD includes a variable heavy chain having a vhCDR1, a vhCDR2 and a vhCDR3 of A10m3 and a variable light chain having a vhCDR1, a vhCDR2 and a vhCDR3 of A10m3 (Figure 2D). In some embodiments, the ABD fusion protein includes the variable heavy chain and variable light chain of A10m3 (Figure 2D).

ABD fusion proteins described herein include a fusion partner. In some embodiments, the fusion partner include two fusion partners (a first fusion partner (FP1) and a second fusion partner (FP2). In embodiments that include two fusion partners, the fusion partners can be attached to the ABD in several orientations. In some embodiments, the ABD fusion protein is according to the formula, from N-terminus to C-terminus: FP1-ABD-FP2, FP1-PF2-ABD or ABD-FP1-FP2, wherein FP1 is a first fusion partner and FP2 is a second fusion partner.

Any suitable fusion partner wherein half-life extension of the fusion partner is desired, can be included in the subject ABD fusion proteins. Fusion partners may include, for example, cytokines (*e.g*., interferons and interleukins), growth factors, polypeptides, proteins, and hormones (*e.g*., growth hormones, parathyroid hormone).

In certain embodiments, the fusion partner is an antibody-based binding moiety that includes a variable heavy chain and a variable light chain. Such binding moieties can bind to any target of interest including, for example, tumor specific targets or cytokines. In an exemplary embodiment, the fusion partner is a single chain variable fragment (scFv). Antibody-based fusion partners also include, but are not limited to, ds-scFv, single domain antibodies (sdAb), diabodies, dsFvs, ds-scFvs, Fabs, and full length antibodies. Antibody-based fusion partners also include multispecific (*e.g*., bispecific) antibodies and fragments.

The subject ABD fusion proteins described herein utilize linkers between components (ABD and fusion partners) and within components. For example, scFv fusion partners utilize standard peptide linkers, generally based on glycine and serine, to attach the variant heavy and light chains to form the scFv. Further, standard peptide linkers are utilize to attached ABDs to fusion partners (*e.g*., cytokine fusion partners). Moreover, linkers are used to attach components of particular moieties, for example, the p35 and p40 subunit of IL-12 and IL-15 with IL-15Rα. In some embodiments, the linker is a (Gly4Ser)ₓ linker, where x is 1, 2, 3 ,4 6, 7 or 8. In particular embodiments, the ABD is connected to fusion partners by (Gly4Ser)₅ linkers.

As shown herein, there are a number of suitable linkers that can be used, including traditional peptide bonds, generated by recombinant techniques. The linker peptide may predominantly include the following amino acid residues: Gly, Ser, Ala, or Thr. The linker peptide should have a length that is adequate to link two molecules in such a way that they assume the correct conformation relative to one another so that they retain the desired activity. In one embodiment, the linker is from about 1 to 50 amino acids in length, preferably about 1 to 30 amino acids in length. In one embodiment, linkers of 1 to 20 amino acids in length may be used, with from about 5 to about 10 amino acids finding use in some embodiments. Useful linkers include glycine-serine polymers, including for example (GS)n, (GSGGS)n, (GGGGS)n, and (GGGS)n, where n is an integer of at least one (and generally from 3 to 4), glycine-alanine polymers, alanine-serine polymers, and other flexible linkers. Alternatively, a variety of nonproteinaceous polymers, including but not limited to polyethylene glycol (PEG), polypropylene glycol, polyoxyalkylenes, or copolymers of polyethylene glycol and polypropylene glycol, may find use as linkers, that is may find use as linkers.

Other linker sequences may include any sequence of any length of CL/CH1 domain but not all residues of CL/CH1 domain; for example the first 5-12 amino acid residues of the CL/CH1 domains. Linkers can be derived from immunoglobulin light chain, for example Cκ or Cλ. Linkers can be derived from immunoglobulin heavy chains of any isotype, including for example Cγ1, Cy2, Cy3, Cγ4, Cα1, Cα2, Cδ, Cε, and Cµ. Linker sequences may also be derived from other proteins such as Ig-like proteins (*e.g*., TCR, FcR, KIR), hinge region-derived sequences, and other natural sequences from other proteins.

Often the linker is not substantially sensitive to the extracellular environment. As used herein, "not substantially sensitive to the extracellular environment," in the context of a linker, means that no more than about 20%, 15%, 10%, 5%, 3%, or no more than about 1% of the linkers, in a sample of antibody-drug conjugate compound, are cleaved when the antibody-drug conjugate compound presents in an extracellular environment (for example, in plasma).

Whether a linker is not substantially sensitive to the extracellular environment can be determined, for example, by incubating with plasma the antibody-drug conjugate compound for a predetermined time period (for example, 2, 4, 8, 16, or 24 hours) and then quantitating the amount of free drug present in the plasma.

In other, non-mutually exclusive embodiments, the linker promotes cellular internalization. In certain embodiments, the linker promotes cellular internalization when conjugated to the therapeutic agent (that is, in the milieu of the linker-therapeutic agent moiety of the antibody-drug conjugate compound as described herein). In yet other embodiments, the linker promotes cellular internalization when conjugated to both the auristatin compound and the ABD fusion proteins of the invention.

A variety of exemplary linkers that can be used with the present compositions and methods are described in WO 2004-010957, U.S. Publication No. 2006/0074008, U.S. Publication No. 20050238649, and U.S. Publication No. 2006/0024317.

Exemplary linkers that can be utilized with the subject ABDs as domain linkers, scFv linkers, as well as attach components of particular fusion partners are further depicted in Figure 48.

In some embodiments, the linker is a "domain linker", used to link any two domains as outlined herein together (*e.g.,* a cytokine fusion partner (*e.g.,* an interleukin) and an ABD). While any suitable linker can be used, many embodiments utilize a glycine-serine polymer, including for example (GS)n, (GSGGS)n, (GGGGS)n, and (GGGS)n, where n is an integer of at least one (and generally from 3 to 4 to 5) as well as any peptide sequence that allows for recombinant attachment of the two domains with sufficient length and flexibility to allow each domain to retain its biological function.

In some embodiments, the fusion partner is an scFv that includes a variable heavy chain and a variable light chain. In such embodiments, the ABD variable heavy chain is attached to the variable light chain with an scFv linker.

Exemplary ABD fusion proteins are further discussed below.

### 1. Cytokine-ABD fusion proteins

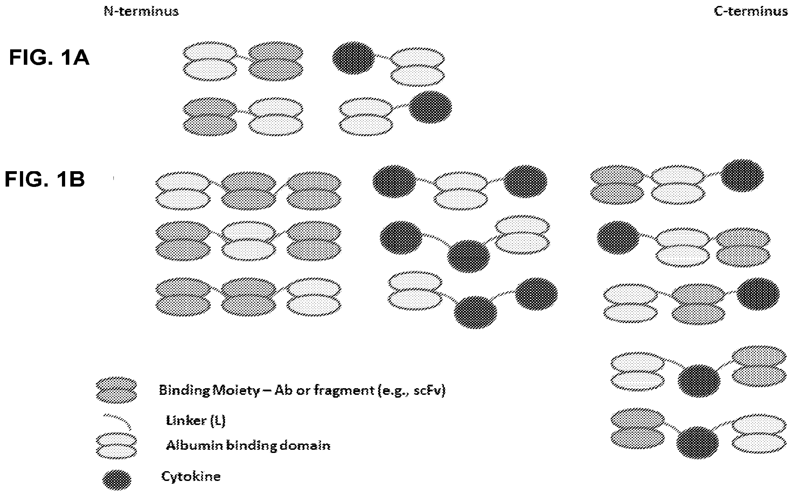

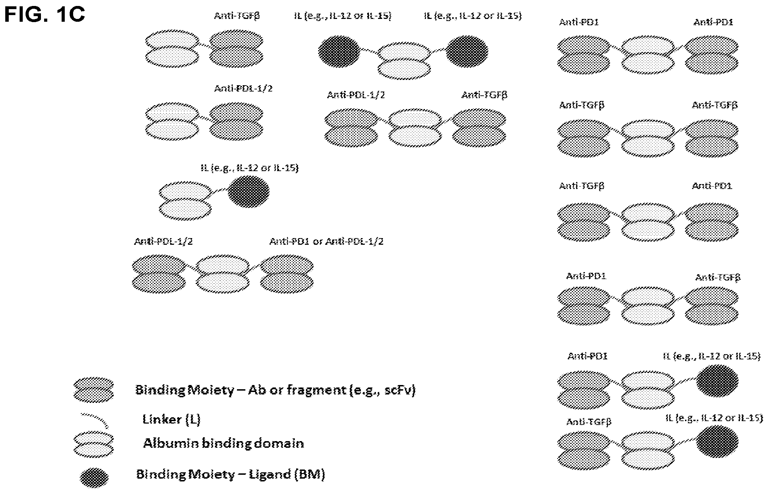

In some embodiments, the ABD fusion protein includes a cytokine fusion partner, i.e., a cytokine-albumin binding domain (cytokine-ABD) fusion proteins (Figure 1). In some embodiments, the cytokine-ABD fusion protein includes an IL-12,or IL-12/IL-15.

Subject cytokine-ABD fusion proteins are useful for immunomodulatory effects in a subject where such immunomodulatory effects are needed (*e.g*., treatment of a cancer or an autoimmune disease). Moreover, subject cytokine-ABDs exhibit a longer half-life and improved pharmacokinetic properties as compared to interleukin therapeutics alone.

Any ABD can be used with the subject cytokine-ABD fusion proteins described herein. In some embodiments, the ABD includes a ABD variable heavy chain. In certain embodiments the ABD includes a ABD variable light chain. In exemplary embodiments, the ABD is an scFv that includes a variable heavy chain attached to a variable light chain by a linker (*e.g*., any one of the linkers disclosed herein and in Figure 48).

In certain embodiments, the variable heavy chain includes the vhCDR1-3 of any of the ABD variable heavy chains described herein, including those ABD variable heavy chains depicted in Figure 2. In certain embodiments, the ABD variable heavy chain includes the vhCDR1-3 of the A10m3 variable heavy chain (Figure 2D). In some embodiments, the ABD variable heavy chain includes the A10m3 vhCDR1-3 as depicted in Figure 2D. In exemplary embodiments, the ABD variable heavy chain has the amino acid sequence of A10m3 variable heavy chain.

In certain embodiments, the ABD includes a ABD variable light chain. In some embodiments, the ABD variable light chain includes the vlCDR1-3 of any of the ABD variable light chains described herein, including those ABD variable light chains depicted in Figure 2. In certain embodiments, the ABD variable light chain includes the vlCDR1-3 of the A10m3 variable light chain (Figure 2D). In some embodiments, the ABD variable light chain includes the A10m3 vlCDR1-3 as depicted in Figure 2D. In exemplary embodiments, the ABD variable light chain has the sequence of A10m3 variable light chain.

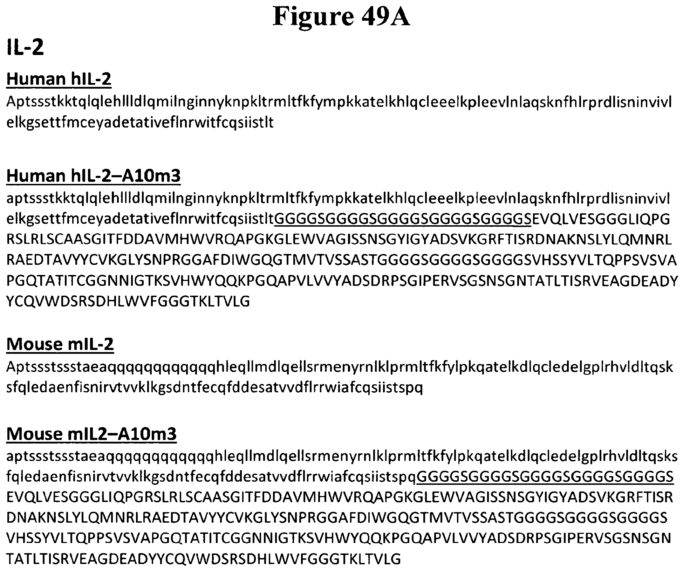

The amino acid sequences of exemplary cytokines that can be used in the subject cytokine-ABD fusions, as well as exemplary cytokine-ABD fusion proteins, where the ABD is A10m3 are depicted in Figure 49A-G.

In some embodiments, the IL-ABD is according to the formula, from N-terminus to C-terminus, cytokine-L-ABD or ABD-L-cytokine, where L is a linker that attaches the cytokine to the ABD (*e.g.,* a peptide linker). In certain embodiments, the cytokine -ABD includes a variable heavy chain and the cytokine is attached to the N-terminal of the ABD variable heavy chain. In some embodiments, the cytokine is attached to the C-terminal of the ABD variable heavy chain. In an exemplary embodiment, the interleukin-ABD includes an ABD that also includes a variable light chain (*e.g*., an ABD scFv). In some embodiments, the cytokine is attached to the C-terminal of the variable light chain. In certain embodiment, the N-terminal of the cytokine is attached to the ABD. In other embodiments, the C-terminal of the cytokine is attached to the ABD.

In certain embodiments, the cytokine is cytokine selected from IL-12, or IL-12/IL-15. Cytokine molecules include, for example, full length cytokine and cytokine fragments, for example, a part that is important for the particular cytokine's function (*e.g*., a part of the interleukin that binds to its receptor).

The cytokine-ABD includes an IL-2 molecule or fragment thereof. As used herein, "interleukin 2," "IL-2,", and "IL2" are refer to a member of the cytokine having four alpha helix bundles and signals through the IL-2 receptor (Genbank Accession numbers: NM_000586 and NP_000577 (human) and NM_008366 and NP_032392 (mouse)). IL-2 plays key roles in immune system function, tolerance and immunity, primarily via its direct effects on T cells. In the thymus, IL-2 prevents autoimmune diseases by promoting the differentiation of certain immature T cells into regulatory T cells, which kill off other T cells that are primed to attack normal healthy cells in the body. IL-2 has been used for the treatment of cancers (malignant melanoma, renal cell cancer) in large intermittent doses and has been extensively used in continuous doses. Exemplary IL-2-ABDs are shown in Figure 49A.

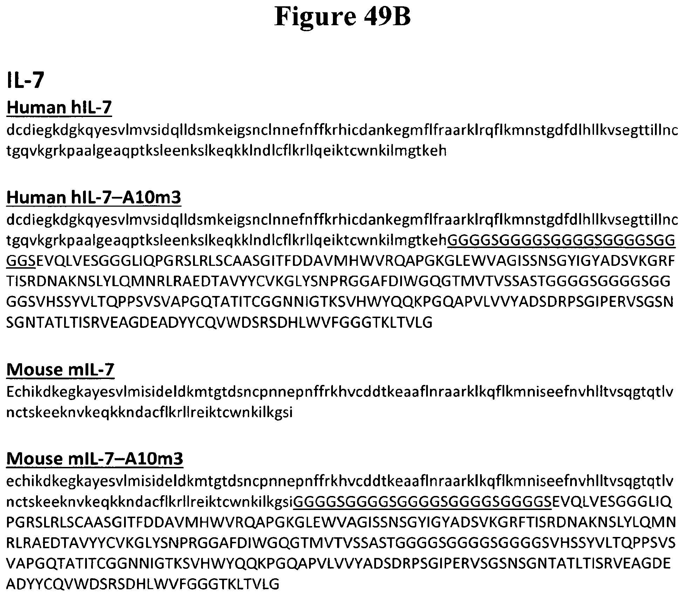

The cytokine-ABD fusion protein includes an IL-7 molecule or fragment thereof. As used herein, "interleukin 7" "IL-7", and "IL7" (Genbank Accession numbers: NM_000880 and NP_000871 (human); and NM_008371 and NP_032397 (mouse)) all refer to a member of a cytokine that is a hematopoietic growth factor secreted by stromal cells in the bone marrow and thymus and binds to IL-7 receptor. Interleukin-7 (IL-7) is a non-hematopoietic cell-derived cytokine with a central role in the adaptive immune system. It promotes lymphocyte development in the thymus maintains survival of naive and memory T cell homeostasis in the periphery. Moreover, it is important for the organogenesis of lymph nodes (LN) and for the maintenance of activated T cells recruited into the secondary lymphoid organs (SLOs). IL-7 is an ideal solution for the immune reconstitution of immunosuppressed cancer patients by promoting peripheral T cell expansion. In animal models, IL-7 has been proven to prolong the survival of tumor-bearing hosts. Exemplary IL-7-ABDs are shown in Figure 49B.

In certain embodiments, cytokine-ABD includes a an IL-12 molecule or fragment thereof. In some embodiments, the IL-12 is a single chain IL-12 as described herein and as depicted in Figures 20 (shown as a fusion peptide with ABD) and 49C.

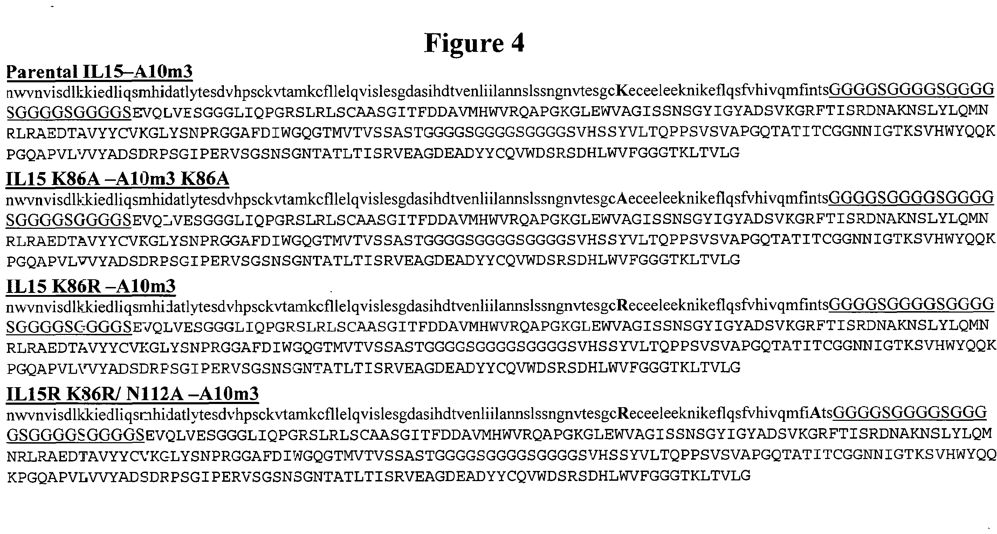

In certain embodiments, the cytokine-ABD includes an IL-15 molecule or fragment thereof. In some embodiments, the IL-15-ABD includes a variant IL-15 as described herein and at Figures 3 and 4. In some embodiments, the IL-15 is a wildtype IL-15, as shown in Figure 3 (*e.g*., "parental IL-15"). In certain embodiments, the wild-type IL-15 is attached to an IL-15 receptor alpha (IL-15Rα).

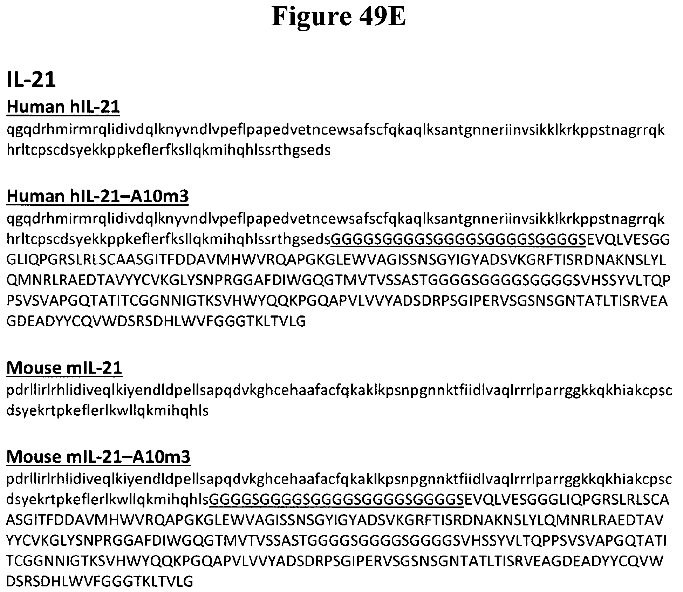

The cytokine-ABD includes an IL-21 molecule or fragment thereof. As used herein, "interleukin 21" "IL-21", and "IL21" (Genbank Accession numbers: NM_001207006 and NP_001193935 (human); and NM_0001291041 and NP_001277970 (mouse)) all refer to a member of a cytokine that binds to IL-21 receptor and has potent regulatory effects on cells of the immune system, including natural killer (NK) cells and cytotoxic cells and binds to IL-21 receptor that can destroy virally infected or cancerous cells. Therefore, without being bound by any particular theory of operation, it is believed that the subject cytokine-ABD having an IL-21 are useful for the treatment of various cancers. Exemplary IL-21-ABDs are shown in Figure 49E.

Other useful cytokines that may be included in the subject cytokine-ABDs includes, not are not limited to IL-27, IFN-α and GM-CSF. In certain embodiments, the interleukin-ABD polypeptide also includes an interferon-alpha, interferon-beta or GM-CSF molecule. Such molecules are useful for delivery into tumors and reduction of toxicity.

In some embodiments, the cytokine-ABD includes two cytokines attached to an ABD selected from the following: IL-2 and IL-12; IL-15 and IL-12; and GMC-SF and IL-12.

Figure 1 depicts several exemplary orientations in which two cytokines can be attached to the ABD. Any ABD can be used in such fusion protein, including those that include any of the ABD variable heavy domains and variable light domains shown in Figure 2. In some embodiments, the ABD is A10m3.

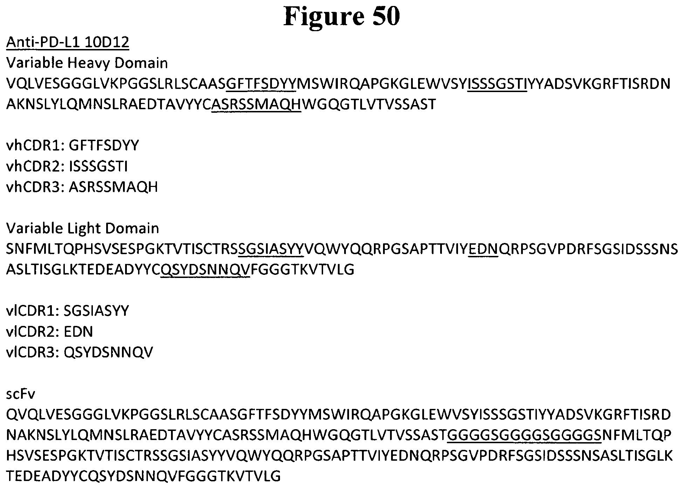

Any linker can be used to attached each cytokine to the ABD, including any linker depicted in Figure 48. An exemplary backbone linker-A10m3-linker sequence for use in such fusion proteins is depicted in Figure 50. Exemplary linkers than can be used include (GGGGS)ₓ linkers wherein X is 1-10. In certain embodiments, a (GGGGS)₅ is used to attach the cytokine to the ABD.

In some embodiments, the cytokine-ABD is according to a formula selected from the following: (from N-terminus to C-terminus): Cytokine 1-L1-ABD-L2-Cytokine 2; Cytokine 1-L1-Cytokine 2-L2-ABD; and ABD-L1-Cytokine 1-L2- Cytokine 2, where L1 and L2 are linkers that connect the cytokines and ABD components and Cytokine 1 and Cytokine 2 are selected from the following cytokine pairs: IL-2 and IL-12; IL-15 and IL-12; and GM-CSF and IL-12. In some embodiments, the ABD is A10m3 (Figure 2D).

In such cytokine-ABDs, either cytokine can be "Cytokine 1" and the other cytokine can be "Cytokine 2". For example, in one embodiment of the IL-2 and IL-12 cytokine pair, IL-2 is "Cytokine 1" and IL-12 is "Cytokine 2". In another embodiment of the IL-2 and IL-12 cytokine pair, IL-12 is "Cytokine 1" and IL-2 is "Cytokine 2".

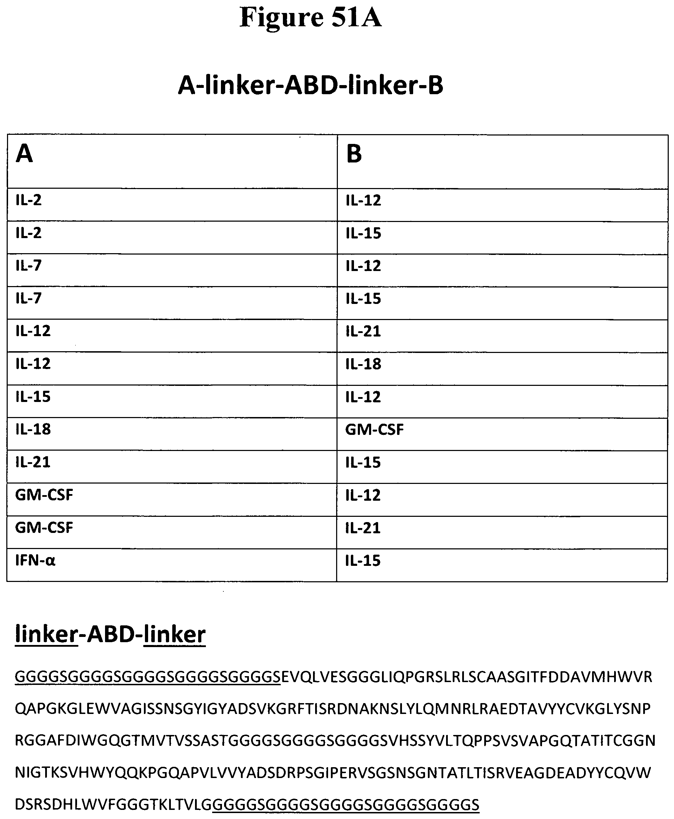

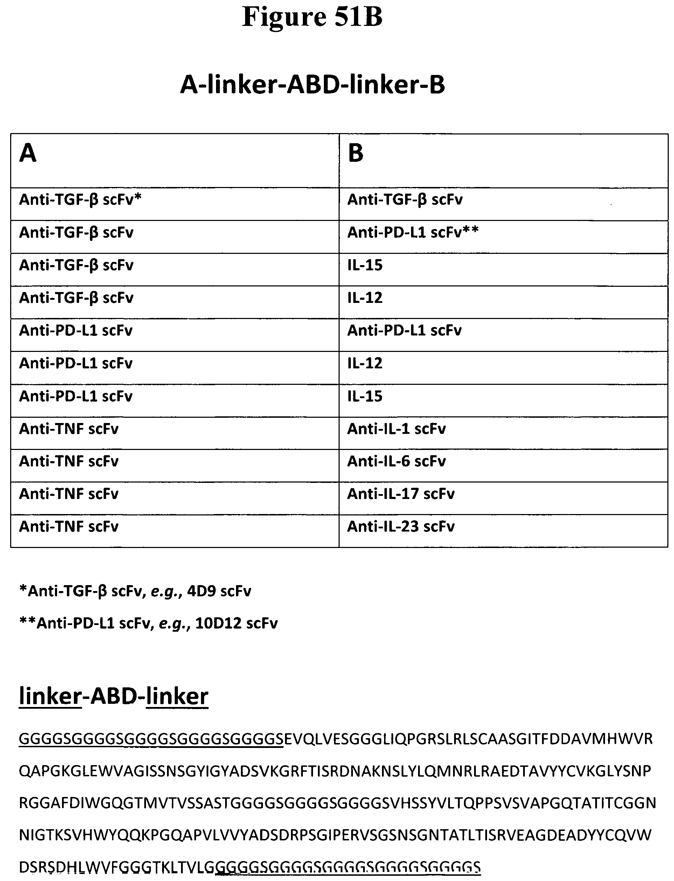

In some embodiments, the cytokine-ABD include two of the same interleukins. In other embodiments, the interleukin-ABD polypeptide includes two different interleukins (*e.g*., an IL-12 and an IL-15), as described above. ABD fusion proteins that include particular cytokine combinations are disclosed in Figure 51. Exemplary cytokine-ABD fusion proteins that include a combination of different cytokines are further discussed in detail below.

### a. IL-12 and IL-15

In certain embodiments, the cytokine-ABD fusion protein includes an IL-12 and an IL-15. Exemplary IL-12 and IL-15 sequences that can be included in such embodiments are depicted in Figures 3 and 49C. It is believed that cytokine-ABDs that include both an IL-12 and IL-15 fusion partner exhibit the anti-tumor effects of both interleukins. The combination of IL-12 and IL-15 was shown to induce enhanced anti-tumor activity as compared to either cytokine alone. Such enhanced anti-tumor activity was correlated with the reciprocal upregulation of each cytokine's receptors through the synergistic induction of IFN-γ. IL-12 in combination with IL-15 was further shown to promote anti-tumor activity in peritoneal macrophages through the synthesis of nitric oxide. Without being bound by any particular theory of operation, it is believed that polypeptides having both an IL-12 and an IL-15 fusion partner are capable of rapidly activate the innate response (IL-12) as well as potently stimulate the proliferation of T cells and maintain memory CD8+ T cells (IL-15). Animal studies show that sequential delivery of IL-12 and IL-15 expressing cells also cured mice in a therapeutic setting of established tumor. Depleting CD8+ cells eliminated the protection of this therapy, suggesting clonal expansion of tumor CTL. *See, e.g.,* Croce et al., Clin Cancer Res 11(2 Pt 1):735-742 (2005).

In some embodiments, the cytokine-ABD is according to the formula, from N-terminus to C-terminus:
a) (IL-12)-L1-(ABD)-L2-(IL-15); or
b) (IL-15)-L1-(ABD)-L2-(IL-12).

Any suitable ABD can be used including, for example, an ABD having a ABD variable heavy chain that includes the vhCDR1-3 of any of the ABD variable heavy chains listed in Figure 2. In an exemplary embodiment, the variable heavy chain includes the vhCDR1-3 of a A10m3 variable heavy chain (Figure 2D). In certain embodiments, the ABD includes a ABD variable light chain that includes the vlCDR1-3 of a A10m3 variable light chain. In an exemplary embodiment, the ABD is an A10m3 scFv.

Any suitable IL-12 and IL-15 can be used. In some embodiments, the IL-15 is a variant IL-15 as described herein (*see, e.g.,* Figure 3). The IL-15 can also be a wild-type IL-15 or a wild-type IL-15 that is attached to an IL-15Rα. In one embodiment, the IL-15 is a variant IL-15 selected from those depicted in Figure 3. In an exemplary embodiment, the Il-15 is a variant IL-15 having amino acid substitutions K86R and N112A.

IL-12s that can be used include those IL-12s that have a p35 and p40 domain. In some embodiments, the IL-12 is a single chain IL-12 as described herein (*see, e.g.,* Figure 20, as shown as part of an IL-12-ABD fusion polypeptide and Figure 49C).

In such embodiments, L1 and L2 are a first and second linker, respectively, L1 and L2 can be any linker that is suitable for attaching the IL-15 and IL-12 domains to the ABD domain (*e.g*., the linkers listed in Figure 48). Exemplary linkers than can be used include (GGGGS)ₓ linkers wherein X is 1-10. In certain embodiments, L1 and L2 are each (GGGGS)₅.

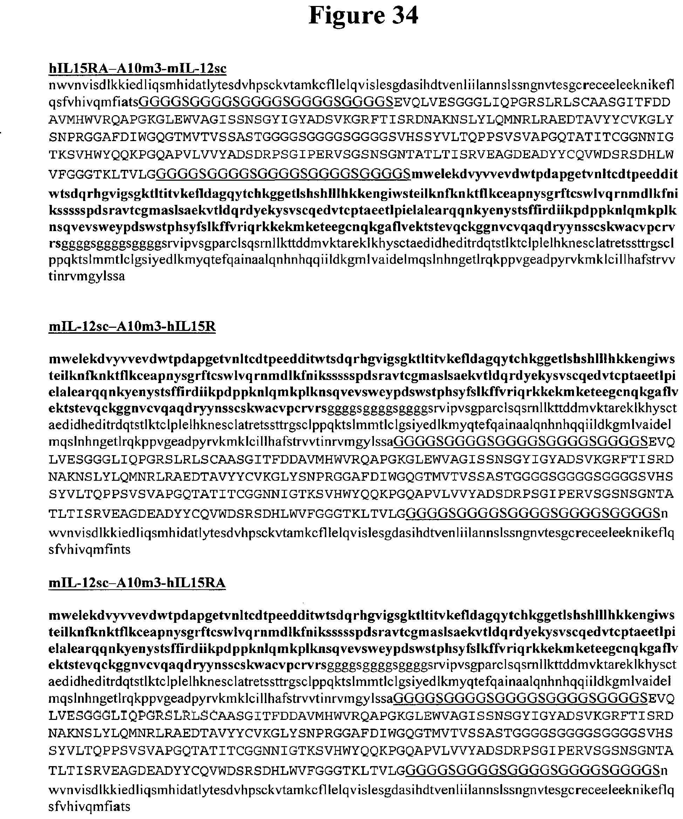

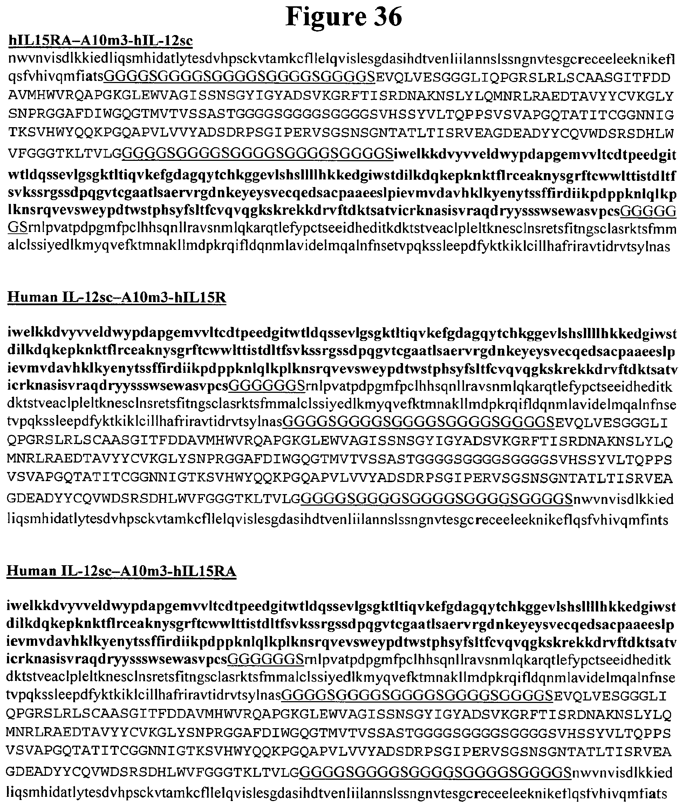

Sequences of exemplary ABD polypeptides that include an IL-12 and an IL-15 are shown in Figures 34 and 36.

### b. IL-2 and IL-12

In certain embodiments, the cytokine-ABD includes an IL-2 and an IL-12.

IL-2 and IL-12 reciprocally upregulate each other's receptors and use separate signaling pathways to induce different but complementary biological effects. Both IL-2 and IL-12 can stimulate mitogen or CD3-activated T cells to proliferate & produce IFN-γ. Moreover, studies show that the delivery of both IL-2 and IL-12 genes into mice bearing B16 melanoma elicited a significant reduction in tumor burden and enhancement in overall survival (*see, e.g.,* Dietrich et al., Arch Surg 387(34):177-182 (2002)). Thus, it is believed that cytokine-ABD fusion proteins having both an IL-2 and an IL- are useful for the reduction of tumors and treatment of cancers.

### c. IL-2 and IL-15

The cytokine-ABD fusion protein includes an IL-2 and an IL-15. Both IL-2 and IL-15 are capable of stimulating the proliferation of NK cells and activated T cells, as well as supporting the expansion of effector T cells. It is believed that cytokine-ABD fusion proteins that include both an IL-2 and IL-15 are useful for the reduction of tumors and treatment of cancers.

### d. IL-7 and IL-12

In certain embodiments, the cytokine-ABD fusion protein includes an IL-7 and an IL-12 fusion partner.

Interleukin-7 (IL-7) is a non-hematopoietic cell-derived cytokine with a central role in the adaptive immune system. It promotes lymphocyte development in the thymus maintains survival of naive and memory T cell homeostasis in the periphery. Moreover, it is important for the organogenesis of lymph nodes (LN) and for the maintenance of activated T cells recruited into the secondary lymphoid organs (SLOs). The immune capacity of cancer patients is suppressed and is characterized by lower T cell counts, less effector immune cells infiltration, higher levels of exhausted effector cells and higher levels of immunosuppressive cytokines, such as transforming growth factor β (TGF-β). IL-7 is an ideal solution for the immune reconstitution of immunosuppressed cancer patients by promoting peripheral T cell expansion. In animal models, IL-7 has been proven to prolong the survival of tumor-bearing hosts. *See* Gao et al., Int. J. Mol. Sci. 16: 10267-10280 (2015).

IL-12 acts directly on CD8+ T cells to enhance their IL-7 mediated proliferation. It is believed that cytokine-ABD fusion proteins that include an IL-7 and IL-12 binding domain advantageously promote the proliferation of CD8+ T cells and enhance cytolytic activity against tumors.

### e. IL-7 and IL-15

The cytokine-ABD fusion protein includes an IL-7 and an IL-15. As mentioned above, interleukin 7 and 15 are considered powerful pro-inflammatory cytokines that have the ability to reduce tumorgenesis. It is believed that cytokine ABD fusion proteins that include both an IL-7 and IL-15 are useful for the reduction of tumors and treatment of cancers.

### f. IL-12 and IL-21

In certain embodiments, the cytokine-ABD includes an IL-12 and an IL-21.

As mentioned above, IL-12 is capable of stimulating the proliferation of NK cells and activated T cells and supporting the expansion of effector T cells. IL-21 is a regulator of NK and T cell function that bridges innate and adaptive immune systems. IL-21 promotes the maturation of NK cells from bone marrow progenitors, activates human peripheral NK cells, promotes NK expansion and maturation, and enhances CD8+ T cell-mediated effector functions.

It is believed that cytokine-ABD fusion proteins that include both an IL-12 and IL-21 are useful for the reduction of tumors and treatment of cancers.

### g. IL-12 and IL-18

In certain embodiments, the cytokine-ABD includes an IL-12 and an IL-18.

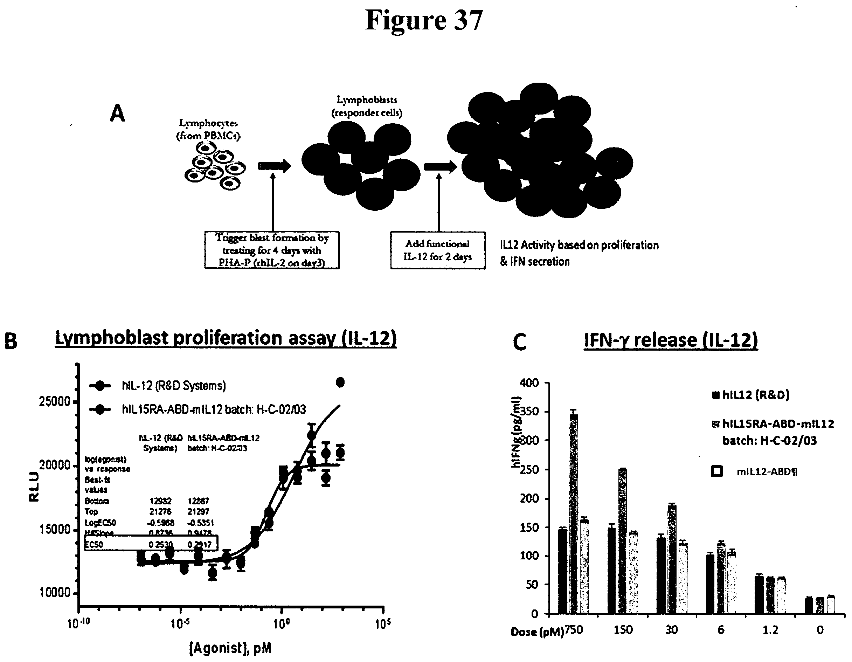

IL-18 is known to induce IFN-γ production, promote Th1 cell development and NK activation. IL-12 is known to induce the upregulation of the IL-18 receptor in an IFN-γ-dependent manner. Administration of SCK murine mammary carcinoma cells co-expressing IL-18 and IL-12 to mice reduced tumor burden and inhibited angiogenesis *(See, e.g*., Coughlin et al., J Clin Invest 101 (6):1441-1452 (1998)). IL-18 in combination with IL-12 to tumor bearing mice synergistically induced a prolonged serum level of IFN-y, while tumor bearing mice treated with IL-18 or IL-12 alone induced minimal serum IFN-γ that was rapidly attenuated (*See, e.g*., Subleski et al., Cancer Res 66(22):11005-11012 (2006)). Exemplary IL-18-ABDs are shown in Figure 37.

It is believed that cytokine-ABD fusion proteins that include both an IL-12 and IL-18 fusion partner are useful for the reduction of tumors and treatment of cancers.

### h. GM-CSF and IL-12

The cytokine-ABD includes a GM-CSF and an IL-18. GM-CSF regulates hematopoietic progenitor cell differentiation and proliferation. GM-CSF also enhances the capacity of APC to process and present antigen, which in turn leads to the activation of cytotoxic T cells, increased IFN-γ production and, ultimately, tumor regression. Both GM-CSF and IL-12 are able to elicit significant anti-tumor responses in several different preclinical tumor models, including a liver tumor model and a lung tumor model. (*See, e.g*., Kilinc et al., J Immunol 177(10):6962-6973 (2006)).

It is believed that cytokine-ABD fusion proteins that include both a GM-CSFand IL-12 fusion partner are useful for the reduction of tumors and treatment of cancers.

### i. IFN alpha and IL-12

Cooperative nature of these two cytokines extends beyond mere similar biological effects. For example, IL-12, which is well known to induce IFN-γ production, can lead to the production of additional soluble factors that enhance IFN-α signaling. Interferons, including, interferon alpha, are known to induce apoptosis in malignant cells. *See, e.g*., Thyrell, L. et al., Oncogene 21, 1251-1262 (2002).

It is believed that cytokine-ABD fusion proteins that include both an IFN alpha and IL-12 are useful for the reduction of tumors and treatment of cancers.

Additional cytokine-cytokine combinations that can be included in the subject cytokine-ABD fusion proteins are depicted in Figure 51A.

### 2. Binding moiety-ABD Fusion Proteins

In some embodiments, the ABD fusion proteins includes a binding moiety (*e.g.,* an scFv) fusion partner, i.e., a BM-ABD fusion protein. In some embodiments, the BM-ABD includes one binding moiety. In certain embodiments, the BM-ABD includes two binding moieties (*e.g*., scFvs). In other embodiments, the BM-ABD includes a cytokine and a binding moiety (Figure 1B). Figure 1 depicts several exemplary orientations in which the binding moiety or binding moiety-cytokine/binding moiety-binding moiety combinations can be attached to the ABD.

Binding moieties that are useful for practice with the subject BM-ABDs binding moieties that are based on antibody variable heavy domain and variable light domain. In some embodiments, the binding moiety includes a variable heavy domain and a variable light domain. In some embodiments, the binding moiety is a single chain variable fragment (scFv).

Any ABD can be used in such fusion protein, including those that include any of the ABD variable heavy domains and variable light domains shown in Figure 2. In certain embodiments, the ABD includes the variable heavy and light domains of A10m3. In some embodiments, the ABD is A10m3 scFv.

Any linker can be used to attached the cytokine and/or binding moiety to the ABD, including any linker depicted in Figure 48. In an exemplary embodiment, the linker is (GGGGS)₅. An exemplary backbone linker-A10m3-linker sequence for use in such fusion proteins is depicted in Figure 48.

Exemplary combinations of two binding moieties (*e.g*., scFvs) or a cytokine and a binding moiety that can be such ABD fusion proteins are depicted in Figure 51B.

In some embodiments, the BM-ABD includes an anti-TGFβ binding domain (*e.g.,* an anti-TGFβ scFv). Exemplary anti-TGFβ scFv sequences are depicted in Figure 45. In some embodiments, the anti-TGFβ scFv includes the variable heavy and variable light domain of 4D9 anti-TGFβ scFv. In some embodiments, the ABD fusion protein includes an anti-TGFβ scFv and a cytokine or additional binding moiety, where the cytokine or additional binding moiety is a second anti-TGFβ scFv, IL-15, IL-12, or an anti-PD-L1 binding domain (10D12). *See* Figure 51B.

In some embodiments, the BM-ABD includes an anti-PD-L1 binding domain (*e.g.,* an anti-T PD-L1 scFv). In some embodiments, the anti- PD-L1 scFv includes the variable heavy and variable light domain of 4D9 anti- PD-L1 10D12 scFv. In some embodiments, the ABD fusion protein includes an anti- PD-L1 scFv and a cytokine or additional binding moiety, where the cytokine or additional binding moiety is a second anti-anti- PD-L1 scFv, IL-15, IL-12, or an anti- TGFβ binding domain (4D9). *See* Figure 51B.

### a. TGF-β binding moieties

In certain embodiments, the BM-ABD provided herein includes a TGF-β binding moiety. As used herein, "TGF-β," "TGFβ," "TGFb," and "transforming growth factor beta" all refer to a member of a family of cytokines that are involved that controls proliferation, cellular differentiation, and other functions in most cells and exist in at least three isoforms: TGFβ1 (Genbank Accession numbers: NM_000660 and NP_000651 (human); and NM_011577 and NP_035707 (mouse)), TGFβ2 (Genbank Accession numbers NM_001135599 and NP_001129071 (human)) and NM_009367 and NP_33393 (mouse)) , and TGFβ3 (Genbank Accession number: NM_003239). TGFβ family members have an N-terminal signal peptide of 20-30 amino acids that are required for secretion from cells, a pro-region, and a 112-113 amino acid C-terminal region that becomes the mature TGFβ molecule following its release form the pro-region by proteolytic cleavage. In certain embodiments, the mature TGFβ protein can dimerize to produce a 25kDa active molecule with many conserved structural motifs, include nine cysteine residues, eight of which form disulfide bonds within the TGFβ molecule to create a cysteine knot structure. The ninth conserved cysteine forms a bond with the ninth cysteine of another TGFβ to produce the dimer.

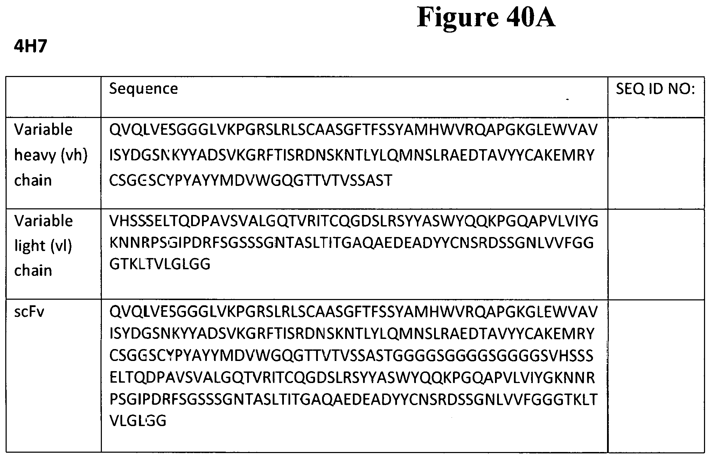

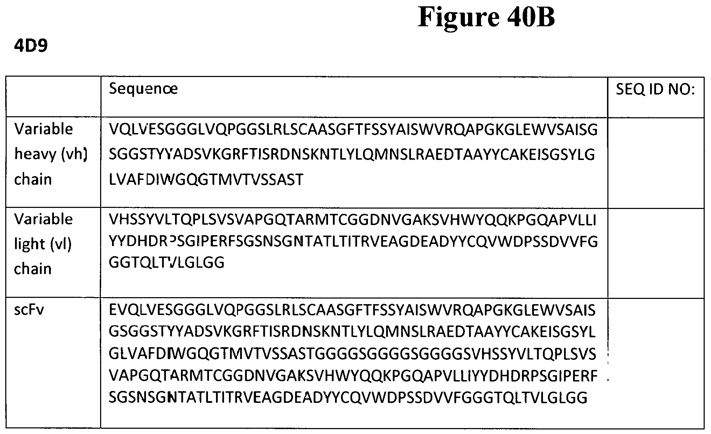

Without being bound by any particular theory of operation, it is believed that subject BM-ABD that bind TGFβ can be used to treat subjects having cancers (*e.g*., a late stage cancer). In certain embodiments, the BM-ABD includes a TGFβ1 binding moiety. In certain embodiments, the BM-ABD includes a TGFβ2 binding moiety. In certain embodiments, the BM-ABD includes a TGFβ3 binding moiety. In some embodiments, the multivalent binding polypeptide includes a binding moiety that can bind TGFβ1, TGFβ2, and/or TGFβ3 or any combination thereof (*e.g*., binds TGFβ1 and TGFβ2; binds TGFβ2 and TGFβ3; binds TGFβ1 and TGFβ3; or binds TGFβ1, TGFβ2 and TGFβ3). In some embodiments, the TGFβ binding moiety binds TGFβ1, TGFβ2 and TGFβ3. In some embodiments, the TGFβ binding moiety that includes the variable heavy domain and variable light domain of a TGFβ binding moiety in Figure 40. In one embodiment, the TGFβ binding moiety includes the variable heavy domain and variable light domain of TGFβ binding moiety 4D9 (Figure 40B). In a particular embodiment, the TGFβ binding moiety is the 4D9 scFv. 4D9 has been shown to prevent the expansion of CD4⁺FoxP3⁺ regulatory T cell, prevent Smad activation (*e.g*., Smad2 phosphorylation), as well as prevent cellular epithelial-to-mesenchymal transition and/or cancer cell migration.

In some embodiments, the TGFβ binding moiety-ABD fusion protein is further attached to another binding moiety or cytokine. In certain embodiments, the other binding moiety is a PD-L1 binding moiety or another TGFβ binding moiety. In some embodiments, the cytokine is IL-12 (*See* Figure 51B).

### b. PD-L1 binding moieties

In certain embodiments, the BM-ABD provided herein includes a Programmed Cell Death 1 Ligand 1 (PD-L1) binding moiety. As used herein, "Programmed Cell Death 1 Ligand 1," "Programmed Death Ligand 1," "PDL1," and "PD-L1" (Genbank Accession numbers NM_001267706 and NP_001254635 (human) and NM_021893 and NP_068693 (mouse)) all refer to a member of a 40kDa type 1 transmembrane protein that has binds to PD1 receptor, found on activated T cells, B cells, and myeloid cells, to modulate activation or inhibition. PD-L1 upregulation allows cancers to evade the immune system. *See, e.g.,* Hamanishi et al., Proc Natl Acad Sci USA 104(9): 3360-5 (2007). As such, it is believed that subject BM-ABDs that include a PD-L1 binding moiety may be useful in the treatment of cancers.

In one embodiment, the PD-L1 binding moiety includes the variable heavy domain and variable light domain of PD-L1 binding moiety 10D12 (Figure 50). In a particular embodiment, the PD-L1 binding moiety is the 10D12 scFv (Figure 50). 10D12 binds to hPD-L1 at low pH and is crossreactive with mPD-L1. 10D12 does not bind to hPD-L2 or mPD-L2. Further, 10D12 blocks PD-1/PD-L1 interaction, as well as B71/PD-L1 interaction.

In some embodiments, the PD-L1 binding moiety-ABD fusion protein is further attached to another binding moiety or cytokine. In certain embodiments, the other binding moiety is a TGFβ binding moiety or another PD-L1 binding moiety. In some embodiments, the cytokine is IL-12 (*See* Figure 51B).

### c. TNF and other binding moieties

In one embodiment, the ABD fusion protein provided herein includes a tumor nercosis factor (TNF) binding moiety. With out being bound by any particular theory of operation, it is believe that such ABD fusion proteins are useful as anti-inflammation and/or cancer therapeutics. In some embodiments, the TNF binding moiety is an scFv. In particular embodiments, the TNF binding moiety-ABD fusion protein is further attached to another fusion partner that is a binding moiety or an inhibitor peptide. In certain embodiments, the second binding moiety is a second TNF binding moiety, an IL-1 binding moiety, an IL-6 binding moiety, an IL-8 binding moiety, an IL-17 (isoforms A-F) binding moiety, or an IL-23 binding moiety.

In another embodiment, the ABD fusion protein includes a binding moiety selected from an IL-1, IL-6, IL-8, IL-17(A-F) and IL-23 binding moiety.

In some embodiments, such TNF and interleukin binding moieties-ABDs are useful for the treatment of diseases such as rheumatoid arthritis, Crohn's disease, psoriatic arthritis, ankylosing spondylitis, ulcerative colitis, chronic plaque psoriasis and TNF based diseases.

### F. Diagnostic Uses

The description describes methods for imaging and/or detecting tumors. The description describes methods that comprise contacting a tumor cell, tumor cell culture, tumor vasculature cell, tumor vasculature cell culture, tumor tissue, and other tissues and cells with a subject ABD fusion protein that is labeled.

The ABD fusion protein also find use in the in vitro or in vivo imaging of tumors or autoimmune disease states associated with the antigen binding partners of the ABD fusion protein herein. The fusion protein described herein are used for both diagnosis and treatment, or for diagnosis alone. A subject ABD fusion protein may be labeled.

Diagnosis can be done either in vivo, by administration of a diagnostic protein that allows whole body imaging as described below, or in vitro, on samples removed from a patient. "Sample" in this context includes any number of things, including, but not limited to, bodily fluids (including, but not limited to, blood, urine, serum, lymph, saliva, anal and vaginal secretions, perspiration and semen), as well as tissue samples such as result from biopsies of relevant tissues.

By "labeled" herein is meant that the ABD fusion protein disclosed herein have one or more elements, isotopes, or chemical compounds attached to enable the detection in a screen or diagnostic procedure. In general, labels fall into several classes: a) immune labels, which may be an epitope incorporated as a fusion partner that is recognized by an antibody, b) isotopic labels, which may be radioactive or heavy isotopes, c) small molecule labels, which may include fluorescent and colorimetric dyes, or molecules such as biotin that enable other labeling methods, and d) labels such as particles (including bubbles for ultrasound labeling) or paramagnetic labels that allow body imagining. Labels may be incorporated into the proteins at any position (for example, through one or more of the linkers described herein) and may be incorporated in vitro or in vivo during protein expression, as is known in the art. Specific labels include optical dyes, including, but not limited to, chromophores, phosphors and fluorophores, with the latter being specific in many instances. Fluorophores can be either "small molecule" fluores, or proteinaceous fluores.

By "fluorescent label" is meant any molecule that may be detected via its inherent fluorescent properties. Suitable fluorescent labels include, but are not limited to, fluorescein, rhodamine, tetramethylrhodamine, eosin, erythrosin, coumarin, methyl-coumarins, pyrene, Malacite green, stilbene, Lucifer Yellow, Cascade BlueJ, Texas Red, IAEDANS, EDANS, BODIPY FL, LC Red 640, Cy 5, Cy 5.5, LC Red 705, Oregon green, the Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow and R-phycoerythrin (PE) (Molecular Probes, Eugene, Oreg.), FITC, Rhodamine, and Texas Red (Pierce, Rockford, Ill.), Cy5, Cy5.5, Cy7 (Amersham Life Science, Pittsburgh, Pa.). Suitable optical dyes, including fluorophores, are described in Molecular Probes Handbook by Richard P. Haugland,.

Suitable proteinaceous fluorescent labels also include, but are not limited to, green fluorescent protein, including a Renilla, Ptilosarcus, or Aequorea species of GFP (Chalfie et al., Science 263:802-805 (1994)), EGFP (Clontech Laboratories, Inc., Genbank Accession Number U55762), blue fluorescent protein (BFP, Quantum Biotechnologies, Inc. 1801 de Maisonneuve Blvd. West, 8th Floor, Montreal, Quebec, Canada H3H 1J9; Stauber, 1998, Biotechniques 24:462-471; Heim et al., 1996, Curr. Biol. 6:178-182), enhanced yellow fluorescent protein (EYFP, Clontech Laboratories, Inc.), luciferase (Ichiki et al., J. Immunol. 150:5408-5417 (1993)), .beta galactosidase (Nolan et al., Proc. Natl. Acad. Sci. U.S.A. 85:2603-2607 (1998)) and Renilla (WO92/15673, WO95/07463, WO98/14605, WO98/26277, WO99/49019, U.S. Pat. Nos. 5,292,658, 5,418,155, 5,683,888, 5,741,668, 5,777,079, 5,804,387, 5874304, 5876995, 5925558).

### G. Production of Albumin Binding Domains and Fusion Proteins

As will be appreciated by those in the art, standard protocols are used to make the subject ABDs. General methods for antibody molecular biology, expression, purification, and screening are described in Antibody Engineering, edited by Kontermann & Dubel, Springer, Heidelberg, 2001; and Hayhurst & Georgiou, Curr Opin Chem Biol 5:683-689 (2001); Maynard & Georgiou, Annu Rev Biomed Eng 2:339-76 (2000).

In one embodiment disclosed herein, nucleic acids are created that encode the ABD fusion protein, and that may then be cloned into host cells, expressed and assayed, if desired. Thus, nucleic acids, and particularly DNA, may be made that encode each protein sequence. These practices are carried out using well-known procedures. For example, a variety of methods that may find use in generating ABD fusion proteins, similar to the production of antibodies, are disclosed herein are described in Molecular Cloning - A Laboratory Manual, 3rd Ed. (Maniatis, Cold Spring Harbor Laboratory Press, New York, 2001), and Current Protocols in Molecular Biology (John Wiley & Sons).

There are a variety of techniques that may be used to efficiently generate DNA encoding ABDs disclosed herein. Such methods include, but are not limited to, gene assembly methods, PCR-based method and methods which use variations of PCR, ligase chain reaction-based methods, pooled oligo methods such as those used in synthetic shuffling, error-prone amplification methods and methods which use oligos with random mutations, classical site-directed mutagenesis methods, cassette mutagenesis, and other amplification and gene synthesis methods. As is known in the art, there are a variety of commercially available kits and methods for gene assembly, mutagenesis, vector subcloning, and the like, and such commercial products find use in for generating nucleic acids that encode ABD fusion proteins.

The ABDs disclosed herein may be produced by culturing a host cell transformed with nucleic acid, *e.g*., an expression vector, containing nucleic acid encoding the ABD fusion proteins, under the appropriate conditions to induce or cause expression of the protein. The conditions appropriate for expression will vary with the choice of the expression vector and the host cell, and will be easily ascertained by one skilled in the art through routine experimentation. A wide variety of appropriate host cells may be used, including but not limited to mammalian cells, bacteria, insect cells, yeast, and plant cells. For example, a variety of cell lines that may find use in generating ABD fusion proteins disclosed herein are described in the ATCC^{®} cell line catalog, available from the American Type Culture Collection.

In one embodiment, the ABDs are expressed in mammalian expression systems, including systems in which the expression constructs are introduced into the mammalian cells using virus such as retrovirus or adenovirus. Any mammalian cells may be used, *e.g*., human, mouse, rat, hamster, and primate cells. Suitable cells also include known research cells, including but not limited to Jurkat T cells, NIH3T3, CHO, BHK, COS, HEK293, PER C.6, HeLa, Sp2/0, NS0 cells and variants thereof. In an alternate embodiment, library proteins are expressed in bacterial cells. Bacterial expression systems are well known in the art, and include Escherichia coli (E. coli), Bacillus subtilis, Streptococcus cremoris, and Streptococcus lividans. In alternate embodiments, the ABD fusion proteins are produced in insect cells (*e.g*., Sf21/Sf9, Trichoplusia ni Bti-Tn5b1-4) or yeast cells (*e.g.,* S. cerevisiae, Pichia, etc). In an alternate embodiment, the ABD polypetides are expressed *in vitro* using cell free translation systems. In vitro translation systems derived from both prokaryotic (*e.g.,* E. coli) and eukaryotic (*e.g.,* wheat germ, rabbit reticulocytes) cells are available and may be chosen based on the expression levels and functional properties of the protein of interest. For example, as appreciated by those skilled in the art, in vitro translation is required for some display technologies, for example ribosome display. In addition, the ABD fusion proteins may be produced by chemical synthesis methods. Also transgenic expression systems both animal (*e.g*., cow, sheep or goat milk, embryonated hen's eggs, whole insect larvae, etc.) and plant (*e.g*., corn, tobacco, duckweed, etc.).

The nucleic acids that encode ABD fusion proteins disclosed herein may be incorporated into an expression vector in order to express the protein. A variety of expression vectors may be utilized for protein expression. Expression vectors may comprise self-replicating extra-chromosomal vectors or vectors which integrate into a host genome. Expression vectors are constructed to be compatible with the host cell type. Thus expression vectors which find use in generating antibodies disclosed herein include, but are not limited to, those which enable protein expression in mammalian cells, bacteria, insect cells, yeast, and in *in vitro* systems. As is known in the art, a variety of expression vectors are available, commercially or otherwise, that may find use for expressing antibodies disclosed herein.

The disclosed ABD fusion proteins can be encoded by multiple nucleic acid molecules. For example, the variable heavy and light chains can be introduced into a host cell independently. Though present on separate nucleic acids, their expression yields a single polypeptide.

Expression vectors typically comprise a protein operably linked with control or regulatory sequences, selectable markers, any fusion partners, and/or additional elements. By "operably linked" herein is meant that the nucleic acid is placed into a functional relationship with another nucleic acid sequence. Generally, these expression vectors include transcriptional and translational regulatory nucleic acid operably linked to the nucleic acid encoding the multivalent ABD fusion proteins, and are typically appropriate to the host cell used to express the protein. In general, the transcriptional and translational regulatory sequences may include promoter sequences, ribosomal binding sites, transcriptional start and stop sequences, translational start and stop sequences, and enhancer or activator sequences. As is also known in the art, expression vectors typically contain a selection gene or marker to allow the selection of transformed host cells containing the expression vector. Selection genes are well known in the art and will vary with the host cell used.

In one embodiment, ABDs are purified or isolated after expression. ABDs and ABD fusion proteins may be isolated or purified in a variety of ways known to those skilled in the art. Purification may be particularly useful for separating heterodimeric heavy chain species from homodimeric heavy chain species, as described herein. Standard purification methods include chromatographic techniques, including ion exchange, hydrophobic interaction, affinity, sizing or gel filtration, and reversed-phase, carried out at atmospheric pressure or at high pressure using systems such as FPLC and HPLC. Purification methods also include electrophoretic, isoelectric focusing, immunological, precipitation, dialysis, and chromatofocusing techniques. Ultrafiltration and diafiltration techniques, in conjunction with protein concentration, are also useful. Fusion is employed, Ni+2 affinity chromatography if a His-tag is employed, or immobilized anti-flag antibody if a flag-tag is used. For general guidance in suitable purification techniques, *see, e.g*.,

Protein Purification: Principles and Practice, 3rd Ed., Scopes, Springer-Verlag, NY, 1994. The degree of purification necessary will vary depending on the screen or use of the antibodies. In some instances no purification is needed.

### H. Therapeutic Uses of Albumin Binding Domains and ABD Fusion Proteins

The subject ABDs and ABD fusion proteins find use in a variety of therapeutic uses as described herein.

The description describes a method of inhibiting tumor growth in a subject in need thereof by administering to the subject albumin binding domain (ABD) fusion proteins described herein. Useful ABD fusion proteins include, but are not limited to, those disclosed in Figures 4, 20, 34, 36, 40, 45, 50-51.

In some embodiments, the ABD fusion protein includes an IL-12 (*e.g.,* an IL-12-ABD fusion protein). As described herein, IL-15 ABD fusion proteins are capable of inhibiting tumor growth in a dose dependent manner. Such IL-15 mediated tumor growth inhibition is accompanied by an increase in tumor infiltrating lymphocytes, including cytotoxic T lymphocytes (CTLs) and activated natural killer (NK) cells. In certain embodiments, the ABD fusion protein includes an IL-12 molecule.

The ABD fusion protein includes an IL-15. In yet other embodiments, the cytokine-ABD includes an IL-12 and an IL-15. The IL-15-ABD includes a variant IL-15 as described herein (*see, e.g.,* Figure 3).

Also provided herein is a method of treating a subject having cancer by administering to the subject albumin binding domain (ABD) fusion protein. In some embodiments, the ABD fusion protein includes an IL-12 (*e.g.,* an IL-12-ABD
fusion protein). IL-12 and IL-15 are cytokines for immunomodulation of the tumor microenvironment owing to its ability to proliferate and extend survivability of CD8+ T cells. Other useful ABD fusion proteins include, but are not limited to, those disclosed in Figures 4, 20, 34, 36, 40, 45, 50-51.

Examples of cancer to be treated herein include, but are not limited to, carcinoma, blastoma, sarcoma, certain leukemia or lymphoid malignancies. More particular examples of such cancers include squamous cell cancer (*e.g*., epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, skin cancer/melanoma, as well as head and neck cancer and metasteses associated with any of the primary tumors.

The description describes a method of increasing proliferation and/or survivability of a CD8+ T cell. The method comprises contacting the cell with an albumin binding domain fusion protein that includes in IL-12 and/or IL-15 (*e.g.,* an IL-12-ABD or IL-15-ABD fusion protein). In certain embodiments, the ABD fusion protein includes an IL-12 molecule. The ABD fusion protein includes an IL-15 molecule. In yet other embodiments, the fusion protein the ABD fusion protein includes an IL-12 and an IL-15.

### 1. Pharmaceutical Formulations, Administration and Dosing

The description describes a therapeutic composition comprising any subject albumin binding domain (ABD) polypeptide and a carrier. Subject therapeutic compositions used in the practice of the foregoing methods can be formulated into pharmaceutical compositions comprising a carrier suitable for the desired delivery method. Suitable carriers include any material that when combined with the therapeutic composition retains the anti-tumor function of the therapeutic composition and is generally non-reactive with the patient's immune system. Examples include, but are not limited to, any of a number of standard pharmaceutical carriers such as sterile phosphate buffered saline solutions, bacteriostatic water, and the like (see, generally, Remington's Pharmaceutical Sciences 16th Edition, A. Osal., Ed., 1980).

### 1. Compositions for In Vivo Administration

Formulations of the albumin binding domain (ABD) fusion protein used in accordance with the present disclosure are prepared for storage by mixing an ABD fusion protein having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) fusion proteins; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA or DPTA; sugars such as sucrose, mannitol, trehalose or sorbitol; salt-forming counter-ions such as sodium; metal complexes (*e.g*., Zn-protein complexes); and/or non-ionic surfactants such as TWEEN^{™}, PLURONICS^{™} or polyethylene glycol (PEG) of various molecular weights.

The formulation herein may also contain more than one active compound as necessary for the particular indication being treated, preferably those with complementary activities that do not adversely affect each other. For example, it may be desirable to provide ABD fusion proteins with other specificities. Alternatively, or in addition, the composition may comprise a cytotoxic agent, cytokine, growth inhibitory agent and/or small molecule antagonist. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration should be sterile, or nearly so. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g*., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and .gamma ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods.

When encapsulated albumin binding domain fusion proteins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

### 2. Administrative modalities

The subject albumin binding domain fusion proteins and therapeutic agents are administered to a subject, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous or subcutaneous administration of the antibody is preferred.

### 3. Treatment modalities

In the methods provided herein, therapy is used to provide a positive therapeutic response with respect to a disease or condition. By "positive therapeutic response" is intended an improvement in the disease or condition, and/or an improvement in the symptoms associated with the disease or condition. For example, a positive therapeutic response would refer to one or more of the following improvements in the disease: (1) a reduction in the number of tumor cells; (2) an increase in tumor cell death; (3) inhibition of tumor cell survival; (5) inhibition (i.e., slowing to some extent, preferably halting) of tumor growth; (6) an increased patient survival rate; and (7) some relief from one or more symptoms associated with the disease or condition.

Positive therapeutic responses in any given disease or condition can be determined by standardized response criteria specific to that disease or condition. Tumor response can be assessed for changes in tumor morphology (i.e., overall tumor burden, tumor size, and the like) using screening techniques such as magnetic resonance imaging (MRI) scan, x-radiographic imaging, computed tomographic (CT) scan, bone scan imaging, endoscopy, and tumor biopsy sampling including bone marrow aspiration (BMA) and counting of tumor cells in the circulation.

In addition to these positive therapeutic responses, the subject undergoing therapy may experience the beneficial effect of an improvement in the symptoms associated with the disease.

Thus, for B cell tumors, for example, the subject may experience a decrease in the so-called B symptoms, i.e., night sweats, fever, weight loss, and/or urticaria. For pre-malignant conditions, therapy with an multivalent therapeutic agent may block and/or prolong the time before development of a related malignant condition, for example, development of multiple myeloma in subjects suffering from monoclonal gammopathy of undetermined significance (MGUS).

An improvement in the disease may be characterized as a complete response. By "complete response" is intended an absence of clinically detectable disease with normalization of any previously abnormal radiographic studies, bone marrow, and cerebrospinal fluid (CSF) or abnormal monoclonal protein in the case of myeloma.

Such a response may persist for at least 4 to 8 weeks, or sometimes 6 to 8 weeks, following treatment according to the subject methods. Alternatively, an improvement in the disease may be categorized as being a partial response. By "partial response" is intended at least about a 50% decrease in all measurable tumor burden (i.e., the number of malignant cells present in the subject, or the measured bulk of tumor masses or the quantity of abnormal monoclonal protein) in the absence of new lesions, which may persist for 4 to 8 weeks, or 6 to 8 weeks.

Treatment includes a "therapeutically effective amount" of the medicaments used. A "therapeutically effective amount" refers to an amount effective, at dosages and for periods of time necessary, to achieve a desired therapeutic result.

A therapeutically effective amount may vary according to factors such as the disease state, age, sex, and weight of the individual, and the ability of the medicaments to elicit a desired response in the individual. A therapeutically effective amount is also one in which any toxic or detrimental effects of the antibody or antibody portion are outweighed by the therapeutically beneficial effects.

A "therapeutically effective amount" for tumor therapy may also be measured by its ability to stabilize the progression of disease. The ability of a compound to inhibit cancer may be evaluated in an animal model system predictive of efficacy in human tumors.

Alternatively, this property of a composition may be evaluated by examining the ability of the compound to inhibit cell growth or to induce apoptosis by in vitro assays known to the skilled practitioner. A therapeutically effective amount of a therapeutic compound may decrease tumor size, or otherwise ameliorate symptoms in a subject. One of ordinary skill in the art would be able to determine such amounts based on such factors as the subject's size, the severity of the subject's symptoms, and the particular composition or route of administration selected.

### EXAMPLES

The presently claimed invention is as defined in the claims. Any of the following Examples that do not fall within the scope of the claims do not form part of the claimed invention and are provided for comparative purposes only.

### Example 1: Screening and Identification of Human Serum Albumin Binding Domains

Solid phase panning and solution panning methods were carried out to identify phage scFv albumin binding domains (ABD). Albumin binding domains that were selected from a primary screen for human serum albumin binding domains were subsequently screened for cross-reactivity to mouse serum albumin using standard ELISA techniques. Primary albumin binding domain candidates obtained using the screening methods were sequenced and subsequently assayed for target concentration-dependent binding, pH stability, FcRn binding interference and kinetic binding. In particular, candidate ABDs were chosen for their ability to bind human serum albumin (kD ~20-60 nM), mouse serum albumin (kD ~10-30 nM) and cyno serum albumin (kD ~20-60 nM) at low pH (pH5.5) and neutral pH (pH 7.7). Candidate ABDs were assayed to ensure that they did not compete with FcRn binding to serum albumin. As explained herein, ABDs that bind at such pH and did not compete with FcRn binding are capable of undergoing FcRn mediated endosomal recycling. Thus, biologics (*e.g*., cytokines and antibody based biologics) that include such ABDs are also capable of undergoing such FcRn mediated recycling and, thus, exhibit a longer half-life compared to counterparts that do not include such ABDs.

Five albumin binding clones were selected based on these criteria: A9, A10, A6, 2B4, 2H10. Of these five clones, A10 was selected based on high expression level and best activity profile. A10 was then mutated to eliminate regions that could putatively cause immunogenicity. From these A10 variants, A10m3 was selected as the lead based on its high affinity for serum albumin.

The sequences of exemplary human serum albumin bind domains are shown in Figure 2 including A10m3 (Figure 2D).

### Example 2: Variant IL-15 and IL-15-ABDs

### Expression of IL15-A10m3 protein is extremely poor by HEK293T cells, which cannot be accounted by transcription

IL-15-ABD (IL-15-A10m3) constructs were produced in HEK293 cells in three independently transfected cell cultures and assessed by Western blotting using either anti-His tag antibody (Figure 5A, left) or by functional ELISA binding to mouse serum albumin (Figure 5A, right). As shown in Figure 5A, expression of IL-15-A10m3 could not be assessed by either of these methods. To assess whether the lack of IL-15-A10m3 expression in HEK293 cells was due to low transcription levels, mRNA was prepared from four independently IL-15-A10m3 transfected cells and RT-PCR was performed to quantify the mRNA level of IL15-A10m3 mRNA (Figure 5B, lanes 2-5) in comparison with that of a house-keeping gene, GAPDH (Figure 5B, lane 6). As shown in Figure 5B, IL15-A10m3 mRNA was detected in significant quantities from the transfected cells relative to that control GAPDH, suggesting that the low expression levels of IL-15-A10m3 produced in HEK293 cells is not due to transcription, but most likely due associated with the translational or post-translational process.

### Identification of a putative ubiquitination site in IL15 that is adjacent to the IL15 receptor alpha binding site

Studies have shown that IL15 protein were expressed in the cells but are very unstable with a short half-life. Co-expression of IL15Rα with IL15 in the same cell greatly increased the amount of cell surface IL15Rα as well as IL15. Further studies confirmed that IL15Rα acts as a chaperon of IL15 and binds IL15 intracellularly to protect and stabilize IL15 before secretion. These findings suggest that translation may not account for the low productivity of IL15. Rather, post-translational modification (PTM) might play a role in IL15 intracellular instability, and that the intracellular instability of IL15 can be overcome by IL15Rα blocking the specific yet-not-known post-translational modification. Ubiquitination is a well-documented mechanism that allows cells to mark intracellular proteins for degradation.

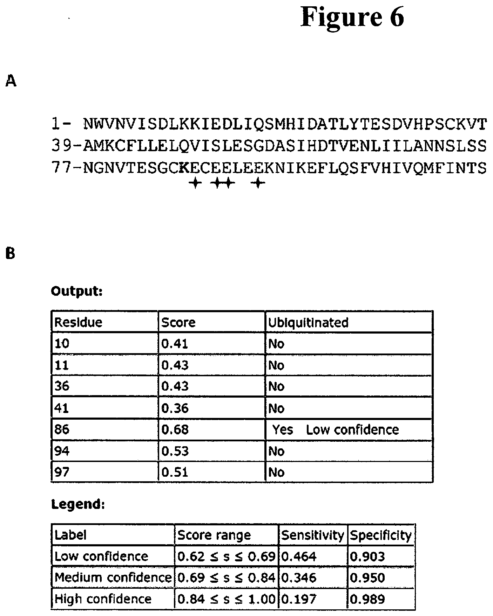

Given that IL15 is a very potent proinflammatory cytokine, whose expression is tightly controlled by cells, it is possible that cells utilize ubiquitination to actively control IL15 protein levels. Potential ubiquitination sites on IL-15 that are putatively protected upon binding to IL-15Rα were identified (Figure 6). In particular, amino acid K86 is a putative ubiquitination site that is next to the IL-15/ IL-15Rα binding site (Figure 6A), suggesting the possibility that the binding of IL15Rα to IL15 blocks the accessibility of ubiquitin ligase (*e.g.,* E3) to K86 on the IL15 protein. K86 was further confirmed as a ubiquitination site using UbPred, an online ubiquitination site database (www.ubpred.org) Figure 6B.

### Mutation of K86 on IL15 restores the expression of the IL15-A10m3 protein by HEK293T cells

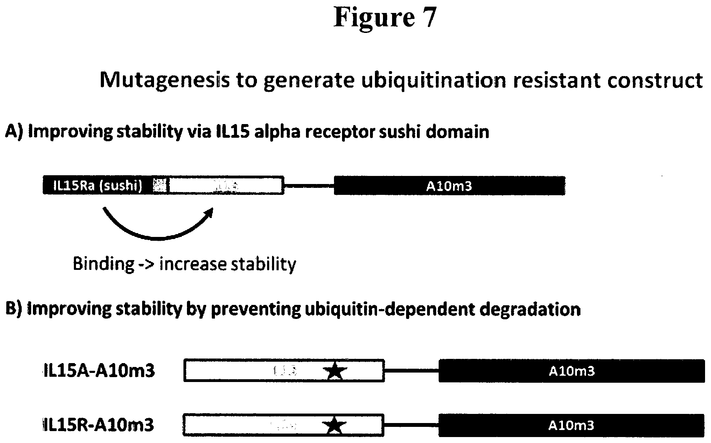

To assess whether ubiquitination at K86 affects the intracellular stability of IL-15, IL-15 variants containing amino acid substitutions at K86, including K86A and K86R, were made. The sequences of several of these IL-15 variants are shown in Figure 3. Without being bound by any particular theory of operation, it is believed that amino acid substitutions at these particular ubiquitination sites lead to ubiquitination resistant IL-15 that exhibit increased stability over wildtype IL-15. Such IL-15 variants were attached to an ABD (A10m3) for further half-life extension (Figure 4). Figure 7 provides schematic illustrations of such IL-15-ABD fusion proteins including an IL-15-ABD with a IL-15Rα/IL15 "sushi domain" (A) and IL-15-ABD fusion proteins that include IL-15 variants having amino acid substitutions at ubiquitination site K86 (B).

HEK293 cell-produced IL-15-ABD K86R and K86A variants and IL-15Rα/IL15-ABD were assessed for their ability to bind mouse serum albumin (MSA) and IL-15Rα. As shown in Figure 8A, HEK293-produced IL-15-ABD K86R and K86A variants (12 clones) and IL-15Rα/IL15-ABD (12 clones) exhibited higher expression as compared to HEK-293-produced wildtype IL-15-ABD (12 clones). Such constructs also were capable of binding to MSA. Moreover, as shown Figure 8B, the K86R (clone R6, green star) and K86A (clone A3, yellow star) substitutions did not interfere with the ability of the variants to bind to IL-15Rα. Interestingly, IL15Rα-fused IL15-A10m3 failed to show any binding to IL15Rα, suggesting that the internal IL15Rα sushi domain bound to IL15 intra-molecularly and thus blocked the binding to external IL15Rα coated on the plate. This is consistent with our and others' finding that binding of IL15 to IL15Rα sushi domain increases the IL15 expression (Figure 7B).

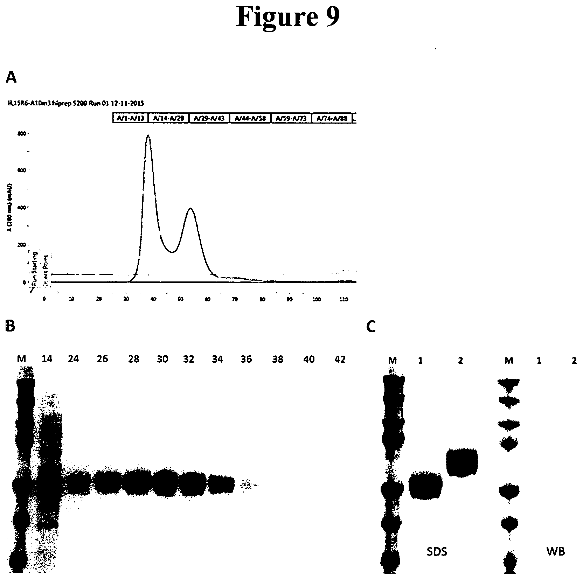

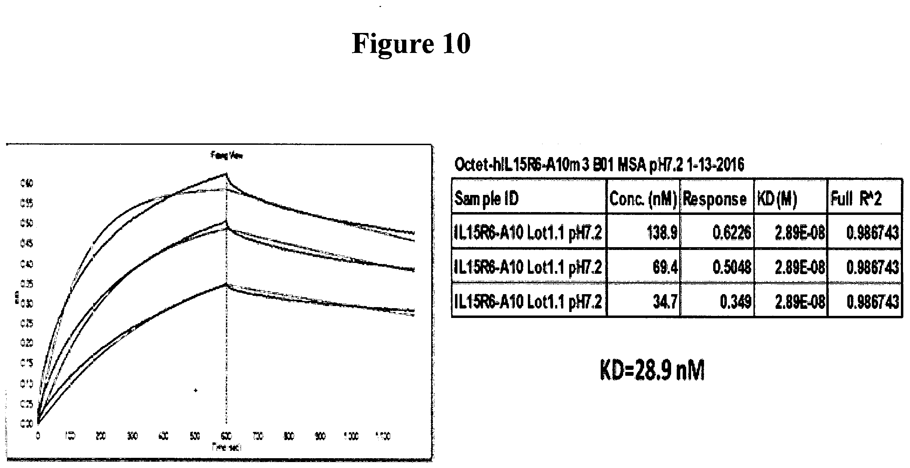

A scaled up production of one particular variant HEK293 cell-produced IL-15-ABD containing amino acid substitution K86R (IL-15 K86R-A10m3) was carried out and the IL-15 K86R-A10m3 construct was assessed for serum albumin binding *in vitro.* As shown in Figure 9, IL-15-ABD having a K86 mutation (IL-15 K86R-A10m3) and IL-15Rα/IL15-ABD (IL-15Rα/IL15-A10m3) were capable of being produced in HEK 293 cells in scaled up quantities, as confirmed by SDS-PAGE (Figure 9C, left) and Western blotting with an anti-His tag antibody (right). Further, as shown in Figure 10, K86R-A10m3 exhibited binding to mouse serum albumin (MSA).

### The bioactivity of IL15 K86R -A10m3 produced from HEK293T cells is impaired, which can be rescued by de-glycosylation.

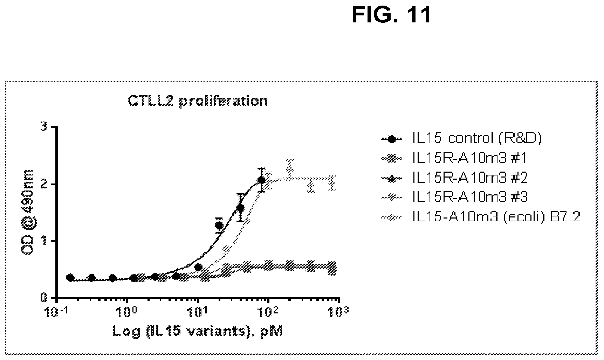

Bioactivity of various IL-15-ABDs were tested using CTLL2 proliferation assays. IL-15-ABDs tested include wildtype IL-15-A10m3 and three different IL-15 K86R-A10m3s produced in HEK293T cells. Commercially available IL-15 and in-house produced IL-15-ABD, both made using E. coli, were used as controls. As shown in Figure 11, IL-15 K86R-A10m3 produced from HEK293T showed a significantly reduced ability to promote CTLL2 proliferation compared to controls produced in E. coli, including commercial wildtype IL-15 (R&D) and IL-15-A10m3 produced in-house. Given the difference in CTLL2 proliferation assays between IL15 K86R -A10m3 from HEK293T and IL15-A10m3 from E. coli, it was hypothesized that since IL15-A10m3 produced from E. coli does not undergo the classic N-glycosylation as it does in mammalian cells, it is possible that glycosylation of IL15 K86R -A10m3 in HEK293 T cells may interfere in the interaction of IL15 K86R -A10m3 with its receptors.

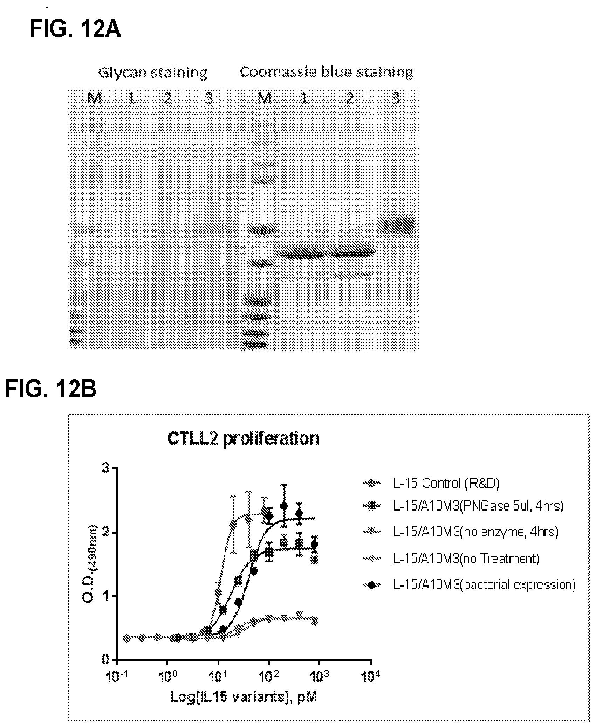

To assess whether the reduced bioactivity of HEK cell-produced IL-15 K86R-A10m3 is due to glycosylation, IL-15 K86R-A10m3 was deglycosylated using PNGase and CTLL2 proliferation assays were carried out to assess bioactivity of the deglycosylated IL15 K86R-A10m3. After PNGase mix treatment of IL-15 K86R-A10m3 under native conditions, glycan was completely removed and visualized by SDS-PAGE followed by glycan staining (Figure 12A, left) and coomassie blue staining (Figure 12A, right). 1) IL-15R-A10m3+ 5ul PNGase mix; 2) protein+ 10ul PNGase mix; 3) no enzyme control. As shown in Figure 12B, deglycosylation of IL-15 K86R-A10m3 (blue) nearly completely rescued activity in CTLL2 proliferation assays, in comparison with non-treated samples (purple, yellow). WT IL-15 from R&D systems (red) and in-house E.coli produced IL-15-A10m3 (black) were used as positive controls.

### N112 of IL-15 K86R-A10m3 is critical for its bioactivity to promote CTLL2 proliferation

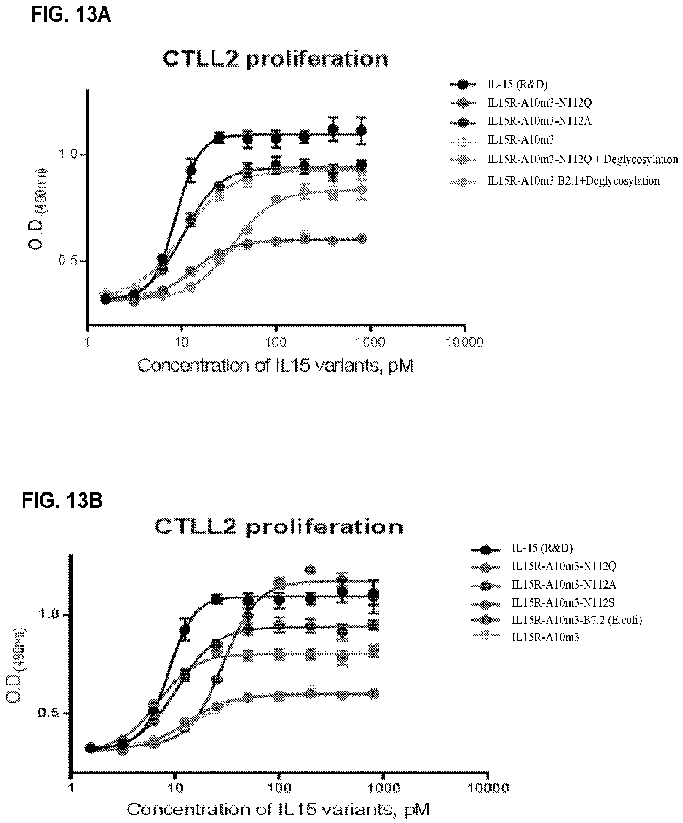

Amino acid position N112 of IL-15 K86R-A10m3 is a key site for IL-15 bioactivity, particularly in the context of IL-15-ABD, as it is critical for a proper establishment of the interaction between IL-15 and IL15 receptor gamma. Variant IL-15 K86R was further mutated at N112A to determine whether mutations at this site could restore IL-15 bioactivity, similar to deglycosylated IL-15. In particular, IL15 K86R-A10m3 were mutated to further include a N112Q, N112A or N112S IL-15 amino acid substitution and CTTLL2 proliferation assays were carried out to test bioactivity. As shown in Figure 13A, introducing amino acid substitution N112A into IL-15 K86R-A10m3 (blue) restored its bioactivity comparable to that of deglycosylated IL-15 K86R-A10m3 (green) in CTLL2 proliferation assays, while N112Q mutation (red) has no effect on bioactivity relative to the parental IL-15R-A10m3 without deglycosylation (yellow). WT IL-15 (Black) from R&D systems served as positive control.

Mutations with different side chains at N112 were tested to further demonstrate the size effect on the bioactivity. As show in Figure 13B, N112Q (big, red), N112S (medium, green) and N112A (small, blue) displayed an increased bioactivity inversely proportional to the size of the side chains. Moreover, the suggested hydrogen bond established by N112 of IL-15 and Y103 of IL15 receptor gamma does not seem to be important for this activity, as N112A cannot form such a bond. WT IL-15 from R&D systems (black) and in-house E.coli produced IL-15-A10m3 (purple) were used as the positive control; parental IL15 K86R-A10m3 without deglycosylation (yellow) was used as the negative control.

### Example 3: In vivo Activity of Variant IL-15 and IL-15-ABDs

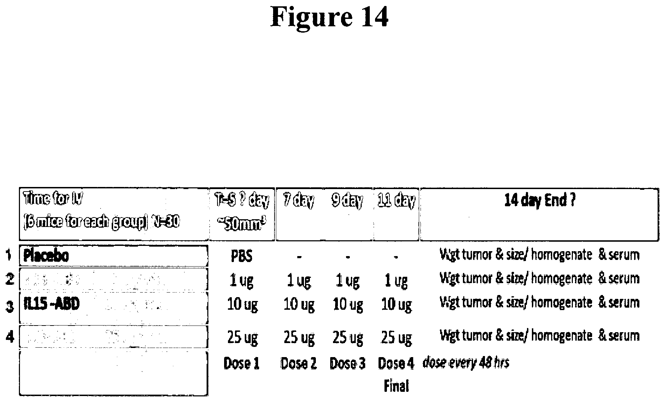

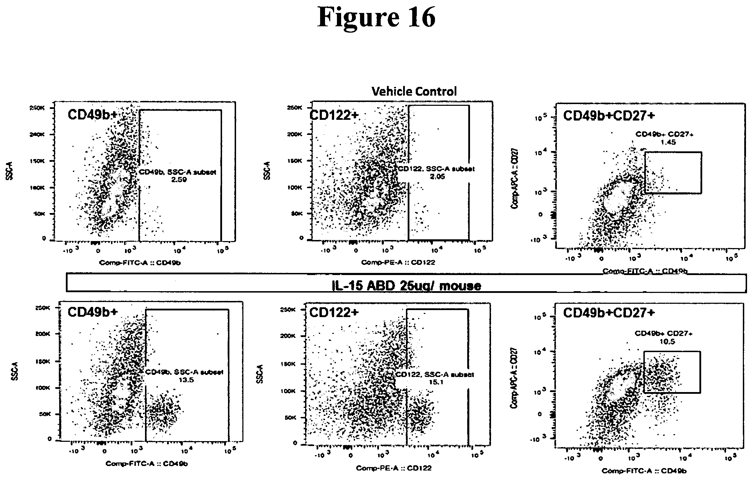

The ability for IL-15 and IL-15-ABD to inhibit tumor growth was assessed using a B16-F10 mouse melanoma model . As summarized in Figure 14, mice were treated with IL-15, PBS placebo or various doses of IL-15-ABD by IV injection at four different time points, spaced 48 hours apart. As shown in Figure 16, IL-15-ABD inhibits tumor growth in a dose dependent manner.

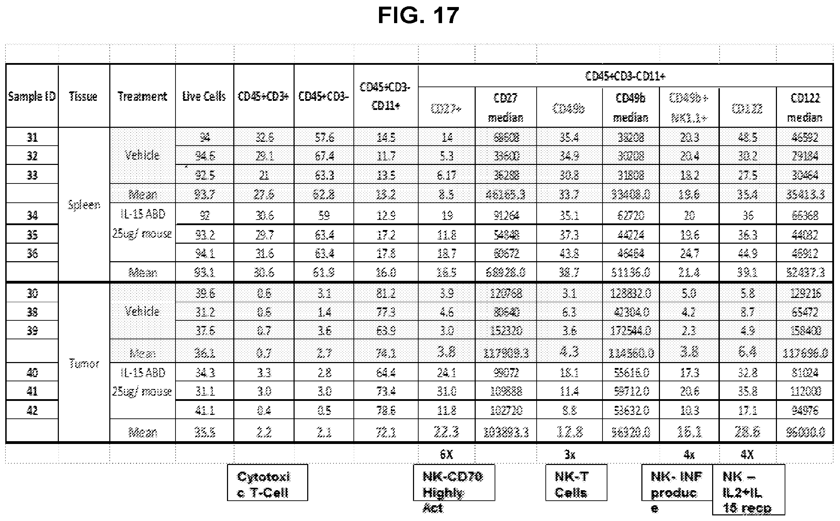

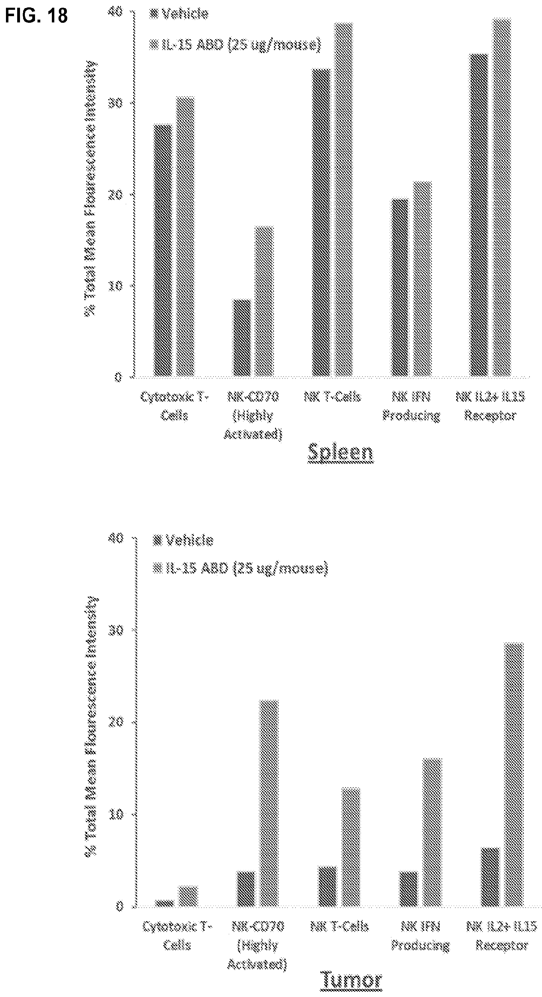

FACS analyses were performed to further assess the profile of tumor infiltration lymphocyte populations in IL-15-ABD treated mice from these studies. As shown in Figure 16, tumors in IL-15-ABD treated mice exhibited an increase in NK cell populations. This data coupled with observations of increased tumor accumulation and retention in IL-15-ABD treated mice, as described above, suggest the ABD enhances the proinflammatory effect of IL-15 within the tumor. The effects of IL-15-ABD treatment on lymphocyte populations in spleens and tumors are summarized in Figures 17 and 18. As shown in Figures 17 and 18, FACS analysis of lymphocytic populations show a 3 - 6 fold increases of tumor infiltrating CTL and NK cell populations in tumors of IL-15 ABD treated mice. No significant differences were observed in spleen. Taken altogether, the results of these studies show the tumor immunomodulatory capability of IL-15-ABDs *in vivo.*

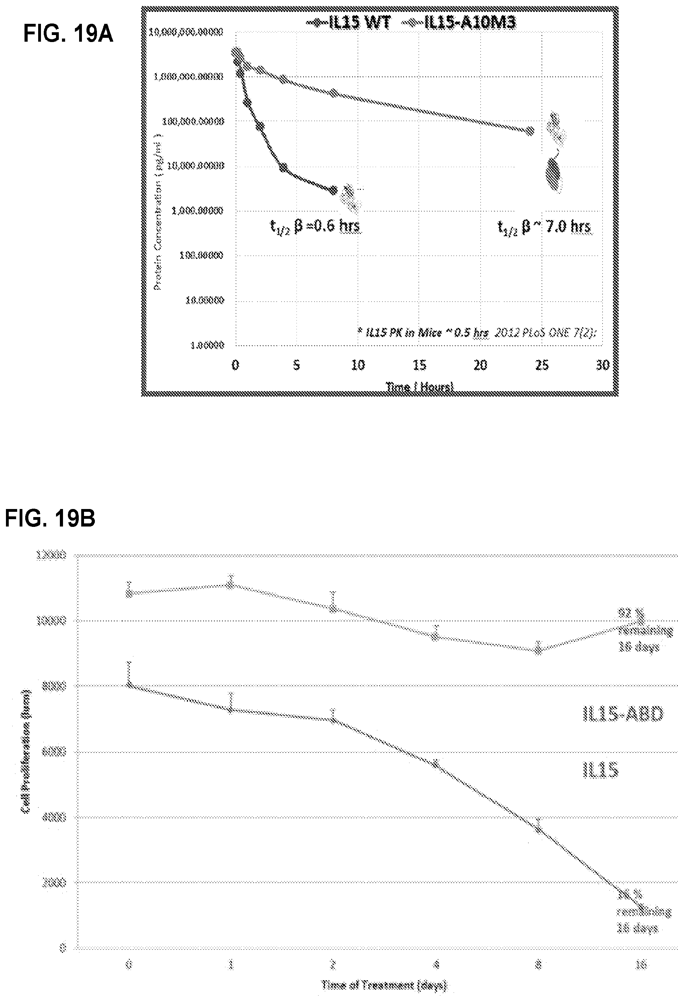

To assess the ability of ABD fusion proteins to increase the half-life of IL-15, C57B mice were injected intravenously with 5 µg of IL-15-ABD or IL-15 alone and serum concentrations of IL-15-ABD and IL-15 were subsequently assessed. As shown in Figure 20A, IL-15-ABD exhibited a greater PK as compared to IL-15 WT. IL-15 T ½β = 0.6 hrs, similar to those reported in the public domain (~0.5 hrs). Study results show ABD extends IL-15 T ½β to ~7.0 hours, which is a ~10X fold increase. IL-15-ABD was also assayed for stability in human serum using a cell based assay. As shown in Figure 19B, IL-15-ABD was more stable in human serum compared to commercial IL-15 control, without ABD.

### Example 4: IL-12-ABD

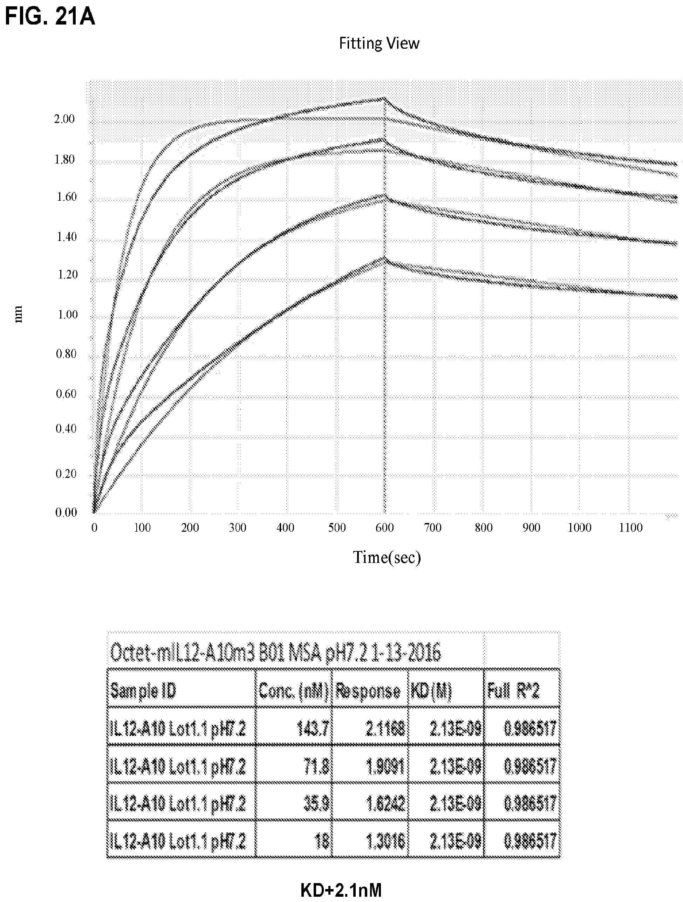

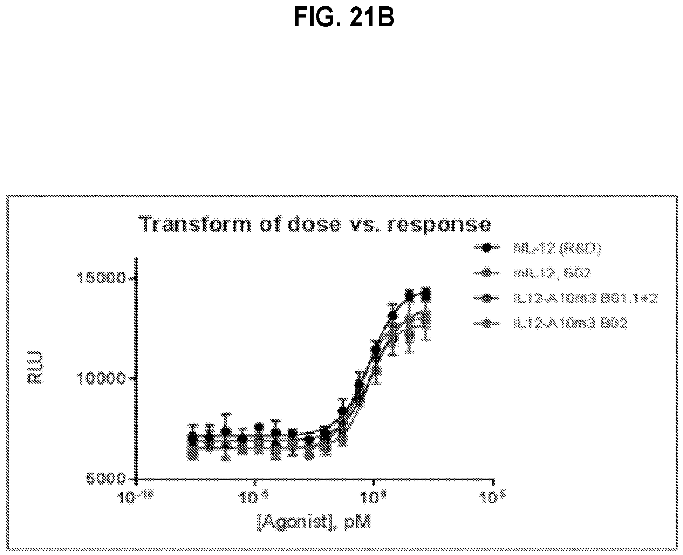

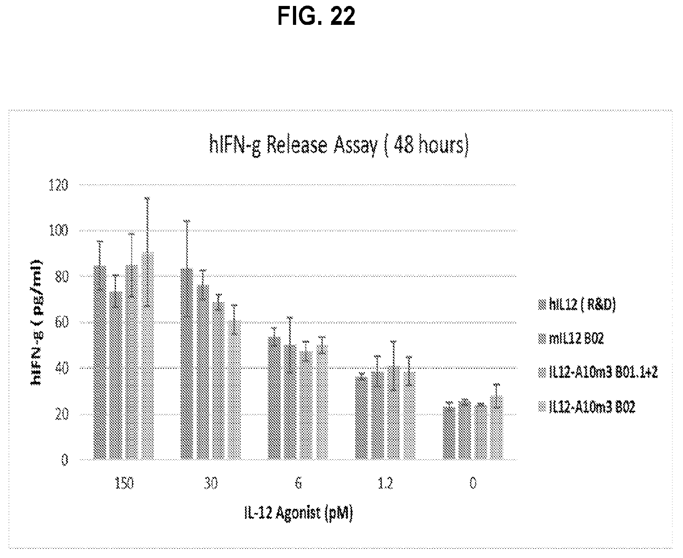

Mouse IL-12 single chain-ABD constructs were made in HEK293T cells and purified by size exclusion chromatograph. IL-12-A10m3 produced from HEK293T cells are fully active in both *in vitro* assays and cell based assays. As shown in Figure 21A, IL12-A10m3 is capable of binding to mouse serum albumin, with an equilibrium dissociation constant (KD) of 2.1nM. IL12-A10m3 produced from HEK293T were also capable of stimulating human PBMC proliferation, comparable to that of in-house produced mouse IL12 and commercial-available mouse IL-12 (R&D)(Figure 21B). Moreover, IL12-A10m3 produced from HEK293T stimulated secretion of interferon gamma from human PBMC, comparable to that of in-house produced mouse IL-12 and commercial-available mouse IL-12 (R&D) (Figure 22).

### Treatment with IL-12-ABD reduces tumor volume in vivo.

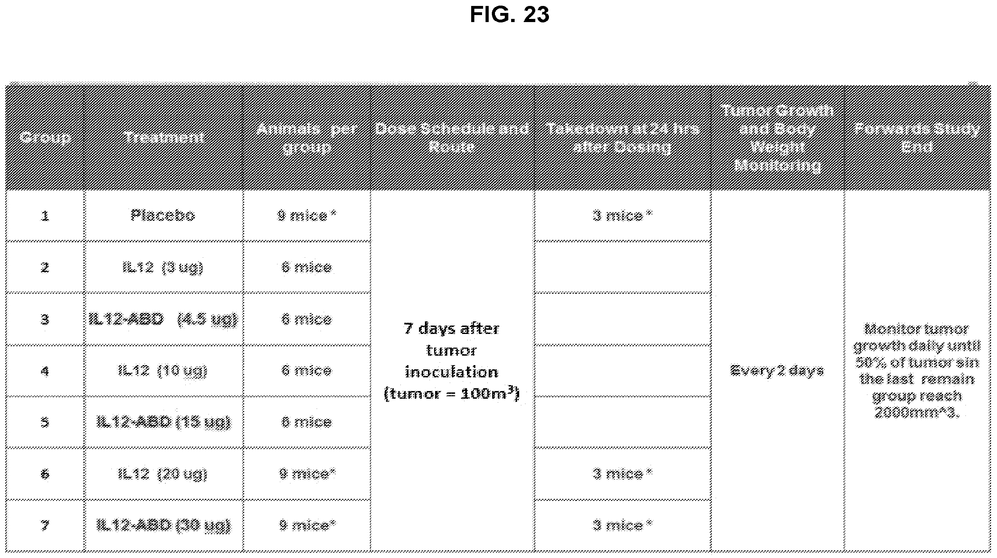

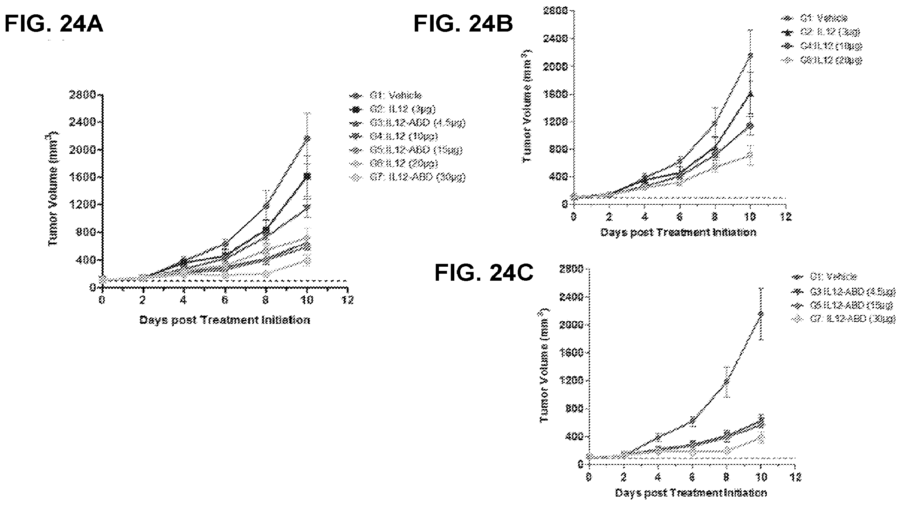

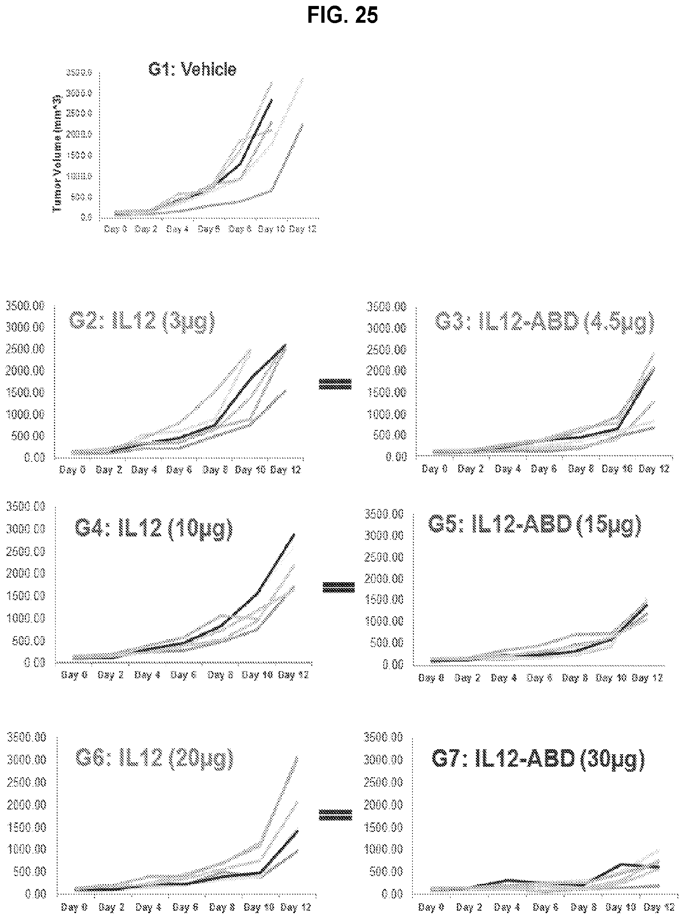

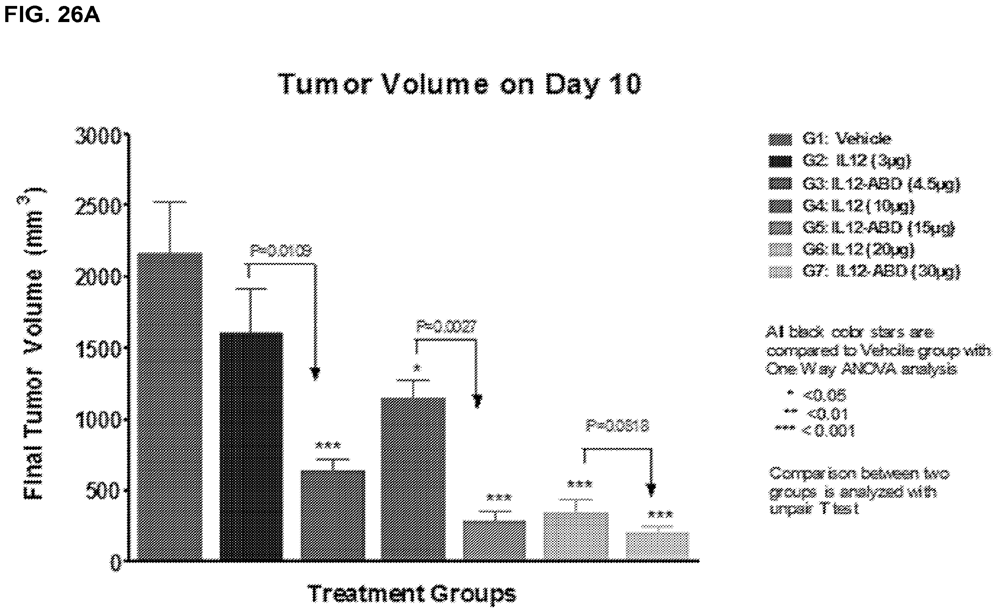

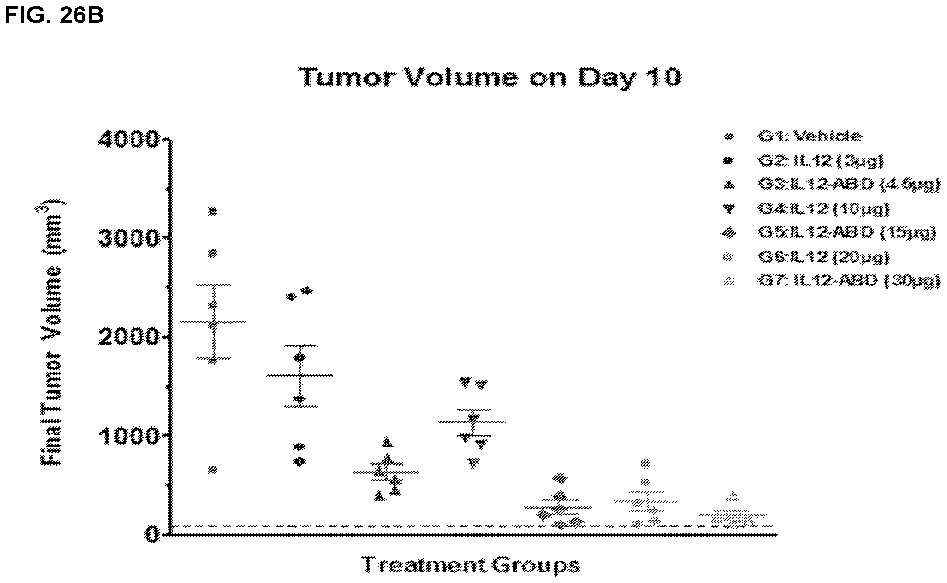

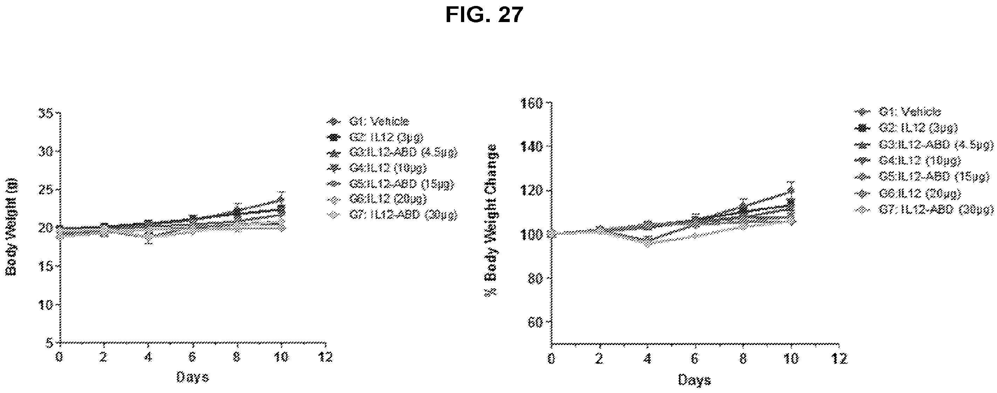

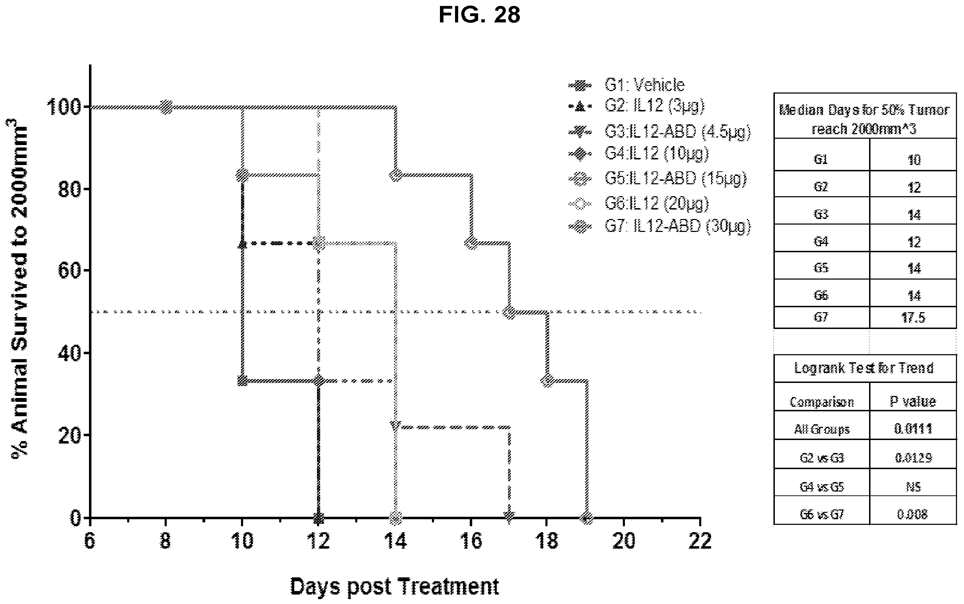

The ability for IL-12 and IL-12-ABD to inhibit tumor growth was assessed using a B16-F10 mouse melanoma model . As summarized in Figure 23, mice were treated with either IL-12-ABD or IL-12 at three similar doses by IV injection on day 7 after tumor inoculation (day 0), when tumor volume reached 100 mm³. Tumor growth was monitored every 2 days post treatment for 10 days. PBS placebo served as a control. As shown in Figures 24-26 and 28, both IL-12 and IL-12-ABD were able to reduce tumor growh in a dose dependent manner. Moreover, IL-12-ABD was able to reduce tumor volume more effectively compared to IL-12 alone at similarly concentrations (*See, e.g*., Figure 26 at 10 days and Figure 28, median days for 50% tumors to reach 2000 mm³). Longitudinal body mass measurements of mice from these studies show minimal changes in weight across all IL-12-ABD treatment groups (Figure 27). Lack of observed significant changes in mass, suggest a lack of IL-12-ABD toxicity in treatment groups over the 12 day post treatment course.

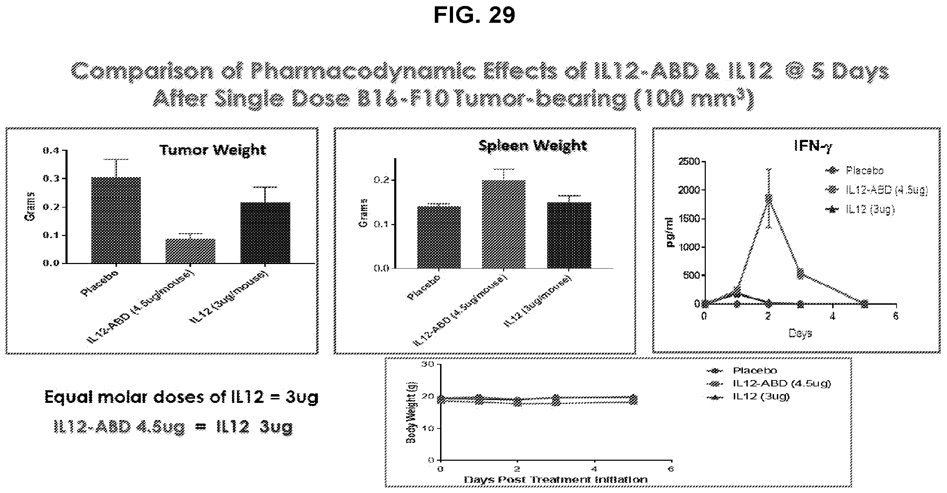

Further characterization of the pharmacodynamic effects of a single dose of IL-12-ABD (4.5 µg IL-12-ABD, same molar dose as 3 µg IL-12 control) in B16-F10 tumor-bearing mice after 5 days demonstrated that IL-12-ABD exhibited a similar greater suppression of tumor growth as compared to a similar molar dose of IL-12 control. IL-12-ABD treated mice also exhibited a corresponding increase in immune activation as shown by an increase in spleen weight and IFN-y, without an effect on mouse bodyweight, as compared to control (Figure 29).

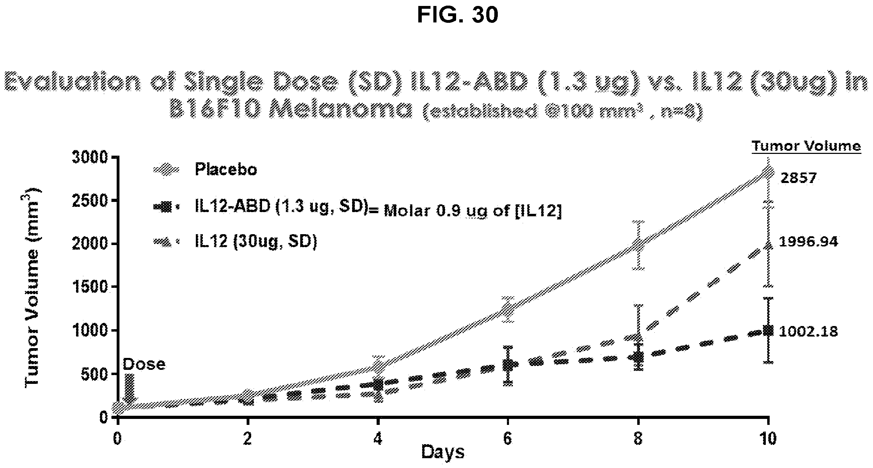

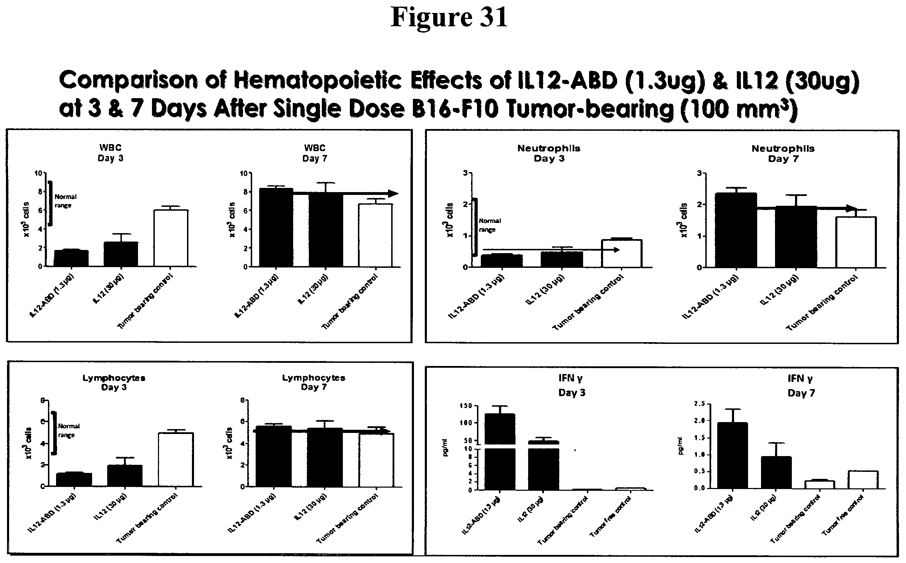

Figure 30 further shows the result of a study comparing tumor volume of B16-F10 tumor-bearing mice at 10 days, injected either with IL-12-ABD (1.3 µg), IL-12 (30 µg), or placebo. Although IL-12 (1 µg) and IL-12-ABD (1.3 µg) are molar equivalent and have the same bioactivity in vitro, IL-12-ABD is ~ 30+ fold more potent than IL-12 in vivo (compare results at day 10 in Figure 30, 1.3 µg IL-12-ABD ≥ 30 µg IL-12). Figure 31 further depicts the hematopoietic effects of IL-12-ABD and IL-12 in mice from the study depicted in Figure 30 at 3 and 7 days. As shown in Figure 31, mice treated with IL-12-ABD exhibited a transient lowering of WBC, neutrophils and lymphocytes on day 3 as compared to IL-12 treated mice and placebo control. Such cell populations, however, returned to normal by day 7. Further, IFN-y levels in mice treated with IL-12-ABD were higher at days 3 and 7, as compared to mice treated with IL-12 and control.

### Evaluation of anti-tumor effects of IL-12-ABD or IL-12 in combination with anti-PD-1 antibody in vivo.

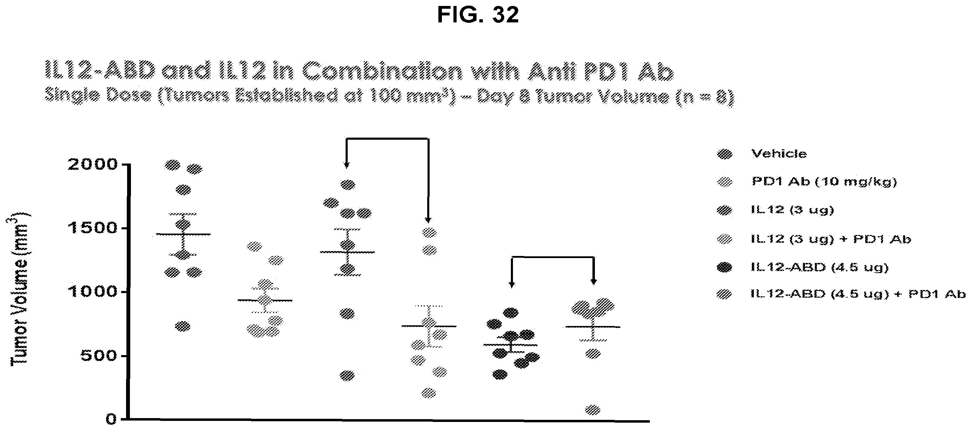

The effect of single dose combination therapies using IL-12-ABD or IL-12 with anti-PD-1 antibodies was assessed in B16-F10 tumor-bearing mice at 8 days (Figure 32).

Animals (7 - 10 weeks old) were assigned into 8 groups (8 animals per group) 10 days after B16-F10 tumor cell inoculation (2 x 10⁴ cell/mouse). Animals were assigned based on the tumor volume. At the time of assignment, the average tumor volume per group was 100 mm³). On day 0 (when tumors reached 100 mm³) each group was given an I.V. single dose of either PBS (Placebo), IL12-ABD (1.5 ug, 5 ug, 15 ug) or IL15-ABD-IL12 (1.7 ug, 6 ug, 17 ug).

Groups were examined for body weight, tumor volume and pseudo survivability. Body weights were measured prior to tumor inoculation and at time of tumor measurements. Tumor size was measured every 2 days in two dimensions using a caliper, and the volume expressed in mm³ using the formula: V = 0.5 x a x b² where a and b are the long and short diameters of the tumor. The study was conducted as pseudo-survival; each mouse was euthanized when its tumor reached 2000 mm³ or when determined to be moribund.

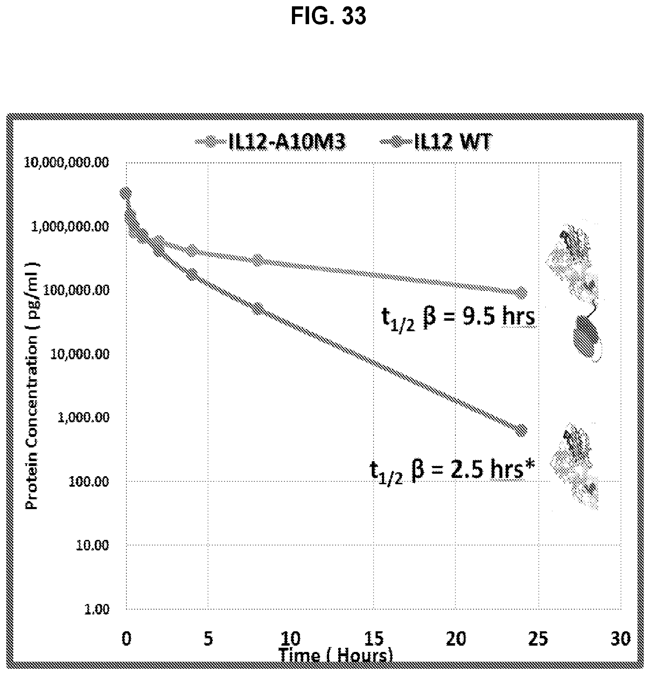

As shown in Figure 33, IL-12-ABD was more effective than treatment with either anti-PD-1 or the molar equivalent dose of recombinant IL-12. Moreover, IL-12-ABD was as effective as the combination of recombinant IL-12 and anti-PD-1 treatment. Interestingly, the addition of anti-PD-1 Ab to recombinant IL-12 improved the efficacy of either treatment alone, whereas, anti-PD-1 treatment provided no further benefit to IL-12-ABD

To assess the ability of ABD fusion proteins to increase the half-life of IL-12, C57B mice were injected intravenously with 5 µg of IL-12-ABD or IL-12 alone and serum concentrations of IL-12-ABD and IL-12 were assessed. As shown in Figure 33, IL-12-ABD exhibited a higher PK than IL-12 WT. IL-12 T ½β = 2.5 hrs, similar to those reported in the public domain (~3.5 hrs). Study results show ABD extends IL-12 T ½β to 9.5 hours, a ~4X fold increase.

### Example 5: Bispecific IL-15-ABD-IL-12

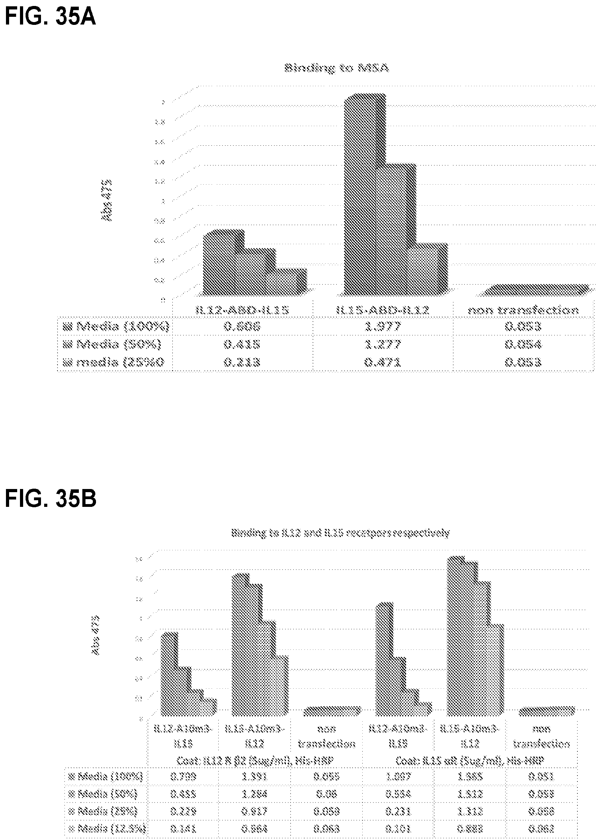

IL-15-ABD-IL-12, hIL15 (K86R/N112A)-A10m3-mIL-12sc and mIL-12sc - A10m3- hIL15 (K86R/N112A) constructs were made in HEK293T cells and purified by size exclusion chromatograph . The sequences of these constructs are depicted in Figure 34. The ability of hIL15 (K86R/N112A)-A10m3-mIL-12sc and mIL-12sc -A10m3- hIL15 (K86R/N112A) constructs to bind MSA, IL12 receptor beta 2 and IL-15 receptor alpha were assessed by ELISA. As shown in Figure 35, both IL-15-ABD-IL-12 constructs were able to bind to MSA in a dose dependent manner in cell culture media. Further, both bispecific constructs were able to bind to IL12 receptor beta2 and IL15 receptor alpha in a dose dependent manner in cell culture media. As shown in Figure 35, IL-12/IL-15-ABDs having the orientation, for N-terminus to C-terminus, IL-15-ABD-IL-12, exhibited better antigen binding as compared to IL-12-ABD-IL-15. Additional bispecifics that include IL-15 and IL-12 are disclosed in Figure 36.

### Assessment of IL-15-ABD-IL-12 for IL-12 and IL-15 activity

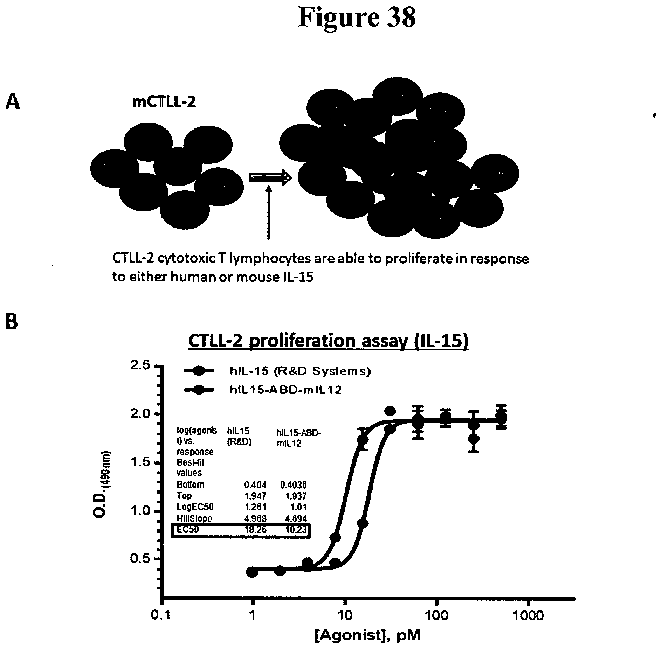

IL-15-ABD-IL-12 was further assessed for IL-12 and IL-15 activity (Figures 37 and 38).

To assess IL-12 activity, lymphocytes from PBMCs were triggered undergo blast formation by treatment with PHA-P for four days and rhIL-2 on the third day. Lymphoblasts were then treated with either IL-15-ABD-IL-12 or IL-12 control for two days and IL-12 activity was assessed, based on lymphoblast proliferation and IFN-y secretion (Figure 37A). IL-15 activity was assessed using a CTLL-2 cytotoxic T lymphocyte proliferation assay (Figure 38A).

As shown in Figure 37, IL-15-ABD-IL-12 exhibited IL-12 activity as assess by lymphoblast proliferation (Figure 37B) and IFN-y secretion (Figure 37C). Moreover, IL-15-ABD-IL-12 exhibited IL-15 in the CTLL-2 proliferation assay (Figure 38B). As such, subject IL-15-ABD-IL-12 exhibited both IL-12 and IL-15 bioactivity.

### Anti-tumor effects of IL-15-ABD-IL-12 in a B16-F10 mouse melanoma model

Without being bound by any particular theory of operation it is believed that IL-15/IL-12 ABDs provide synergistic biological activity. In particular, IL-12 increases IL-15 alpha receptor, IFN-y. NK/T cells, and TH1 immunity, while downregulating Treg cells. IL-15 increases IL-12 beta 1 receptor, and NK cells , while reducing CD8 cell memory loss.

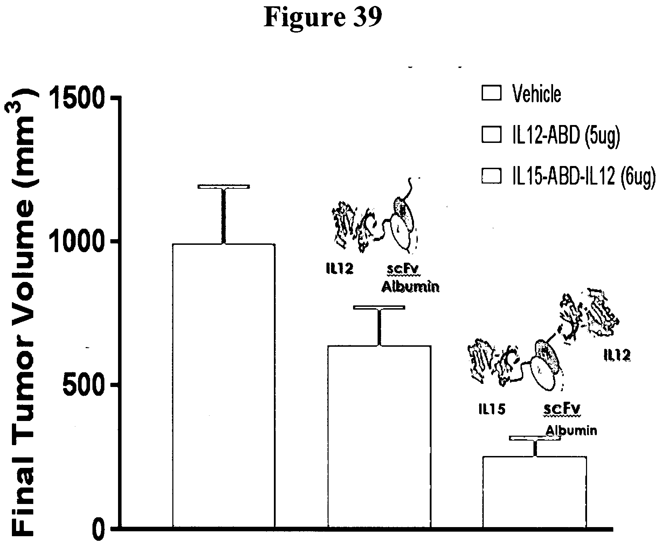

The anti-tumor effects and pseudo-survivability of IL-12-ABD to IL15-ABD-IL12 was assessed using in B16-F10 mouse melanoma model (Figure 39).

Animals (7-10 weeks old) were assigned into 8 groups (8 animals per group) 10 days after B16-F10 tumor cell inoculation (2 x 10⁴ cell/mouse). Animals were assigned based on the tumor volume. At the time of assignment, the average tumor volume per group was 100 mm³). On day 0 (when tumors reached 100 mm³) each group was given an I.V. single dose of either PBS (Placebo) or molar does equivalents of IL12-ABD (1.5 ug, 5 ug, 15 ug) or IL15-ABD-IL12 (1.7 ug, 6 ug, 17 ug).

As shown in Figure 39, IL-15-ABD-IL-12 was superior in anti-tumor activity as compared to IL-12-ABD in the B16-F10 mouse model at equal molar concentration. Other similar in-vivo studies show that free IL-12 (5ug) in combination with I-15 (1ug) is less than 50% as potent as IL-15-ABD-IL-12 (6ug) (data not shown).

### Example 6: Anti-TGFβ-ABD

After biopanning and screening conducted using surface plasmon resonance technology, anti-hTGFβ1 binding domains were identified: 1A10, 1F11, 2H6, 4B9, 4C10, 4D9, 4G3, 4G6, 4H4, 4H7, and 6H11. These clones exhibited cross reactivity to hTGFβ2, 3 and mTGFβ1 and with potential inhibition of the binding of the hTGFβ1 to its receptor II. The clones were subsequently selected for purification as scFvs and further characterization

The scFvs were screened for cross-reactivity to mouse and human TGFβ-1 using standard ELISA techniques. Binding ELISAs show that 2H6, 4G3, 4H7, 4B9, 4D9 & 6H11 have good cross-reactivity against both mTGFβ-1 and hTGFβ-1.

Binding & blocking ELISA was performed to determine whether the anti-TGFβ-1 scFvs were able to bind TGFβ-1 and block its interaction with TGFβR-II. 2H6, 4G3, 4H7, 4B9 & 4D9 all show good blocking efficacy and inhibition of mTGFβ & mTGFβR-II interaction.

Several of these anti-TGFβ-1 scFvs were tested for ability to interfere with TGFβ-1 bioactivity. The sequences of anti-TGFβ-1 scFvs 4H7 and 4D9 are shown in Figures 40A and B.

### Blockade of TGFβ1-induced expansion of CD4⁺Foxp3⁺ Regulatory T cells

Regulatory T cells (Tregs) are able to influence the homeostasis of the immune system. Such Treg are essential for maintaining self-tolerance as defects can lead to severe autoimmune diseases. In cancer, tumor cells are able to secret cytokines that affect immune system homeostasis. In particular, tumor cells may secrete TGFβ, which then can affect the number of circulating Tregs. It has been previously demonstrated that exposure to TGFβ1 leads to an expansion of CD4⁺Foxp3⁺ Treg subsets from CD4⁺Foxp3⁻ T cells. These induced Tregs are then able to contribute to the induction of a T cell anergic response by inhibiting the activation of tumor antigen specific cytotoxic CD8⁺ T cells.

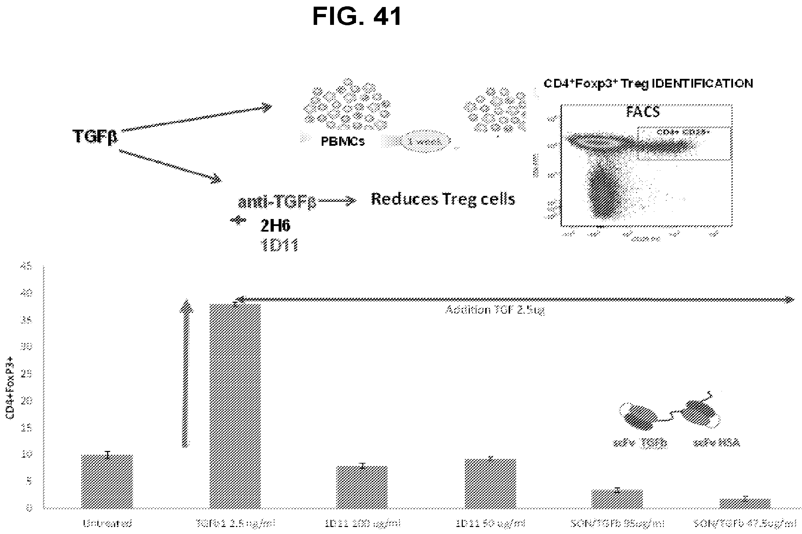

As shown in Figure 41, recombinant TGFβ1 is capable of stimulating the expansion of CD4⁺Foxp3⁺ Tregs from a mixed T cell population isolated from healthy human donor PBMCs. TGFβ blockade with anti- TGFβ 1D11 antibody or the TGF-β1 scFv 2H6, 4H7, and 4D9, however, all significantly inhibit the TGFβ induced expansion of CD4⁺Foxp3⁺ Tregs also in a dose dependent manner. Thus, such TGFβ1scFvs are useful for reducing TReg expansion in cancers.

### Blockade of TGFβ1-induced epithelial-to-mesenchymal transition (EMT)

Exposure to TGFβ is known to induce epithelial-to-mesenchymal transition. During this process, epithelial cells transdifferentiate from an organized, polarized and tightly connected epithelial sheet of cells with cobblestone morphology into disorganized and motile cells that appear mesenchymal in morphology. During EMT, the invasive capacity of the cells is activated and thereby enhances the tumorigenic ability of the cells. E-cadherin is a commonly used marker of epithelial cells, and is localized to the adherens junctions between epithelial cells. Loss of E-cadherin is a strong marker of EMT that is indicative of the transdifferentiation process. (Figure 42A). Additionally, vimentin is associated with highly motile cells. Thus, the induction of vimentin expression in cells is also indicative of increased motility and increased localized invasion *in vivo.*

As shown in Figures 42 and 43,TGF-β blockade by anti- TGFβ 1D11 antibody or the TGF-β1 scFv reverses TGF-β1-induced epithelial-to-mesenchymal transition (Figure 42) and migration (Figure 43). Mouse 4T1 cells were cultured in growth media supplemented with TGF-β1 (panel 2); TGF-β1 and 1D11 (panel 3); or TGF-β1 and anti-TGF-β1 scFv (panel 4), then fixed and stained with E-cadherin antibody (green) and vimentin antibody (purple). Nuclei were counterstained with DAPI (blue). Treatment with TGF-β1 induced loss of E-cadherin from cell-cell junctions and increased expression of vimentin. This effect is reversed by the addition of 1D11 or subject anti-TGF-β1 scFv described herein (Figure 42 panels 3 and 4). Moreover, the anti-TGF-β1 scFv described herein is capable of blocking TGF-β1-mediated carcinoma cell migration (Figure 43).

### Neutralization of TGFβ1-induced Smad activation

The TGFβ superfamily consists of pleiotropic cytokines that regulate various biological processes including cell proliferation, differentiation, migration, cell survival, angiogenesis, wound healing and immune surveillance. In humans, the predominant isoform is TGFβ1, which is expressed in various tissue types. TGFβ inhibits the proliferation of most normal epithelial cells. Additionally, during the early stages of cancers of epithelial origin, TGFβ functions as a cell growth inhibitor. Thus, during cancer initiation, TGFβ acts as a tumor suppressor. However, during later stages of cancer progression, tumor cells become resistant to the growth inhibitory effects of TGFβ, and TGFβ takes on a tumor promoter role. Indeed, TGFβ1 has been shown to be overexpressed in various tumors. Activation of the TGFβ pathway occurs through the binding of TGFβ ligand to the type II TGFβ receptor (TβRII), which then induces an association and oligomerization between TβRII and TβRI. When this oligomer forms, Smad2 and Smad3 are recruited and phosphorylated by TβRI. Phosphorylated Smad2 or Smad3 then bind to Smad4 in the cytoplasm, and this complex translocates to the nucleus where it interacts with the promoter region and activate transcription of target genes. Thus, the activation of the TGFβ pathway is measurable by the rapid phosphorylation of Smad2 after addition of TGFβ to serum starved cells. However, effective blockade of TGFβ will inhibit Smad2 phosphorylation. Here, the absence of Smad2 phosphorylation can be used as a measure of effective blockade of TGFβ by the subject anti-TGFβ scFv constructs.

Using serum-starved human (Figure 44A) or mouse cells (Figure 44B), it was determined that addition of human recombinant TGFβ1 (Figure 44A) or mouse TGF-β1, -β2 and -β3 (Figure 44B) induced Smad2 phosphorylation. Such phosphorylation is reduced in a dose dependent manner when the TGFβ is preincubated with control anti- TGFβ 1D11 antibody or the TGF-β1 scFv constructs 2H6, 4H7, and 4D9. These data suggest that the 2H6, 4H7 and 4D9 scFv constructs are able to sequester TGFβ1 and inhibit its interaction with TβRII/TβRI, thereby inhibiting the TGFβ activation cascade during late stage cancer.

### 4D9 anti-TGFβ-1-ABD

The sequences of exemplary TGF-β1 scFv-ABD constructs (4D9M-A6m and 4H7-A6m), are shown in Figure 45A. As shown in Figure 45B, anti-TGFβ-1-ABD extended the anti-TGFβ-1 scFv T ½β from 106 minutes to 10.6 hours.

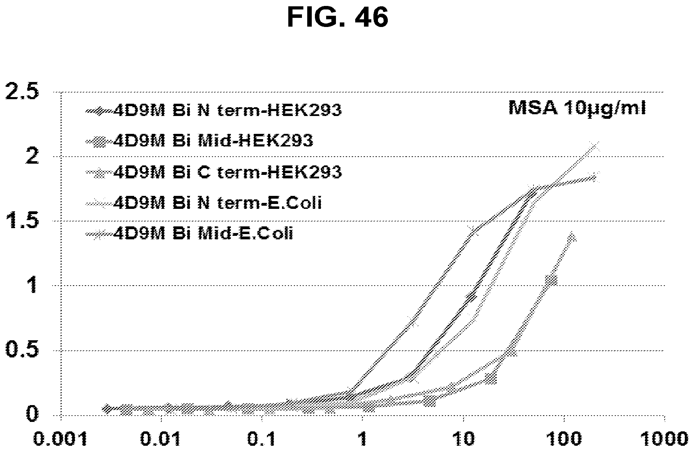

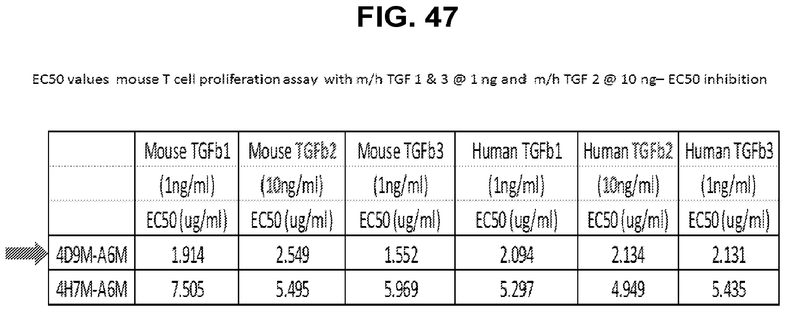

Anti-TGF-β1 scFv-ABDs (bivalent for TGF-β1) produced in E. coli and HEK cells were assessed for binding to mouse serum albumin (Figure 47). Three different orientations of the constructs were assessed: two anti-TGF-β1 scFvs attached to the N terminal of the ABD ("Bi N Term"); two anti-TGF-β1 scFvs attached to the C terminal of the ABD ("Bi C Term); or one anti-TGF-β1 scFv attached to each of the N terminal and C terminal of the ABD ("Bi Mid"). As shown in Figure 46, all constructs exhibited binding to mouse serum albumin. With respect to constructs produced in E. coli, Bi Mid exhibited better binding to MSA than the N Term orientation. With respect to constructs produced in HEK cells, the Bi N Term orientation exhibited better binding to MSA than either the Bi Mid or Bi C term orientation.

As shown in Figure 47, TGFβ-1 mediated inhibition of T cell proliferation was reversed (i.e., increase T cell proliferation) by such constructs (Figure 46B). Further, 4D9M-ABD has been shown to block human TGFβ-1 and human TGFβ-3 binding with cognate receptors (data not shown).

## Claims

1. An IL-12 albumin binding domain fusion protein comprising the amino acid sequence of

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK
NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLG.

2. A nucleic acid encoding the IL-12 albumin binding domain fusion protein of claim 1.

3. An expression vector comprising the nucleic acid of claim 2.

4. A host cell comprising the nucleic acid of claim 2 or the expression vector of claim 3.

5. A method of making the IL-12 albumin binding domain fusion protein of claim 1, comprising culturing the host cell of claim 4 under conditions wherein the IL-12 albumin binding domain fusion protein is expressed, and recovering the IL-12 albumin binding domain fusion protein.

6. An IL-12/IL-15 albumin binding domain fusion protein comprising the amino acid sequence of

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK

NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLGGGGGSGGGGSGGGGSGGGGSGGGGSNWVNVISDLKKIEDLIQS
MHIDATLYTESDVHPSCKVTAMKCFLLELQVISLESGDASIHDTVENLIILANNSLSSN
GNVTESGCRECEELEEKNIKEFLQSFVHIVQMFIATS.

7. A nucleic acid encoding the IL-12/IL-15 albumin binding domain fusion protein of claim 6.

8. An expression vector comprising the nucleic acid of claim 7.

9. A host cell comprising the nucleic acid of claim 7 or the expression vector of claim 8.

10. A method of making an IL-12/IL-15 albumin binding domain fusion protein comprising culturing the host cell of claim 9 under conditions wherein the IL-12/IL-15 albumin binding domain fusion protein is expressed, and recovering the IL-12/IL-15 albumin binding domain fusion protein.

## Patentansprüche

1. Fusionsprotein mit IL-12-Albuminbindungsdomäne, umfassend die Aminosäuresequenz von

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK
NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLG.

2. Nukleinsäure, die für das Fusionsprotein mit IL-12-Albuminbindungsdomäne nach Anspruch 1 codiert.

3. Expressionsvektor, umfassend die Nukleinsäure nach Anspruch 2.

4. Wirtszelle, umfassend die Nukleinsäure nach Anspruch 2 oder den Expressionsvektor nach Anspruch 3.

5. Verfahren zum Herstellen des Fusionsproteins mit IL-12-Albuminbindungsdomäne nach Anspruch 1, umfassend Kultivieren der Wirtszelle nach Anspruch 4 unter Bedingungen, bei denen das Fusionsprotein mit IL-12-Albuminbindungsdomäne exprimiert wird, und Rückgewinnen des Fusionsproteins mit IL-12-Albuminbindungsdomäne.

6. Fusionsprotein mit IL-12/IL-15-Albuminbindungsdomäne, umfassend die Aminosäuresequenz von

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK
NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLGGGGGSGGGGSGGGGSGGGGSGGGGSNWVNVISDLKKIEDLIQS
MHIDATLYTESDVHPSCKVTAMKCFLLELQVISLESGDASIHDTVENLIILANNSLSSN
GNVTESGCRECEELEEKNIKEFLQSFVHIVQMFIATS.

7. Nukleinsäure, die für das Fusionsprotein mit IL-12/IL-15-Albuminbindungsdomäne nach Anspruch 6 codiert.

8. Expressionsvektor, umfassend die Nukleinsäure nach Anspruch 7.

9. Wirtszelle, umfassend die Nukleinsäure nach Anspruch 7 oder den Expressionsvektor nach Anspruch 8.

10. Verfahren zum Herstellen eines Fusionsproteins mit IL-12/IL-15-Albuminbindungsdomäne, umfassend Kultivieren der Wirtszelle nach Anspruch 9 unter Bedingungen, bei denen das Fusionsprotein mit IL-12/IL-15-Albuminbindungsdomäne exprimiert wird, und Rückgewinnen des Fusionsproteins mit IL-12/IL-15-Albuminbindungsdomäne.

## Revendications

1. Protéine de fusion IL-12-domaine de liaison à l'albumine comprenant la séquence d'acides aminés de

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK
NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLG.

2. Acide nucléique codant pour la protéine de fusion IL-12-domaine de liaison à l'albumine de la revendication 1.

3. Vecteur d'expression comprenant l'acide nucléique de la revendication 2.

4. Cellule hôte comprenant l'acide nucléique de la revendication 2 ou le vecteur d'expression de la revendication 3.

5. Procédé de production de la protéine de fusion IL-12-domaine de liaison à l'albumine de la revendication 1, comprenant la culture de la cellule hôte de la revendication 4 dans des conditions dans lesquelles la protéine de fusion IL-12-domaine de liaison à l'albumine est exprimée, et la récupération de la protéine de fusion IL-12-domaine de liaison à l'albumine.

6. Protéine de fusion IL-12/IL-15-domaine de liaison à l'albumine comprenant la séquence d'acides aminés de

IWELKKDVYVVELDWYPDAPGEMVVLTCDTPEEDGITWTLDQSSEVLGSGKTLTIQ
VKEFGDAGQYTCHKGGEVLSHSLLLLHKKEDGIWSTDILKDQKEPKNKTFLRCEAK
NYSGRFTCWWLTTISTDLTFSVKSSRGSSDPQGVTCGAATLSAERVRGDNKEYEY
SVECQEDSACPAAEESLPIEVMVDAVHKLKYENYTSSFFIRDIIKPDPPKNLQLKPLK
NSRQVEVSWEYPDTWSTPHSYFSLTFCVQVQGKSKREKKDRVFTDKTSATVICRK
NASISVRAQDRYYSSSWSEWASVPCSGGGGGGSRNLPVATPDPGMFPCLHHSQ
NLLRAVSNMLQKARQTLEFYPCTSEEIDHEDITKDKTSTVEACLPLELTKNESCLNS
RETSFITNGSCLASRKTSFMMALCLSSIYEDLKMYQVEFKTMNAKLLMDPKRQIFLD
QNMLAVIDELMQALNFNSETVPQKSSLEEPDFYKTKIKLCILLHAFRIRAVTIDRVTSY
LNASGGGGSGGGGSGGGGSGGGGSGGGGSEVQLVESGGGLIQPGRSLRLSCAA
SGITFDDAVMHWVRQAPGKGLEWVAGISSNSGYIGYADSVKGRFTISRDNAKNSLY
LQMNRLRAEDTAVYYCVKGLYSNPRGGAFDIWGQGTMVTVSSASTGGGGSGGG
GSGGGGSVHSSYVLTQPPSVSVAPGQTATITCGGNNIGTKSVHWYQQKPGQAPV
LVVYADSDRPSGIPERVSGSNSGNTATLTISRVEAGDEADYYCQVWDSRSDHLWV
FGGGTKLTVLGGGGGSGGGGSGGGGSGGGGSGGGGSNWVNVISDLKKIEDLIQS
MHIDATLYTESDVHPSCKVTAMKCFLLELQVISLESGDASIHDTVENLIILANNSLSSN
GNVTESGCRECEELEEKNIKEFLQSFVHIVQMFIATS.

7. Acide nucléique codant pour la protéine de fusion IL-12/IL-15-domaine de liaison à l'albumine de la revendication 6.

8. Vecteur d'expression comprenant l'acide nucléique de la revendication 7.

9. Cellule hôte comprenant l'acide nucléique de la revendication 7 ou le vecteur d'expression de la revendication 8.

10. Procédé de production d'une protéine de fusion IL-12/IL-15-domaine de liaison à l'albumine comprenant la culture de la cellule hôte de la revendication 9 dans des conditions dans lesquelles la protéine de fusion IL-12/IL-15-domaine de liaison à l'albumine est exprimée, et la récupération de la protéine de fusion IL-12/IL-15-domaine de liaison à l'albumine.
